(19) Europäisches Patentamt
European Patent Office
Office européen des brevets

(11) **EP 3 436 478 B1**

(12) **EUROPEAN PATENT SPECIFICATION**

(45) Date of publication and mention
of the grant of the patent:
**20.10.2021 Bulletin 2021/42**

(51) Int Cl.:
***C07K 16/28*** *(2006.01)*     ***C07K 16/30*** *(2006.01)*
***A61K 39/395*** *(2006.01)*     ***A61P 35/00*** *(2006.01)*

(21) Application number: **17714739.4**

(22) Date of filing: **30.03.2017**

(86) International application number:
**PCT/EP2017/057608**

(87) International publication number:
**WO 2017/167919 (05.10.2017 Gazette 2017/40)**

(54) **MULTISPECIFIC ANTIBODIES FOR USE IN THE TREATMENT OF A NEOPLASM OF THE URINARY TRACT**

MULTISPEZIFISCHE ANTIKÖRPER ZUR VERWENDUNG BEI DER BEHANDLUNG VON NEOPLASMEN DER HARNWEGE

ANTICORPS MULTISPÉCIFIQUES DESTINÉS À ÊTRE UTILISÉS POUR LE TRAITEMENT D'UN NÉOPLASME DU TRACTUS URINAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2016 PCT/EP2016/000531**

(43) Date of publication of application:
**06.02.2019 Bulletin 2019/06**

(73) Proprietor: **Lindis Biotech GmbH 82152 Planegg (DE)**

(72) Inventors:
• **BAUER, Hartwig-Wilhelm
82152 Planegg (DE)**
• **LINDHOFER, Horst
82152 Planegg (DE)**

(74) Representative: **Graf von Stosch Patentanwaltsgesellschaft mbH Prinzregentenstraße 22 80538 München (DE)**

(56) References cited:
• KERR ET AL: "Immunotherapy of renal cell carcinoma using the G250/OKT3 bispecific antibody", JOURNAL OF UROLOGY, 1 January 1990 (1990-01-01), page 321A, XP008182452, LIPPINCOTT WILLIAMS & WILKINS, BALTIMORE, MD, US ISSN: 0022-5347

• LUITEN R M ET AL: "Generation of chimeric bispecific G250/anti-CD3 monoclonal antibody, a tool to combat renal cell carcinoma.", BRITISH JOURNAL OF CANCER, vol. 74, no. 5, September 1996 (1996-09), pages 735-744, XP002764982, ISSN: 0007-0920
• PILANC KEZBAN NUR ET AL: "Dramatic Response to Catumaxomab Treatment for Malign Ascites Related to Renal Cell Carcinoma With Sarcomotoid Differentiation.", AMERICAN JOURNAL OF THERAPEUTICS, July 2014 (2014-07), pages 1-4, XP002764983, ISSN: 1075-2765
• KROESEN B J ET AL: "Bispecific antibodies for treatment of cancer in experimental animal models and man", ADVANCED DRUG DELIVERY REVIEWS, vol. 31, no. 1-2, 6 April 1998 (1998-04-06), pages 105-129, XP002764984, ISSN: 0169-409X
• Linke: "Catumaxomab Clinical development and future directions", , 1 January 2010 (2010-01-01), XP55687390, Retrieved from the Internet: URL:https://www.ncbi.nlm.nih.gov/pmc/artic les/PMC2840231/pdf/mabs0202_0129.pdf [retrieved on 2020-04-20]
• KUROKI ET AL: ANTICANCER RESEARCH, vol. 34, 2014, pages 4481-4488,

Remarks:
The file contains technical information submitted after the application was filed and not included in this specification

**Description**

The present invention relates to the treatment of a neoplasm of the urinary tract, in particular to immunotherapy of bladder cancer.

Neoplasms affecting the urinary tract are among the fastest growing neoplasm incidences worldwide, particularly due to the rapidly aging populations of most countries. Bladder cancer is the most prevalent of the neoplasms of the urinary tract. In the United States alone, more than 70,000 people are newly diagnosed with bladder cancer each year, 80 % of these have non-invasive bladder cancer. For example, 76,960 cases and 16,390 associated deaths are estimated for 2016 by the American Cancer Society (http://www.cancer.org/acs/ groups/ content/@research/ documents/ document/acspc-047079.pdf). Worldwide, bladder cancer is the 9th leading cause of cancer with 430,000 new cases (World Cancer Report 2014. World Health Organization. 2014. pp. Chapter 1.1. ISBN 9283204298) and about 165,000 deaths occurring yearly. Moreover, bladder cancer is likely to recur and, thus, patients with bladder cancer must undergo surveillance for an extended period. The overall 5-year survival rate for bladder cancer is 77%, and this rate has not changed significantly over the last 10 years, a period during which no new drugs for bladder cancer were approved by the FDA. When considered by stage, the 5-year relative survival rates for patients with tumors restricted to the inner layer of the bladder or those with disease localized to the bladder are 96% and 69%, respectively. The rates drop to 34% for those with disease that has spread locally beyond the bladder and to 6% for patients with distant metastases. Although most newly-diagnosed bladder cancers have not invaded the muscle layer, patients with high-grade tumors still have a significant risk of dying from their cancers. Tumor recurrence is also a major concern even for patients with low-grade disease and requires extensive follow-up.

Most bladder cancers begin in transitional epithelial cells that make up the inner lining of the bladder (the urothelium, also known as uroepithelium). As these tumors grow, they can invade the surrounding connective tissue and muscle. In advanced disease, tumors spread beyond the bladder to nearby lymph nodes or pelvic organs or metastasize to more distant organs, such as the lungs, liver, or bone. The urothelium, which is a "transitional epithelium", lines much of the urinary tract including the renal pelvis, the ureters, the bladder and parts of the urethra. The most common type of bladder cancer in particular and of urinary tract cancer in general is cancer affecting the urothelium (uroepithelium), which is known as "transitional cell carcinoma" (TCC, also referred to as "urothelial cell carcinoma"). About 90% of bladder cancer cases are classified as TCC, while the remaining 10% are mainly squamous cell or adenocarcinoma (Fair WR, Fuks ZY, Scher Hl. Cancer: Principles & Practice of Oncology, 4th ed. (ed. DeVita VT, Hellman S Rosenberg SA). J.B. Lippincott Co., Philadelphia, PA. 1993:1052-1072.). Upon the point of time of first diagnosis, 75% of tumors are "superficial", i.e. they have not (yet) entered the muscle layer and are typically classified according to the *"TNM Classification of Malignant Tumours"* as pTa, pT1 or pTIS. Thereof, 50 to 80% will have one or several recurrences, and 15 to 25% will progress to invasive tumors (Messing EM. Urothelial tumors of the bladder. In: Wein AJ, et al. Campbell-Walsh Urology. Philadelphia, Pa.: Saunders Elsevier; 2007;1445.).

The treatment of neoplasm of the urinary tract, in particular of bladder cancer, depends on how deep the neoplasm invades into the urinary tract wall.

Standard treatment for patients with muscle invasive bladder cancer includes cisplatin-based chemotherapy followed by surgical removal of the bladder or radiation therapy and concomitant chemotherapy. Recurrent bladder cancer is treated with combination chemotherapy regimens, including gemcitabine plus cisplatin (GC) or methotrexate, vinblastine, doxorubicin, and cisplatin (MVAC).

Standard treatment for patients with non-muscle invasive bladder cancer, comprises surgical removal of the tumor followed by one dose of chemotherapy, usually mitomycin C administered intravesically (intravesical chemotherapy). Cancer resection may lead to cure in some early stage patients. However, it is not applicable to all patients or the cancer may not be detected at an early enough stage. Alternatively or additionally, locally administered chemotherapy is sometimes performed. Superficial tumors (about 75% of TCC) may be "shaved off" using an electrocautery device attached to a cystoscope, which in that case is called a resectoscope. The procedure is called transurethral resection (TUR) and serves primarily for pathological staging. In case of non-muscle invasive bladder cancer, transurethral resection may itself be the treatment, but in many cases such as of muscle invasive cancer, the procedure is insufficient for final treatment ("European Association of Urology (EAU)-Guidelines-Online Guidelines". Uroweb.org. Retrieved 2015-05-07).

After recovering from surgery, patients with a lower risk of disease progression may undergo surveillance or additional intravesical chemotherapy. However, also in early stages, bladder cancer may be aggressive, such as carcinoma in situ (CIS). CIS is the most aggressive stage of non-muscular invasive bladder cancer and is often observed to be refractory to currently available treatment. Patients suffering from moderate- to high-grade disease often receive intravesical immunotherapy with a weakened, live bacterium, bacillus Calmette-Guérin (BCG).

BCG was the first FDA-approved immunotherapy and helps reduce the risk of bladder cancer recurrence by stimulating an immune response that targets the bacteria as well as any bladder cancer cells. Hence, immunotherapy in the form of Bacillus Calmette-Guérin (BCG) instillation emerged as an alternative treatment for TCC and prevention of recurrence of superficial tumors (Böhle. Recent knowledge on BCG's mechanism of action in the treatment of superficial bladder cancer. Braz J Urol 26:488 (2000); Burger et al. The application of adjuvant autologous intravesical macrophage cell therapy vs. BCG in non-muscle invasive bladder cancer: a multicenter randomized trial. J Transl Med 8:54 (2010)). For superficial bladder cancers such as TCC, BCG even became the most commonly used agent for local, e.g. intravesical, therapy and is proved to be currently the most efficacious agent for such superficial bladder cancer. BCG therapy showed to delay - although not necessarily to prevent - tumor progression to a more advanced stage, decrease the need for subsequent cystectomy, and improve overall survival. Currently, BCG is the only agent approved by the FDA as the primary therapy of carcinoma in situ of the urinary bladder. Disease-specific survival rates of 63% at 15 years with BCG compare favourably with those patients treated with cystectomy early in the course of their disease. For BCG vaccine to be effective, the host has to be immunocompetent, the tumor burden has to be small, direct contact with the tumor must occur, and the dose has to be adequate to induce an anti-tumor response. Studies consistently showed that BCG treatment can eradicate this cancer in 70% of patients with carcinoma in situ who meet these criteria. To prevent cancer recurrence, long-term maintenance therapy following the induction phase is necessary.

Typically, BCG is administered weekly for 6 weeks. Another 6-week course may be administered if a repeat cystoscopy reveals tumor persistence or recurrence. Recent evidence indicates that maintenance therapy with a weekly treatment for 3 weeks every 6 months for 1-3 years may provide more lasting results. Periodic bladder biopsies are usually necessary to assess response. While the efficacy of BCG is generally regarded as adequate, its use is debated in low and intermediate risk patients, as its limiting factor is toxicity (Babjuk M, at al. EAU Guidelines on Non-Muscle-Invasive Urothelial Carcinoma of the Bladder. Eur Urol 54(2):303 (2008); Denzinger S, et al. Versus deferred cystectomy for initial high-risk pT1G3 urothelial carcinoma of the bladder: do risk factors define feasibility of bladdersparing approach? Eur Urol 53(1):146 (2008); Witjes. Management of BCG failures in superficial bladder cancer: a review. Eur Urol 49(5):790 (2006)).

Adverse events of BCG are related to its mode of action. BCG stimulates immune reaction and local and systemic inflammatory response occurs. The most frequent immunotherapy linked adverse events include constellations of flu- and cystitis-like symptoms. Systemic toxicities, i.e. fever occur in up to 20% of patients. Due to adverse events a considerable portion of patients has been reported to discontinue BCG and many urologists reduce applications (Herr. Is maintenance Bacillus Calmette-Guérin really necessary? Eur Urol 54(5):971-3 (2008)). BCG acts via complex and diverse mechanisms by stimulating a T-cell mediated local immune response through various cytokines (Zlotta AR, et al. What are the immunologically active components of bacille Calmette-Guérin in therapy of superficial bladder cancer? Int J Cancer 87(6):844 (2000); Luo Yet al. Role of Th1-stimulating cytokines in bacillus Calmette-Guérin (BCG)-induced macrophage cytotoxicity against mouse bladder cancer MBT-2 cells. Clin Exp Immunol 146(1):181 (2006)). It thus triggers granulocyte-related anti-tumor action and macrophage cytotoxicity (Ayari C et al. Bladder tumour infiltrating mature dendritic cells and macrophages as predictors of response to bacillus Calmette-Guerin immunotherapy. Eur Urol 55(6):1386 (2009); de Reijke. Editorial comment on: Bladder tumour infiltrating mature dendritic cells and macrophages as predictors of response to bacillus Calmette-Guérin immunotherapy. Eur Urol 55(6):1395 (2009); Takayama H, et al. Increased infiltration of tumor associated macrophages is associated with poor prognosis of bladder carcinoma in situ after intravesical bacillus Calmette-Guerin instillation. J Urol 181(4):1894 (2009); Brandau. Tumour-associated macrophages: predicting bacillus Calmette-Guerin immunotherapy outcomes. J Urol 181(4):1532 (2009); Siracusano S, et al. The role of granulocytes following intravesical BCG prophylaxis. Eur Urol 51(6):1589 (2007); Brandau S, et al. The role of granulocytes following intravesical BCG prophylaxis. Eur Urol 51:1589-99 (2007)).

In summary, BCG solutions are uncharacterized products composed of an attenuated form of the bacterium *Mycobacterium tuberculosis,* and, therefore, exhibiting a poor safety profile. In addition, BCG-based immunotherapy is only effective in up to 30% of the cases at this non-invasive tumor stage. Patients whose tumors recurred after treatment with BCG are more difficult to treat. Many physicians recommend cystectomy for these patients. This recommendation is in accordance with the official guidelines of the European Association of Urologists and the American Urological Association. However, many patients refuse to undergo this life changing operation, and prefer to try novel conservative treatment options before opting to this last radical resort.

Untreated, superficial cancers may gradually begin to infiltrate the muscular wall of the bladder or other parts of the urinary tract. Consequently, cancers that infiltrate, for example, the bladder require more radical surgery. Therein, e.g. a part or the entire bladder is removed in a cystectomy, and the urinary stream is diverted into an isolated bowel loop. A harsh combination of radiation and chemotherapy may also be required to treat invasive disease forms resulting, e.g., from poorly treated non-invasive forms. Such chemotherapy is associated with severe side effects and should be avoided where possible. Furthermore, micro-metastatic disease originating from bladder cancer may occur which has implications on long-time survival. Hence, also in order to address the latter additional problem, new treatment options are needed which involve early treatment of superficial bladder cancer. Such a novel treatment option at an early stage would beneficially avoid surgery and chemotherapy. Therefore, there is an urgent need to provide an active agent suitable for treating neoplasms of the urinary tract such as neoplastic disease of the urinary bladder, especially non-invasive forms of urothelial bladder cancer, for which current limited options are mainly BCG and surgery.

Thus, there is an unmet need for bladder cancer immunotherapies other than BCG treatment. A promising approach in this context is the use of immune checkpoint inhibitors for treatment of bladder cancer. By blocking inhibitory molecules or, alternatively, activating stimulatory molecules, these treatments are designed to unleash or enhance pre-existing anti-cancer immune responses. For example, the PD-1 ligand PD-L1 is expressed by 12% of bladder tumor cells, 27% of tumor infiltrating immune cells, and in up to 50% of malignant urothelial cells in carcinoma in situ. In addition, 95% of lymphocytes that invade bladder tumors express the PD-1 receptor. Urothelial expression of PD-L1 was also predictive of mortality following cystectomy in patients with organ-limited disease. Accordingly, the PDL1/PD-1 pathway was identified as an attractive therapeutic target for the treatment of bladder cancer similar to the findings in other epithelial tumors such as renal cell cancer, lung cancer, and melanoma. An ongoing phase II trial is testing MPDL3280A as first-line treatment for advanced bladder cancer in patients not candidates for cisplatin-containing regimens or as a second-line option (NCT02108652). Blockade of CTLA-4 is also under active investigation as another immunotherapy strategy in urothelial cancers. CTLA-4 has an important role in the tumor cell mediated immunosuppression, and its blockade with the anti-CTLA-4 monoclonal antibody ipilimumab enhances T lymphocyte function resulting in meaningful tumor responses in melanoma, renal cell carcinoma, and non-small cell lung cancer. Treatment of patients with localized bladder cancer prior to cystectomy with ipilimumab demonstrated the feasibility and safety of this approach (Carthon BC, Wolchok JD, Yuan J, Kamat A, Ng Tang DS, Sun J, et al.: Preoperative CTLA-4 blockade: tolerability and immune monitoring in the setting of a presurgical clinical trial. Clin Cancer Res 2010;16(10):2861-71). Supported by these results, an ongoing phase II trial is evaluating the combination of gemcitabine, cisplatin and ipilimumab as first-line treatment of metastatic urothelial carcinoma (NCT01524991). Furthermore, based on preclinical evidence showing the potential for enhancing anti-tumor activity with combinations of immunotherapies (e.g. anti-PD-1, anti-CTLA-4, and vaccines), ongoing clinical trials are starting to investigate the safety and efficacy of combinations including anti-PD-LI, anti-CTLA-4 and OX-40 agonist in advanced solid tumors including bladder cancer (NCT02205333).

However, despite important clinical benefits, checkpoint inhibition, which "unleashes" immune responses, is associated with a unique spectrum of severe side effects termed immune-related adverse events (irAEs) or, occasionally, adverse events of special interest (Naidoo J, Page DB, Li BT, Connell LC, Schindler K, Lacouture ME, Postow MA, Wolchok JD: Toxicities of the anti-PD-1 and anti-PD-L1 immune checkpoint antibodies. Ann Oncol. 2015;26(12):2375; Champiat S, Lambotte O, Barreau E, Belkhir R, Berdelou A, Carbonnel F, Cauquil C, Chanson P, Collins M, Durrbach A, Ederhy S, Feuillet S, François H, Lazarovici J, Le Pavec J, De Martin E, Mateus C, Michot JM, Samuel D, Soria JC, Robert C, Eggermont A, Marabelle A: Management of immune checkpoint blockade dysimmune toxicities: a collaborative position paper. Ann Oncol. 2015 Dec 28. pii: mdv623. [Epub ahead of print]).

Another option is to use monospecific antibodies against tumor-associated antigens in a targeted therapy approach. One such example is ALT-801 (Altor Bioscience Corporation), which is a bifunctional fusion protein comprising interleukin-2 (IL-2) linked to a soluble, single-chain T-cell receptor domain that recognizes a peptide epitope (aa264-272) of the human p53 antigen displayed on cancer cells in the context of HLA-A*0201 (p53+/HLA-A*0201). Thereby, IL-2 is targeted to cancer cells and the activity of the immune system is enhanced. Two phase I/II trials are testing ALT-801 in combination with gemcitabine in patients with non-muscle invasive bladder cancer who have failed BCG therapy (NCT01625260) and in combination with gemcitabine and cisplatin in patients with muscle invasive bladder cancer (NCT01326871). However, this targeted immunotherapy is suggested to be limited to patients with a specific HLA type.

Another targeted therapy approach uses an antibody against the tumor-associated antigen EpCAM linked to Pseudomonas exotoxin A ("Oportuzumab monatox", also known as VB4-845) (Kowalski et al., 2012: A phase II study of oportuzumab monatox: an immunotoxin therapy for patients with noninvasive urothelial carcinoma in situ previously treated with bacillus Calmette-Guérin. J Urol 188: 1712). To achieve an effective treatment, high doses of several 100 mg of antibody drug were required per patient. Such high doses of antibodies generally bear the risk of immunologic side effects and did indeed provoked side effects in 93.5% of the patients, whereof at least 65% were directly associated with the highly dosed opotuzumab monatox. Hence, Oportuzumab monatox was not further pursued in the following.

In view of the above, there is a need for an improved immunotherapy for use in the treatment of a neoplasm of the urinary tract. It is thus the object of the present invention to overcome the drawbacks of current immunotherapies for bladder cancer outlined above and to provide a novel compound for use in the treatment of a neoplasm of the urinary tract, which improves the survival of patients suffering from a neoplasm of the urinary tract, in particular from bladder cancer, and which has a lower risk for side effects.

This object is achieved by means of the subject-matter of the appended claims.

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

The detailed technical disclosure set out below may in some respects go beyond the disclosure of the invention per se, and may also provide technical background for related technical developments. It will be appreciated that the additional technical background is not intended to define the invention (which is defined exclusively by the appended claims), but rather to place it in a broader technical context. Accordingly, it will be appreciated that the term 'embodiments' reflects a specific detail of the disclosure and that the elements of the additional technical background are not intended to define as part of the invention aspects that do not fall within the scope of the appended claims.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" e.g., a composition "comprising" X may consist exclusively of X or may include something additional e.g., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" e.g., a composition which is "substantially free" from Y may be completely free from Y. Where necessary, the word "substantially" may be omitted from the definition of the invention.

The term "about" in relation to a numerical value x means x ± 10%.

*Multispecific antibody for treatment of neoplasms of the urinary tract*

The invention to which the specification pertains is set out in the claims appended to this description.

By providing both, a specificity against a T cell surface antigen as well as a specificity against a cancer- and/or tumor-associated antigen, the multispecific antibodies, or antigen binding fragments thereof, according to the present invention are able to redirect T-cells to cancer cells. Thereby, "a specificity against a T cell surface antigen" means in particular that the antibody, or the antigen binding fragment thereof, for use according to the present invention comprises a paratope, which recognizes an epitope of a T cell surface antigen. In other words, the phrase "a specificity against a T cell surface antigen" means in particular that the antibody, or the antigen binding fragment thereof, for use according to the present invention comprises a binding site for a T cell surface antigen. Accordingly, "a specificity against a cancer- and/or tumor-associated antigen" means in particular that the antibody, or the antigen binding fragment thereof, for use according to the present invention comprises a paratope, which recognizes an epitope of a cancer- and/or tumor-associated antigen. In other words, the phrase "a specificity against a cancer- and/or tumor-associated antigen" means in particular that the antibody, or the antigen binding fragment thereof, for use according to the present invention comprises a binding site for a cancer- and/or tumor-associated antigen. Importantly, in contrast to conventional ("ordinary") antibodies exhibiting just one single specificity, multispecific antibodies are able to bind to at least two different epitopes, namely, one epitope on a cancer/tumor cell, and one epitope on a T-cell, thereby "redirecting" the T cell to the cancer/tumor cell, resulting in T-cell mediated cell killing. Accordingly, the multispecific antibodies according to the present invention exhibit T-cell redirecting properties, i.e. the antibody is typically capable of reactivating tumor-specific T cells being in the anergic state and/or direct T-cells to the desired antigen (as provided by a specificity against a cancer- and/or tumor-associated antigen of the antibody).

Furthermore, the multispecific antibody or antigen binding fragment thereof is preferably capable of inducing tumor-reactive complement-binding antibodies and therefore induces a humoral immune response. Thereby, T-cell mediated cytotoxic activity and further immunity is promoted, leading to a therapeutic effect specifically against cells bearing the targeted cancer and/or tumor-associated antigen. In consequence, potent means for use in the efficient treatment of neoplasms of the urinary tract is provided.

Such an antibody, or an antigen binding fragment thereof, according to the present invention, is potent enough to be dosed in very low quantities as compared to conventional monospecific antibodies. As shown by the examples, very low quantities of antibodies according to the present invention are indeed sufficient to achieve therapeutic effects in patients with bladder cancer, i.e. in an adverse milieu entirely different from, e.g., blood. This is surprising since multispecific, e.g., bispecific, antibodies, or antigen binding fragments thereof, are generally considered to be more prone to loose functionality, e.g., due to adverse pH or electrolyte conditions, as compared to conventional monospecific antibodies. The reason may be that - in contrast to conventional monospecific antibodies - multispecific antibodies have preferably only one single paratope regarding each specificity (i.e. exactly one paratope for each specificity). For example, when considering bivalent antibodies (i.e. antibodies having two paratopes), a conventional monospecific antibody has two paratopes with the same specificity, thereby providing redundancy, whereas a bispecific, bivalent antibody has only one paratope for each specificity. Thus, when the functionality of one paratope were lost (e.g., due to adverse pH or electrolyte conditions as in urine milieu), the monospecific antibody still has another paratope, whereas the bispecific antibody loses its entire functionality (such as redirecting T-cells to cancer cells). Also, considering the much lower dosages of multispecific antibodies, or antigen binding fragments thereof, compared to common dosages of monospecific antibodies and antigen binding fragments thereof, it is furthermore surprising that dilution effects in the urine did not impair sufficient therapeutic concentrations in the urinary tract.

On the other hand, the low dosing considerably reduces the risk for side effects. Accordingly, the multispecific antibody, or the antigen binding fragment thereof, according to the present invention contributes to a reduction of the release of pro-inflammatory cytokines. Of note, release of pro-inflammatory cytokines is often observed in the environment of cancers and/or tumors. Accordingly, chronic inflammation, which is often observed in the environment of cancers and/or tumors, could be reduced during the course of the administration of the inventive antibody of the present invention. In particular, treatment by using the multispecific antibody, or antigen binding fragment thereof, according to the present invention leads to a reduction of the leucocytes detectable in the urine, as shown in the examples. This proves that inflammatory signs being otherwise typical for neoplasm of the urinary tract, in particular bladder cancer, are reduced or eradicated - which in turn demonstrates the curing potency of the antibody according to the present invention.

Moreover, the therapeutic efficiency of the antibody according to the present invention lasts at least several months and in the undesired case of a relapse, a subsequent treatment cycle with the present multispecific antibody or antigen binding fragment thereof is able to revert said relapse without the need of surgery or even chemotherapy as it is commonly the case with relapses under BCG treatment.

As used herein, the term "antibody" encompasses various forms of antibodies, preferably monoclonal antibodies including, but not being limited to, whole antibodies, antibody fragments, human antibodies, chimeric antibodies, humanized antibodies and genetically engineered antibodies (variant or mutant antibodies) as long as the characteristic properties according to the invention are retained. Human or humanized monoclonal antibodies and recombinant antibodies, in particular recombinant monoclonal antibodies, are preferred. Thus, the antibody, or antigen binding fragment thereof, according to the present invention is preferably a monoclonal antibody, or antigen binding fragment thereof. Moreover, it is also preferred that the antibody is a multichain antibody, i.e. an antibody comprising more than one chain, which is thus different from a single chain antibody.

The term "human antibody", as used herein, is intended to include antibodies having variable and constant regions derived from human immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 3340). Human antibodies can also be produced in phage display libraries (Hoogenboom, H. R., and Winter, G., J. Mol. Biol. 227 (1992) 381-388; Marks, J. D., et al., J. Mol. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95). The term "human antibody" as used herein also comprises such antibodies which are modified, e.g. in the variable region, to generate the properties according to the invention.

As used herein, the term "recombinant antibody" is intended to include all antibodies, which do not occur in nature, in particular antibodies that are prepared, expressed, created or isolated by recombinant means, such as antibodies isolated from a host cell such as for example a CHO cell or from an animal (e.g. a mouse) or antibodies expressed using a recombinant expression vector transfected into a host cell. Such recombinant antibodies have variable and constant regions in a rearranged form as compared to naturally occurring antibodies.

As used herein, the terms "antigen binding fragment," "fragment," and "antibody fragment" are used interchangeably to refer to any fragment of an antibody of the invention that retains the specific binding activity of the antibody for use according to the invention, in particular the specificity against a T cell surface antigen and the specificity against a cancer- and/or tumor-associated antigen. Examples of antibody fragments include, but are not limited to, a single chain antibody, Fab, Fab', $F(ab')_2$, Fv or scFv. Fragments of the antibodies of the invention can be obtained from the antibodies by methods that include digestion with enzymes, such as pepsin or papain, and/or by cleavage of disulfide bonds by chemical reduction. Alternatively, fragments of antibodies can be obtained by cloning and expression of part of the sequences of the heavy and/or light chains. "Fragments" include, but are not limited to, Fab, Fab', F(ab')2 and Fv fragments. The invention also encompasses single-chain Fv fragments (scFv) derived from the heavy and light chains of an antibody of the invention. For example, the invention includes a scFv comprising the CDRs from an antibody of the invention. Also included are heavy or light chain monomers and dimers, single domain heavy chain antibodies, single domain light chain antibodies, as well as single chain antibodies, e.g., single chain Fv in which the heavy and light chain variable domains are joined by a peptide linker. Antibody fragments of the invention may impart monovalent or multivalent interactions and be contained in a variety of structures as described above. For instance, scFv molecules may be synthesized to create a trivalent "triabody" or a tetravalent "tetrabody." The scFv molecules may include a domain of the Fc region resulting in bivalent minibodies. In addition, the sequences of the invention may be a component of multispecific molecules in which the sequences of the invention target the epitopes of the invention and other regions of the molecule bind to other targets. Exemplary molecules include, but are not limited to, bispecific Fab2, trispecific Fab3, bispecific scFv, and diabodies (Holliger and Hudson, 2005, Nature Biotechnology9: 1126-1136). Although the specification, including the claims, may, in some places, refer explicitly to antigen binding fragment(s), antibody fragment(s), variant(s) and/or derivative(s) of antibodies, it is understood that the term "antibody" or "antibody of the invention" includes all categories of antibodies, namely, antigen binding fragment(s), antibody fragment(s), variant(s) and derivative(s) of antibodies.

As used herein, the term "multispecific" refers to the ability to bind to at least two different epitopes, e.g. on different antigens, such as on a T cell surface antigen and on a cancer/tumor antigen. Thus, terms like "bispecific", trispecific", "tetraspecific" etc. refer to the number of different epitopes to which the antibody can bind to. For example, conventional monospecific IgG-type antibodies have two identical epitope binding sites (paratopes) and can, thus, only bind to identical epitopes (but not to different epitopes). A multispecific antibody, in contrast, has at least two different types of paratopes and can, thus, bind to at least two different epitopes. As used herein, "paratope" refers to an epitope-binding site of the antibody. Moreover, a single "specificity" may refer to one, two, three or more identical paratopes in a single antibody (the actual number of paratopes in one single antibody molecule is referred to as "valency"). For example, a single native IgG antibody is monospecific and bivalent, since it has two identical paratopes. Accordingly, a multispecific antibody comprises at least two (different) paratopes. Thus, the term "multispecific antibodies" refers to antibodies having more than one paratope and the ability to bind to two or more different epitopes. The term "multispecific antibodies" comprises in particular bispecific antibodies as defined above, but typically also protein, e.g. antibody, scaffolds, which bind in particular to three or more different epitopes, i.e. antibodies with three or more paratopes.

In particular, the multispecific antibody, or the antigen binding fragment thereof, may comprise two or more paratopes, wherein some paratopes may be identical so that all paratopes of the antibody belong to at least two different types of paratopes and, hence, the antibody has at least two specificities. For example, the multispecific antibody or antigen binding fragment thereof according to the present invention may comprise four paratopes, wherein each two paratopes are identical (i.e. have the same specificity) and, thus, the antibody or fragment thereof is bispecific and tetravalent (two identical paratopes for each of the two specificities). Thus, "one specificity" refers in particular to one or more paratopes exhibiting the same specificity (which typically means that such one or more paratopes are identical) and, thus, "two specificities" may be realized by two, three, four five, six or more paratopes as long as they refer to only two specificities. Most preferably a multispecific antibody comprises one single paratope for each (of the at least two) specificity, i.e. the multispecific antibody comprises in total at least two paratopes. For example, a bispecific antibody comprises one single paratope for each of the two specificities, i.e. the antibody comprises in total two paratopes. It is also preferred that the antibody comprises two (identical) paratopes for each of the two specificities, i.e. the antibody comprises in total four paratopes. Preferably the antibody comprises three (identical) paratopes for each of the two specificities, i.e. the antibody comprises in total six paratopes.

As used herein, the term "antigen" refers to any structural substance which serves as a target for the receptors of an adaptive immune response, in particular as a target for antibodies, T cell receptors, and/or B cell receptors. An "epitope", also known as "antigenic determinant", is the part (or fragment) of an antigen that is recognized by the immune system, in particular by antibodies, T cell receptors, and/or B cell receptors. Thus, one antigen has at least one epitope, i.e. a single antigen has one or more epitopes. An antigen may be (i) a peptide, a polypeptide, or a protein, (ii) a polysaccharide, (iii) a lipid, (iv) a lipoprotein or a lipopeptide, (v) a glycolipid, (vi) a nucleic acid, or (vii) a small molecule drug or a toxin. Thus, an antigen may be a peptide, a protein, a polysaccharide, a lipid, a combination thereof including lipoproteins and glycolipids, a nucleic acid (e.g. DNA, siRNA, shRNA, antisense oligonucleotides, decoy DNA, plasmid), or a small molecule drug (e.g. cyclosporine A, paclitaxel, doxorubicin, methotrexate, 5-aminolevulinic acid), or any combination thereof. Preferably, the antigen is selected from (i) a peptide, a polypeptide, or a protein, (ii) a polysaccharide, (iii) a lipid, (iv) a lipoprotein or a lipopeptide and (v) a glycolipid; more preferably, the antigen is a peptide, a polypeptide, or a protein.

As used herein, "(an epitope of) a cancer- and/or tumor-associated antigen" refers to (an epitope of) a cancer-associated antigen, a cancer-specific antigen, a tumor-associated antigen and/or a tumor-specific antigen. Such epitopes/antigens are typically specific for or associated with a certain kind of cancer/tumor. Suitable cancer/tumor epitopes and antigens can be retrieved for example from cancer/tumor epitope databases, e.g. from van der Bruggen P, Stroobant V, Vigneron N, Van den Eynde B. Peptide database: T cell-defined tumor antigens. Cancer Immun 2013; URL: http://www.cancerimmunity.org/peptide/, wherein human tumor antigens are classified into four major groups on the basis of their expression pattern, or from the database "Tantigen" (TANTIGEN version 1.0, Dec 1, 2009; developed by Bioinformatics Core at Cancer Vaccine Center, Dana-Farber Cancer Institute; URL: http://cvc.dfci.harvard.edu/tadb/). Specific examples of cancer-related, in particular tumor-related, or tissue-specific antigens useful in the context of the present invention include, but are not limited to, the following antigens: Epha2, Epha4, PCDGF, HAAH, Mesothelin; EPCAM; NY-ESO-1, glycoprotein MUC1 and NIUC10 mucins p5 (especially mutated versions), EGFR; cancer antigen 125 (CA 125), the epithelial glycoprotein 40 (EGP40) (Kievit et al., 1997, Int. J. Cancer 71: 237-245), squamous cell carcinoma antigen (SCC) (Lozza et al., 1997 Anticancer Res. 17: 525-529), cathepsin E (Mota et al., 1997, Am. J Pathol. 150: 1223-1229), CDC27 (including the mutated form of the protein), antigens triosephosphate isomerase, 707-AP, A60 mycobacterial antigen (Macs et al., 1996, J. Cancer Res. Clin. Oncol. 122: 296-300), AFP, alpha(v)beta(3)-integrin, ART-4, ASC, BAGE, β-catenin/m, BCL-2, bcr-abl, bcr-abl p190, bcr-abl p210, BRCA-1, BRCA-2, CA 19-9 (Tolliver and O'Brien, 1997, South Med. J. 90: 89-90; Tsuruta at al., 1997 Urol. Int. 58: 20-24), CA125, CALLA, CAMEL, carbonic anhydrase, CAP-1, CASP-8, CDC27/m, CDK-4/m, CD1, CD2, CD4, CD6, CD7, CD8, CD11, CD13, CD14, CD19, CD20, CD21, CD22, CD23, CD24, CD30 CD33, CD37, CD38, CD40, CD41, CD44v3, CD44v6, CD47, CD52, CEA (Huang et al., Exper Rev. Vaccines (2002)1:49-63), c-erb-2, CT9, CT10, Cyp-B, Dek-cain, DAM-6 (MAGE-B2), DAM-10 (MAGE-B1), EphA2 (Zantek et al., Cell Growth Differ. (1999) 10:629-38; Carles-Kinch et al., Cancer Res. (2002) 62:2840-7), EphA4 (Cheng at al., 2002, Cytokine Growth Factor Rev. 13:75-85), tumor associated Thomsen-Friedenreich antigen (Dahlenborg et al., 1997, Int. J Cancer 70: 63-71), ELF2M, ETV6-AML1, G250, GAGE-1, GAGE-2, GAGE-3, GAGE-4, GAGE-5, GAGE-6, GAGE-7B, GAGE-8, GD1a, GD1b, GD2, GD3, GnT-V, GM1, GM2, GM3, gp100 (Zajac et al., 1997, Int. J Cancer 71: 491-496), GT1b, GT3, GQ1, HAGE, HER2/neu, HLA, HLA-DR, HLA-A*0201-R170I, HPV-E7, HSP-27, HSP-70, HSP70-2M, HSP-72, HSP-90, HST-2, hTERT, hTRT, iCE, inhibitors of apoptosis such as survivin, KH-1 adenocarcinoma antigen (Deshpande and Danishefsky, 1997, Nature 387: 164-166), KIAA0205, K-ras, LAGE, LAGE-1, LDLR/FUT, Lewis Y antigen, MAGE-1, MAGE-2, MAGE-3, MAGE-6, MAGE-A1, MAGE-A2, MAGE-A3, MAGE-A4, MAGE-A6, MAGE-A10, MAGE-A12, MAGE-B5, MAGE-B6, MAGE-C2, MAGE-C3, MAGE D, MART-1, MART-1/Melan-A (Kawakami and Rosenberg, 1997, Int. Rev. Immunol. 14: 173-192), MC1R, MCSP, MDM-2, MHCII, mTOR, Myosin/m, MUC1, MUC2, MUM-1, MUM-2, MUM-3, neo-polyA polymerase, NA88-A, NFX2, NY-ESO-1, NY-ESO-1a (CAG-3), PAGE-4, PAP, Proteinase 3 (Molldrem et al., Blood (1996) 88:2450-7; Molldrem et al., Blood (1997) 90:2529-34), P15, p53, p97, p190, PD-L1, Pgp, PIK3CA, Pm1/RARα, PRAME, proteoglycan, PSA, PSM, PSMA, RAGE, RAS, RCAS1, RU1, RU2, SAGE, SART-1, SART-2, SART-3, SP17, SPAS-1, SSX2, SSX4 TEL/AML1, TPI/m, Tyrosinase, TARP, telomerase, TRP-1 (gp75), TRP-2, TRP-2/INT2, VEGF, WT-1, Wue antigen, cell surface targets GC182, GT468 or GT512, and alternatively translated NY-ESO-ORF2 and CAMEL proteins, derived from the NY-ESO-1 and LAGE-1 genes. A further specific example of a cancer-related, in particular tumor-related, or tissue-specific antigen useful in the context of the present invention is CD133.

Antigens described in Jones et al., 1997, Anticancer Res. 17: 685-687 are particularly preferred in the context of bladder cancers such as TCC. Numerous other cancer antigens are well known in the art. In particular, urothelial cells may comprise several (surface) structures, which are overexpressed in a neoplasm and can serve as cancer- and/or tumor-specific antigens. Accordingly, such antigens relating to a neoplasm of urothelial cells are preferred.

As used herein, "(an epitope of) a T cell surface antigen" refers to (an epitope from) a T cell surface-associated antigen or a T cell surface-specific antigen (also known as "T cell surface markers"). These are in particular "CD" (cluster of differentiation) molecules specific for T cells. CD molecules are cell surface markers useful for the identification and characterization of leukocytes. The CD nomenclature was developed and is maintained through the HLDA (Human Leukocyte Differentiation Antigens) workshop started in 1982. Whether or not a certain CD molecule is found on T cells (and, thus, represents a T cell surface antigen in the context of the present invention) may be retrieved, for example, from a variety of sources known to the person skilled in the art, such as http://www.ebioscience.com/resources/human-cd-chart.htm, BD Bioscience's "Human and Mouse CD Marker Handbook" (retrievable at https://www.bdbiosciences.com/documents/cd_marker_handbook.pdf) or from www.hcdm.org. Accordingly, examples of T cell surface antigens include for example those (human) CD markers positively indicated for T cells in the BD Bioscience's "Human and Mouse CD Marker Handbook" (retrievable at https://www.bdbiosciences.com/documents/cd_marker_handbook.pdf) or in other sources of "CD marker charts".

The antibody, or the antigen binding fragment thereof, according to the present invention is used in the treatment of a neoplasm of the urinary bladder.

The term "neoplasm" as used herein refers to any abnormal growth of tissue. Such abnormal growth (neoplasia) usually but not always forms a mass. If it forms a mass, it is referred to as "tumor". In particular, a tumor is a solid or fluid-filled cystic lesion that may or may not be formed by an abnormal growth of neoplastic cells and that appears enlarged in size. Neoplasms in the context of the present invention may or may not form a tumor. In particular, leukemia and most forms of carcinoma in situ (CIS) do not form a tumor. Tumor is also not synonymous with cancer. While cancer is by definition malignant, a tumor may be benign, precancerous, or malignant.

In general, neoplasms are classified into four major groups: benign neoplasms, in situ neoplasms, malignant neoplasms and neoplasms of uncertain or unknown behavior.

Malignant neoplasms are also known as "cancers". In particular, a neoplasm can be benign, potentially malignant (pre-cancer), or malignant (cancer). Benign tumors include uterine fibroids and melanocytic nevi (skin moles). They are circumscribed and localized and do not transform into cancer. Potentially-malignant neoplasms include carcinoma in situ. They are localized, do not invade and destroy but in time, may transform into a cancer. Malignant neoplasms are commonly called cancer. They invade and destroy the surrounding tissue, may form metastases and, if untreated or unresponsive to treatment, will prove fatal. Secondary neoplasm refers to any of a class of cancerous tumor that is either a metastatic offshoot of a primary tumor, or an apparently unrelated tumor that increases in frequency following certain cancer treatments such as chemotherapy or radiotherapy. Rarely there can be a metastatic neoplasm with no known site of the primary cancer and this is classed as a cancer of unknown primary origin.

In the context of the present invention, the neoplasm is preferably potentially malignant (pre-cancer), such as carcinoma in situ, or malignant (cancer).

As used herein, the term "urinary tract" (also known as "urinary system") is understood to comprise the kidneys, the ureters, the bladder and the urethra. The urinary tract typically refers to the structures that produce and conduct urine to the point of excretion.

Preferably, the antibody, or the antigen binding fragment thereof, for use according to the present disclosure may be administered systemically or locally into the urinary bladder, preferably via instillation.

An antibody, or the antigen binding fragment thereof, can be administered by various routes of administration, for example, systemically or locally. Routes for systemic administration in general include, for example, transdermal, oral and parenteral routes, which include subcutaneous, intravenous, intramuscular, intraarterial, intradermal and intraperitoneal routes and/or intranasal administration routes. Routes for local administration in general include, for example, topical administration routes, but also administration epithial directly at the site of affliction, such as intratumoral administration.

The antibody, or the antigen binding fragment thereof, for use according to the present invention is administered via instillation, e.g. locally to the urinary tract by instillation such as intravesical instillation. Instillation may be facilitated by any means of administration known to the skilled person, e.g. by catheterization to reach the inner space of the urethra or the urinary bladder.

Most preferably, the antibody, or the antigen binding fragment thereof, for use according to the present invention is administered intravesically.

Intravesical administration means in particular administration directly into the urinary bladder, preferably by using a catheter, such as a urethral catheter. Lidocaine Jelly (2%) Urojet may be used or not used. Intravesical administration is preferably by instillation. Preferably, the bladder is emptied directly before intravesical treatment, such that the antibody, or the antigen binding fragment thereof, is administered into the empty bladder. After intravesical administration, the patient is preferably instructed to attempt to retain the treatment for at least one hour, more preferably for at least two hours. In other words, the patient is preferably instructed to wait for at least one hour, more preferably for at least two hours, after administration before emptying the bladder.

The target area of intravesical administration may be any inner part of the urinary bladder including for example delivery into the void space of the urinary bladder or into the urothelium. Usually, the term "intravesical administration" as used herein refers to a delivery into the void space of the urinary bladder. Therein, the instilled liquid may disperse in urine if present or directly coat the inner walls of the urinary bladder. The antibody, or the antigen binding fragment thereof, as described herein is typically functional in urine environment at least for the time required to act on the desired site of pharmacological action within the urinary bladder.

Surprisingly, the antibody, or the antigen binding fragment thereof, as described herein is functional even in an adverse urine milieu and, thus, able to bind to specific antigens in the preferred target tissue, e.g. in the urothelium, upon intravesical administration. Moreover, it is thought that certain antigens associated with a cancer and/or a tumor of the urinary tract, such as EpCAM, are for example located on or close to the basal membrane of the urothelium. Under physiologic conditions, these antigens are in general not overexpressed, and tight urothelium cells make them difficult to access. However, under neoplastic conditions, in particular in malignant or in situ neoplasms such as in case of TCC, affected cells typically become more permeable, which makes the basal membrane more accessible. Thereby, the potency and efficiency of the antibody, or antigen binding fragment thereof, as described herein, is preferably enhanced.

Moreover, intravesical administration of the antibody, or the antigen binding fragment thereof, as described herein acts preferably only locally, and, thus, systemic effects are avoided. In particular, adverse side effects, which are more likely upon systemic administration, are avoided. In particular, systemic release of pro-inflammatory cytokines, such as IL-6, IL-8, IFN-$\gamma$ and TNF-$\alpha$, is considerably reduced, thereby avoiding undesired side effects like fever, nausea, headache and symptoms of an undesired generalized immune reaction such as redness, itching and even anaphylactic shock. Moreover, undesired HAMA (human anti-mouse antibody) or ADA (anti-drug antibody) reactions are preferably also avoided. Moreover, it is known that the antibody, or the antigen binding fragment thereof, as described herein, e.g. catumaxomab, is immunogenic when administered intraperitoneally (Heiss et al. The trifunctional antibody catumaxomab for the treatment of malignant ascites due to epithelial cancer: results of a prospective randomized phase II/III trial. Int. J Cancer 127: 2209-2221 (2010); Ott et al. Humoral response to catumaxomab correlates with clinical outcome: results of the pivotal phase II/III study in patients with malignant ascites. Int. J Cancer 130:2195-2203 (2012)). However, the intravesical administration of the antigen binding fragment thereof, as described herein, e.g. catumaxomab, is typically not immunogenic - most likely due to the fact that the drug did not become systemic.

Due to avoidance of side effects, which are more likely upon systemic administration, even higher amounts of the antibody, or antigen binding fragment thereof, as described herein may be administered intravesically if required. On the other hand, intravesical administration may enable the reduction of the required dosage due to presence of the active agent directly on the desired site of action. Accordingly, low to very low amounts of the antibody, or the antigen binding fragment thereof, as described herein are sufficient to ensure therapeutic efficiency - despite the possible high dilution in the bladder due to the urine.

In addition, the antibody, or the antigen binding fragment thereof, as described herein does preferably not require a complex formulation to be applicable for intravesical administration. In other words, the antibody, or the antigen binding fragment thereof, as described herein is preferably stable in an adverse urine environment, in particular upon local administration, e.g. intravesical administration.

Preferably, the neoplasm to be treated with the antigen binding fragment thereof, as described herein, is an in situ neoplasm or a malignant neoplasm, more preferably the neoplasm is a malignant neoplasm.

In the context of the present invention, the term "malignant neoplasm" (also referred to as malignancy) refers in general to cancer. In contrast to a benign neoplasm, a malignant neoplasm is typically not self-limited in its growth, is typically capable of invading into adjacent tissues, and may be capable of spreading to distant tissues.

An in situ neoplasm is also referred to as "carcinoma in situ" (CIS). In situ neoplasms are potentially malignant. In contrast to a malignant neoplasm, in situ neoplasms are localized, do not invade and destroy, but may eventually transform into cancer. Typically, a carcinoma in situ is a group of abnormal cells, which preferably grow in their normal place - thus "*in situ*". Malignancy is thus typically characterized by one or more of anaplasia, invasiveness, and metastasis.

In the context of the urinary tract, in particular urothelial CIS is preferred. Urothelial CIS is a high-grade neoplasm and an indicator of recurrence and progression that requires specific treatment. In particular, urothelial CIS is a flat non-invasive high grade urothelial neoplasm. High grade and severe dysplasia as well as some moderate dysplasia are included in carcinoma in situ. While non-invasive papillary urothelial neoplasms are technically also in situ, they are not referred to as carcinoma in situ. Importantly, CIS typically justifies intravesical chemotherapy or cystectomy.

In the "Classification of Malignant Tumors" (TNM), CIS is typically reported as TisN0M0 (Stage 0). The TNM is a cancer staging notation system that gives codes to describe the stage of a person's cancer, when this originates with a solid tumor. "T" describes the size of the original (primary) tumor and whether it has invaded nearby tissue, "N" describes nearby (regional) lymph nodes that are involved, and "M" describes distant metastasis (spread of cancer from one part of the body to another). Further stages are T1, T2, T3, T4, depending on size and/or extension of the primary tumor.

For example, in bladder cancer, the TNM staging system includes the following stages for primary tumors ("T" stages): TX - Primary tumour cannot be assessed, T0 - No evidence of primary tumour, Ta - Non-invasive papillary carcinoma, Tis - Carcinoma in situ ('flat tumour'), T1 - Tumour invades subepithelial connective tissue, T2a - Tumour invades superficial muscle (inner half), T2b - Tumour invades deep muscle (outer half), T3 - Tumour invades perivesical tissue: T3a - Microscopically and T3b - Macroscopically (extravesical mass), T4a - Tumour invades prostate, uterus or vagina and T4b - Tumour invades pelvic wall or abdominal wall; following stages for lymph nodes ("N" stages): NX - Regional lymph nodes cannot be assessed, N0 - No regional lymph node metastasis, N1 - Metastasis in a single lymph node 2 cm or less in greatest dimension, N2 - Metastasis in a single lymph node more than 2 cm but not more than 5 cm in greatest dimension,or multiple lymph nodes, none more than 5 cm in greatest dimension and N3 - Metastasis in a lymph node more than 5 cm in greatest dimension; and the following stages for distant metastasis ("M" stages): MX - Distant metastasis cannot be assessed, M0 - No distant metastasis and M1 - Distant metastasis (Longe, Jacqueline L. (2005). Gale Encyclopedia Of Cancer: A Guide To Cancer And Its Treatments. Detroit: Thomson Gale. p. 137). This stages can be integrated into the following numerical staging of bladder cancer: Stage Oa: Ta, N0, M0; Stage 0 is: Tis, N0, M0; Stage I: T1, N0, M0; Stage II: T2a or T2b, N0, M0; Stage III: T3a, T3b, or T4a, N0, M0; and Stage IV: any of the following: T4b, N0, M0; any T, N1 to N3, M0 or any T, any N, M1.

Preferably, the antibody, or the antigen binding fragment thereof, is used in the treatment of a neoplasm of the urothelium. The invention pertains specifically to the treatment of a neoplasm of the urinary bladder, as set out in claim 1 appended to this description.

The urothelium is a "transitional epithelium" and lines much of the urinary tract including the renal pelvis, the ureters, the bladder and parts of the urethra. Urothelial tissue is highly specific to the urinary tract, and has high elasticity and trans-epithelial electrical resistance. The urothelium typically consists of approximately 3-5 cell layers, accompanied by a thick layer of protective glycoprotein plaques at its luminal (apical) surface.

It is generally understood that the urothelium is susceptible to neoplasms, in particular to carcinoma. "Carcinoma" refers to a type of cancer developing from epithelial cells, such as urothelial cells. In general, a carcinoma is typically a cancer that begins in a tissue that lines the inner or outer surfaces of the body, and that generally arises from cells originating in the endodermal or ectodermal germ layer during embryogenesis.

A particularly preferred neoplasm of the urothelium is transitional cell carcinoma (TCC; also known as urothelial cell carcinoma, UCC). Accordingly, the antibody, or the antigen binding fragment thereof, is preferably used in the treatment of urothelial cell carcinoma (transitional cell carcinoma). "Transitional" refers to the histological subtype of the cancerous cells as seen under a microscope. TCC typically occurs in the urinary system: in the kidney, in the urinary bladder, and in accessory organs. TCC is the most common type of bladder cancer and cancer of the ureter, urethra, and urachus. TCC is the second most common type of kidney cancer. TCC arises from the urothelium, the tissue lining the inner surface of the urinary tract, and can extend from the kidney collecting system to the bladder ("Creeping Tumor"). TCCs are often multifocal, with 30-40% of patients having more than one tumor at diagnosis. The pattern of growth of TCCs can be papillary, sessile (flat) or carcinoma in situ. The 1973 WHO grading system for TCCs (papilloma, G1, G2 or G3) is most commonly used despite being superseded by the 2004 WHO grading (papillary neoplasm of low malignant potential [PNLMP], low grade, and high grade papillary carcinoma).

Preferably, the antibody, or the antigen binding fragment thereof, is used for the treatment of a neoplasm of the urinary tract selected from the group consisting of (i) carcinoma in situ, preferably carcinoma in situ of the urethra, carcinoma in situ of the urinary bladder, carcinoma in situ of the ureter and/or carcinoma in situ of the renal pelvis; (ii) non-muscular invasive urothelial cancer, preferably localized at the renal pelvis, at the ureter, at the urethra, at the trigone of bladder, at the dome of bladder, at the lateral wall of bladder, at the anterior wall of bladder, at the posterior wall of bladder, at the bladder neck, at the ureteric orifice and/or at the urachus; and (iii) muscular invasive urothelial cancer, preferably localized at the renal pelvis, at the ureter, at the urethra, at the trigone of bladder, at the dome of bladder, at the lateral wall of bladder, at the anterior wall of bladder, at the posterior wall of bladder, at the bladder neck, at the ureteric orifice and/or at the urachus.

It is also preferred that the antibody, or the antigen binding fragment thereof, is used for the treatment of a lymphoma and/or a sarcoma localized in the urinary tract.

In particular, it is preferred that the antibody, or the antigen binding fragment thereof, is used for the treatment of transitional cell carcinoma, squamous cell carcinoma, adenocarcinoma, sarcoma, small cell carcinoma, and a secondary deposit from a cancer elsewhere in the body in the urinary tract. TCC are particularly preferred.

Preferably, the neoplasm of the urinary tract is a neoplasm of the lower urinary tract. The lower urinary tract comprises in particular the urinary bladder with all its structural and functional sub-parts and the urethra - but not the kidneys and the ureters, which form the upper urinary tract. The lower urinary tract is in particular accessible by intravesical administration.

Thereby, it is preferred that the antibody, or the antigen binding fragment thereof, is used for the treatment of a neoplasm of the lower urinary tract is selected from the group consisting of (i) carcinoma in situ of the urethra, and/or carcinoma in situ of the urinary bladder; (ii) non-muscular invasive urothelial cancer localized at the urethra, at the trigone of bladder, at the dome of bladder, at the lateral wall of bladder, at the anterior wall of bladder, at the posterior wall of bladder, at the bladder neck, at the ureteric orifice and/or at the urachus; and (iii) muscular invasive urothelial cancer localized at the urethra, at the trigone of bladder, at the dome of bladder, at the lateral wall of bladder, at the anterior wall of bladder, at the posterior wall of bladder, at the bladder neck, at the ureteric orifice and/or at the urachus.

Most preferably, the antibody, or the antigen binding fragment thereof, is used for the treatment of a neoplasm of the urinary bladder, preferably a carcinoma in situ of the urinary bladder or a malignant neoplasm of the urinary bladder. In particular, the antibody, or the antigen binding fragment thereof, is used for the treatment of bladder cancer. Despite the term "cancer", bladder cancer includes all numerical stages as described above, and, thus, a preferred stage of bladder cancer may be selected from the group consisting of (i) Stage Oa: Ta, N0, M0 (the cancer is a non-invasive papillary carcinoma (Ta) and has grown toward the hollow center of the bladder but has not grown into the connective tissue or muscle of the bladder wall; it has not spread to nearby lymph nodes (N0) or distant sites (M0)); (ii) Stage 0is: Tis, N0, M0 (the cancer is a flat, non-invasive carcinoma (Tis), also known as flat carcinoma in situ (CIS), and it is growing in the inner lining layer of the bladder only; it has not grown inward toward the hollow part of the bladder, nor has it invaded the connective tissue or muscle of the bladder wall; it has not spread to nearby lymph nodes (N0) or distant sites (M0)); (iii) Stage I: T1, N0, M0 (the cancer has grown into the layer of connective tissue under the lining layer of the bladder but has not reached the layer of muscle in the bladder wall (T1); the cancer has not spread to nearby lymph nodes (N0) or to distant sites (M0)); (iv) Stage II: T2a or T2b, N0, M0 (the cancer has grown into the thick muscle layer of the bladder wall, but it has not passed completely through the muscle to reach the layer of fatty tissue that surrounds the bladder (T2); the cancer has not spread to nearby lymph nodes (N0) or to distant sites (M0)); (v) Stage III: T3a, T3b, or T4a, N0, M0 (the cancer has grown into the layer of fatty tissue that surrounds the bladder (T3a or T3b); it might have spread into the prostate, uterus, or vagina, but it is not growing into the pelvic or abdominal wall (T4a); the cancer has not spread to nearby lymph nodes (N0) or to distant sites (M0)); and (vi) Stage IV: any of the following: T4b, N0, M0 (the cancer has grown through the bladder wall and into the pelvic or abdominal wall (T4b); the cancer has not spread to nearby lymph nodes (N0) or to distant sites (M0)); any T, N1 to N3, M0 (the cancer has spread to nearby lymph nodes (N1-N3) but not to distant sites (MO)) or any T, any N, M1 (the cancer has spread to distant lymph nodes or to sites such as the bones, liver, or lungs (M1)). More preferably, the antibody, or the antigen binding fragment thereof, is used in the treatment of any of Stage 0a, Stage 0is, Stage I or Stage II, in particular in the treatment of bladder cancer of any of Stage 0a, Stage 0is, Stage I or Stage II. Particularly preferably, the antibody, or the antigen binding fragment thereof, is used in the treatment of Stage 0a, in particular in the treatment of bladder cancer of Stage Oa. Particularly preferably, the antibody, or the antigen binding fragment thereof, is used in the treatment of Stage 0is, in particular in the treatment of bladder cancer of Stage 0is. Particularly preferably, the antibody, or the antigen binding fragment thereof, is used in the treatment of Stage I, in particular in the treatment of bladder cancer of Stage I. Particularly preferably, the antibody, or the antigen binding fragment thereof, is used in the treatment of Stage II, in particular in the treatment of bladder cancer of Stage II.

Preferred examples of bladder cancer include carcinoma in situ of the bladder, non-invasive, invasive and metastatic transitional cell carcinoma and non-transitional cell carcinoma of the bladder. Thus, the neoplasm of the urinary bladder is selected from transitional cell carcinoma, squamous cell carcinoma, adenocarcinoma, sarcoma, small cell carcinoma, and a secondary deposit from a cancer elsewhere in the body, preferably the neoplasm of the urinary bladder is a transitional cell carcinoma. Bladder CIS tends to be a far more malignant process with greater metastatic potential than some large low grade tumors that have begun to invade through the bladder wall.

To determine the aggressiveness of transitional cell tumors, the lamina propria is a useful landmark. The lamina propria is a layer of connective tissue and cells in the bladder wall between the transitional cell layer (urothelium) and the muscle fibers. As abnormal cells continue to multiply, more mutations in their genetic machinery tend to occur. Ordinarily, these mutations might be repaired, but that ability is limited in these cells. Eventually, genetic changes and further growth results in the cells' ability to destroy and penetrate the underlying lamina propria. This is the beginning of an invasive transitional cell tumor. Accordingly, the difference between "superficial" (non-invasive) TCC and "(muscle-)invasive" TCC is an important distinction. Essentially, any tumor that has not invaded the muscle layer is considered superficial (non-invasive). Once the muscle layer has been breached, however, the diagnosis is muscle-invasive TCC. This distinction is critical because it predicts the natural history of these tumors. Superficial TCC tends to recur multiply, but the recurrences are almost always superficial tumors that respond well to local resection. Only 15% of superficial tumors will transform or recur as high grade, invasive lesions. On the hand, recurrent CIS lesions or high grade/invasive tumors are much more difficult to control, and are more likely to result in metastatic spread.

Several tumors other than TCC can also develop within the bladder, and are, thus, also preferred in the context of the present invention. Those include squamous cell carcinoma (SCC). Long-term irritation of the bladder by infectious agents or foreign bodies can cause the transitional epithelium to change into a different cell type known as squamous or "flat" cells. Moreover, any of the various different cell types within the bladder can theoretically develop into cancers: muscle cells (rhabdomyosarcoma), gland cells (adenocarcinoma), nerve cells (neural cell tumors), and even immune-type cells (lymphomas). Tumors arising from adjacent organs can also invade into the bladder and appear as "bladder tumor" (e.g. cervical carcinoma or colon cancers). Accordingly, any of the above bladder cancers may treated with the antibody, or the antigen binding fragment thereof, as described herein.

Preferably, the antibody, or the antigen binding fragment thereof, is used in a neoplasm of the urinary bladder is selected from the group consisting of (i) carcinoma in situ of the urinary bladder; (ii) non-muscular invasive urothelial cancer localized at the trigone of bladder, at the dome of bladder, at the lateral wall of bladder, at the anterior wall of bladder, at the posterior wall of bladder, at the bladder neck, at the ureteric orifice and/or at the urachus; and (iii) muscular invasive urothelial cancer localized at the trigone of bladder, at the dome of bladder, at the lateral wall of bladder, at the anterior wall of bladder, at the posterior wall of bladder, at the bladder neck, at the ureteric orifice and/or at the urachus.

Thus, the antibody or antigen binding fragment thereof for use according to the invention may preferably serve for the treatment of carcinoma in situ of the urinary bladder, in particular urothelial carcinoma in situ. Further, it may preferably serve for the treatment of any malignant neoplasms of the bladder, in particular non-muscular invasive urothelial cancer localised at the trigone of bladder, the dome of bladder, the lateral wall of bladder, the anterior wall of bladder, the posterior wall of bladder, the ureteric orifice, the urachus and/or the bladder neck including the internal urethral orifice. In addition, it may serve for the treatment of any malignant neoplasms of the bladder, in particular muscular invasive urothelial cancer localised at the trigone of bladder, the dome of bladder, the lateral wall of bladder, the anterior wall of bladder, the posterior wall of bladder, the ureteric orifice, the urachus and/or the bladder neck including the internal urethral orifice.

Particularly preferred in the context of the present invention are carcinoma in situ of the urinary bladder and non-muscular invasive urothelial cancer localised at the trigone of bladder, the dome of bladder, the lateral wall of bladder, the anterior wall of bladder, the posterior wall of bladder, the ureteric orifice and the urachus or the bladder neck including the internal urethral orifice.

Preferably, the antibody, or the antigen binding fragment thereof, for use according to the present invention is a monoclonal antibody.

Herein, a monoclonal antibody (mAb or moAb) is understood as antibody made by identical immune cells that are all clones of a unique parent cell, in contrast to polyclonal antibodies which are made from several different immune cells. Generally, it is possible to produce a monoclonal antibody that specifically bind to a specific substance.

Preferably, the T cell surface antigen is selected from the group consisting of CD2, CD3, CD4, CD5, CD6, CD8, CD28, CD40L and CD44. This means that the antibody, or the antigen binding fragment thereof, for use according to the present invention comprises a paratope, which preferably recognizes (is able to bind to) an epitope of a T cell surface antigen selected from the group consisting of CD3, CD2, CD4, CD5, CD6, CD8, CD28, CD40L and/or CD44. Said specificity preferably facilitates the recruitment of T cells. Therein, CD is the abbreviation for "cluster of differentiation" (cluster of designation or classification determinant) as described above. In general, this is known as a protocol used for the identification and investigation of cell surface molecules providing targets for immunophenotyping of cells. In terms of physiology, CD molecules can act in numerous ways, often acting as receptors or ligands (the molecule that activates a receptor) important to the cell. A signal cascade is usually initiated, altering the behavior of the cell (see cell signaling. Some CD proteins do not play a role in cell signaling, but have other functions, such as cell adhesion. At present, CD for humans is numbered up to 364. The present invention refers to T-cell associated CD molecules.

More preferably, the T cell surface antigen is CD2 or CD3, most preferably the T cell surface antigen is CD3. This means that the antibody, or the antigen binding fragment thereof, for use according to the present invention comprises a paratope, which more preferably recognizes an epitope of CD2 or CD3, most preferably the antibody, or the antigen binding fragment thereof, for use according to the present invention comprises a paratope, which recognizes an epitope of CD3.

It is also preferred in the context of the present invention that the cancer- and/or tumor-associated antigen is selected from the group consisting of EpCAM, HER2/neu, CEA, MAGE, proteoglycan, VEGF, EGFR, mTOR, PIK3CA, RAS, alpha(v)beta(3)-integrin, HLA, HLA-DR, ASC, carbonic anhydrase, CD1, CD2, CD4, CD6, CD7, CD8, CD11, CD13, CD14, CD19, CD20, CD21, CD22, CD23, CD24, CD30 CD33, CD37, CD38, CD40, CD41, CD47, CD52, c-erb-2, CALLA, MHCII, CD44v3, CD44v6, p97, GM1, GM2, GM3, GD1a, GD1b, GD2, GD3, GT1b, GT3, GQ1, NY-ESO-1, NFX2, SSX2, SSX4, Trp2, gp100, tyrosinase, MUC-1, telomerase, survivin, p53, PD-L1, CA125, Wue antigen, Lewis Y antigen, HSP-27, HSP-70, HSP-72, HSP-90, Pgp, MCSP, EphA2 and cell surface targets GC182, GT468 or GT512. This means that the antibody, or the antigen binding fragment thereof, for use according to the present invention comprises a paratope, which preferably recognizes (is able to bind to) an epitope of a cancer- and/or tumor-associated antigen selected from the group consisting of EpCAM, HER2/neu, CEA, MAGE, proteoglycan, VEGF, EGFR, mTOR, PIK3CA, RAS, alpha(v)beta(3)-integrin, HLA, HLA-DR, ASC, carbonic anhydrase, CD1, CD2, CD4, CD6, CD7, CD8, CD11, CD13, CD14, CD19, CD20, CD21, CD22, CD23, CD24, CD30 CD33, CD37, CD38, CD40, CD41, CD47, CD52, c-erb-2, CALLA, MHCII, CD44v3, CD44v6, p97, GM1, GM2, GM3, GD1a, GD1b, GD2, GD3, GT1b, GT3, GQ1, NY-ESO-1, NFX2, SSX2, SSX4, Trp2, gp100, tyrosinase, MUC-1, telomerase, survivin, p53, PD-L1, CA125, Wue antigen, Lewis Y antigen, HSP-27, HSP-70, HSP-72, HSP-90, Pgp, MCSP, EphA2 and cell surface targets GC182, GT468 or GT512.

Preferably, in the context of the present invention the cancer- and/or tumor-associated antigen is selected from the group consisting of EpCAM, HER2/neu, CEA, MAGE, proteoglycan, VEGF, EGFR, mTOR, PIK3CA, RAS, alpha(v)beta(3)-integrin, HLA, HLA-DR, ASC, carbonic anhydrase, CD1, CD2, CD4, CD6, CD7, CD8, CD11, CD13, CD14, CD19, CD20, CD21, CD22, CD23, CD24, CD30 CD33, CD37, CD38, CD40, CD41, CD47, CD52, CD133, c-erb-2, CALLA, MHCII, CD44v3, CD44v6, p97, GM1, GM2, GM3, GD1a, GD1b, GD2, GD3, GT1b, GT3, GQ1, NY-ESO-1, NFX2, SSX2, SSX4, Trp2, gp100, tyrosinase, MUC-1, telomerase, survivin, p53, PD-L1, CA125, Wue antigen, Lewis Y antigen, HSP-27, HSP-70, HSP-72, HSP-90, Pgp, MCSP, EphA2 and cell surface targets GC182, GT468 or GT512. This means that the antibody, or the antigen binding fragment thereof, for use according to the present invention comprises a paratope, which preferably recognizes (is able to bind to) an epitope of a cancer- and/or tumor-associated antigen selected from the group consisting of EpCAM, HER2/neu, CEA, MAGE, proteoglycan, VEGF, EGFR, mTOR, PIK3CA, RAS, alpha(v)beta(3)-integrin, HLA, HLA-DR, ASC, carbonic anhydrase, CD1, CD2, CD4, CD6, CD7, CD8, CD11, CD13, CD14, CD19, CD20, CD21, CD22, CD23, CD24, CD30 CD33, CD37, CD38, CD40, CD41, CD47, CD52, CD133, c-erb-2, CALLA, MHCII, CD44v3, CD44v6, p97, GM1, GM2, GM3, GD1a, GD1b, GD2, GD3, GT1b, GT3, GQ1, NY-ESO-1, NFX2, SSX2, SSX4, Trp2, gp100, tyrosinase, MUC-1, telomerase, survivin, p53, PD-L1, CA125, Wue antigen, Lewis Y antigen, HSP-27, HSP-70, HSP-72, HSP-90, Pgp, MCSP, EphA2 and cell surface targets GC182, GT468 or GT512.

Particularly preferably, the cancer- and/or tumor-associated antigen is selected from the group consisting of EpCAM, HER2/neu, CEA, MAGE, VEGF, EGFR, mTOR, PD-L1, PIK3CA, RAS, GD2, CD19, CD20 and CD33, more preferably the cancer- and/or tumor-associated antigen is selected from the group consisting of EpCAM, HER2/neu, CEA, GD2, CD19, CD20 and CD33, even more preferably the cancer- and/or tumor-associated antigen is selected from the group consisting of EpCAM, HER2/neu, GD2 and CD20 and most preferably the cancer-and/or tumor-associated antigen is EpCAM. This means that the antibody, or the antigen binding fragment thereof, for use according to the present invention comprises a paratope, which preferably recognizes an epitope of EpCAM, HER2/neu, CEA, MAGE, VEGF, EGFR, mTOR, PD-L1, PIK3CA, RAS, GD2, CD19, CD20 or CD33; more preferably the antibody, or the antigen binding fragment thereof, for use according to the present invention comprises a paratope, which recognizes an epitope of EpCAM, HER2/neu, CEA, GD2, CD19, CD20 or CD33; even more preferably the antibody, or the antigen binding fragment thereof, for use according to the present invention comprises a paratope, which recognizes an epitope of EpCAM, HER2/neu, GD2 or CD20; and most preferably the antibody, or the antigen binding fragment thereof, for use according to the present invention comprises a paratope, which recognizes an epitope of EpCAM. EpCAM is predominantly expressed in high grade and advanced stage urothelial carcinoma of the bladder (Brunner et al. EpCAM is predominantly expressed in high grade and advanced stage urothelial carcinoma of the bladder. J Clin Pathol 61(3):307 (2008)). It is thus preferred that the cancer and/or tumor-associated antigen (or an epitope thereon, respectively) to be recognized by the antibody, or the antigen binding fragment thereof, for use according to the present invention is EpCAM.

Preferably, the cancer and/or tumor-associated antigen (or an epitope thereon, respectively) to be recognized by the antibody, or the antigen binding fragment thereof, for use according to the present invention is Her2/neu. Preferably, the cancer and/or tumor-associated antigen (or an epitope thereon, respectively) to be recognized by the antibody, or the antigen binding fragment thereof, for use according to the present invention is GD2. Preferably, the cancer and/or tumor-associated antigen (or an epitope thereon, respectively) to be recognized by the antibody, or the antigen binding fragment thereof, for use according to the present invention is GD3. Preferably, the cancer and/or tumor-associated antigen (or an epitope thereon, respectively) to be recognized by the antibody, or the antigen binding fragment thereof, for use according to the present invention is CD20. Preferably, the cancer and/or tumor-associated antigen (or an epitope thereon, respectively) to be recognized by the antibody, or the antigen binding fragment thereof, for use according to the present invention is CD19. Preferably, the cancer and/or tumor-associated antigen (or an epitope thereon, respectively) to be recognized by the antibody, or the antigen binding fragment thereof, for use according to the present invention is CD30. Alternatively, the cancer and/or tumor-associated antigen (or an epitope thereon, respectively) to be recognized by the antibody, or the antigen binding fragment thereof, for use according to the present invention is CEA, MAGE, VEGF, EGFR, mTOR, PD-L1, PIK3CA or RAS.

Preferably, the antibody, or the antigen binding fragment thereof, for use according to the present invention binds (i) by its first specificity, e.g. by its first paratope, to an epitope of the T-cell surface antigen selected from the group consisting of CD2, CD3, CD4, CD5, CD6, CD8, CD28, CD40L and CD44, preferably CD2 or CD3, more preferably CD3; and, (ii) by its second specificity, e.g. by its second paratope, to a cancer and/or tumor-associated antigen preferably selected from the group consisting of the tumor antigens EpCAM, HER2/neu, CEA, MAGE, VEGF, EGFR, mTOR, PD-L1, PIK3CA, RAS, GD2, CD19, CD20 and CD33.

More preferably, the antibody, or the antigen binding fragment thereof, for use according to the present invention binds (i) by its first specificity, e.g. by its first paratope, to an epitope of the T-cell surface antigen selected from the group consisting of CD2, CD3, CD4, CD5, CD6, CD8, CD28, CD40L and CD44, preferably CD2 or CD3, more preferably CD3; and, (ii) by its second specificity, e.g. by its second paratope, to a cancer and/or tumor-associated antigen preferably selected from the group consisting of the tumor antigens EpCAM, HER2/neu, CEA, MAGE, VEGF, EGFR, mTOR, PD-L1, PIK3CA, RAS, GD2, CD19 and CD20.

The antibody, or the antigen binding fragment thereof, for use according to the present invention preferably binds by its first specificity, e.g. by its first paratope, to an epitope of the T-cell surface antigen, preferably CD3, and, by its second specificity, e.g. by its second paratope, to a cancer and/or tumor-associated antigen preferably selected from the group consisting of the tumor antigens EpCAM, HER2/neu, CEA, MAGE, VEGF, EGFR, mTOR, PD-L1, PIK3CA, RAS, GD2, CD19 and CD20 or to the gangliosides GM1, GM2, GM3, GD1a, GD1b, GD3, GT1b, GT3 or GQ1.

Preferably, the antibody, or the antigen binding fragment thereof, for use according to the present invention comprises a first specificity against CD3 and a second specificity against a cancer- and/or tumor-associated antigen selected from the group consisting of EpCAM, HER2/neu, CEA, GD2, CD19, CD20 and CD33.

Accordingly, the antibody, or the antigen binding fragment thereof, for use according to the present invention may comprise one specificity, preferably one paratope, against CD3 and one specificity, preferably one paratope, against EpCAM (anti-CD3 x anti-EpCAM). Preferably, the antibody, or the antigen binding fragment thereof, for use according to the present invention may comprise one specificity, preferably one paratope, against CD3 and one specificity, preferably one paratope, against Her2/neu (anti-CD3 x anti-Her2/neu). Preferably, the antibody, or the antigen binding fragment thereof, for use according to the present invention may comprise one specificity, preferably one paratope, against CD3 and one specificity, preferably one paratope, against GD2 (anti-CD3 x anti-GD2). Preferably, the antibody, or the antigen binding fragment thereof, for use according to the present invention may comprise one specificity, preferably one paratope, against CD3 and one specificity, preferably one paratope, against GD3 (anti-CD3 x anti-GD3). Preferably, the antibody, or the antigen binding fragment thereof, for use according to the present invention may comprise one specificity, preferably one paratope, against CD3 and one specificity, preferably one paratope, against CD20 (anti-CD3 x anti-CD20). Preferably, the antibody, or the antigen binding fragment thereof, for use according to the present invention may comprise one specificity, preferably one paratope, against CD3 and one specificity, preferably one paratope, against CD19 (anti-CD3 x anti-CD19). Preferably, the antibody, or the antigen binding fragment thereof, for use according to the present invention may comprise one specificity, preferably one paratope, against CD3 and one specificity, preferably one paratope, against CEA (anti-CD3 x anti- CEA). Preferably, the antibody, or the antigen binding fragment thereof, for use according to the present invention may comprise one specificity, preferably one paratope, against CD3 and one specificity, preferably one paratope, against MAGE (anti-CD3 x anti- MAGE). Preferably, the antibody, or the antigen binding fragment thereof, for use according to the present invention may comprise one specificity, preferably one paratope, against CD3 and one specificity, preferably one paratope, against VEGF (anti-CD3 x anti- VEGF). Preferably, the antibody, or the antigen binding fragment thereof, for use according to the present invention may comprise one specificity, preferably one paratope, against CD3 and one specificity, preferably one paratope, against EGFR (anti-CD3 x anti-EGFR). Preferably, the antibody, or the antigen binding fragment thereof, for use according to the present invention may comprise one specificity, preferably one paratope, against CD3 and one specificity, preferably one paratope, against mTOR (anti-CD3 x anti-mTOR). Preferably, the antibody, or the antigen binding fragment thereof, for use according to the present invention may comprise one specificity, preferably one paratope, against CD3 and one specificity, preferably one paratope, against PD-L1 (anti-CD3 x anti-PD-L1). Preferably, the antibody, or the antigen binding fragment thereof, for use according to the present invention may comprise one specificity, preferably one paratope, against CD3 and one specificity, preferably one paratope, against PIK3CA (anti-CD3 x anti-PIK3CA). Preferably, the antibody, or the antigen binding fragment thereof, for use according to the present invention may comprise one specificity, preferably one paratope, against CD3 and one specificity, preferably one paratope, against RAS (anti-CD3 x anti-RAS).

Alternatively, the antibody, or the antigen binding fragment thereof, for use according to the present invention may comprise one specificity, preferably one paratope, against CD3 and one specificity, preferably one paratope, against CD30 (anti-CD3 x anti-CD30). Preferably, the antibody, or the antigen binding fragment thereof, for use according to the present invention may comprise one specificity, preferably one paratope, against CD3 and one specificity, preferably one paratope, against CD33 (anti-CD3 x anti-CD33). Preferably, the antibody, or the antigen binding fragment thereof, for use according to the present invention may comprise one specificity, preferably one paratope, against CD3 and one specificity, preferably one paratope, against an arboviral E protein epitope (anti-CD3 x anti- arboviral E protein).

Preferably, the antibody, or the antigen binding fragment thereof, for use according to the present invention comprises two specificities selected from anti-EpCAM x anti-CD3, anti-CD20 x anti-CD3, anti-HER2/neu x anti-CD3, anti-GD2 x anti-CD3 and anti -CD19 x anti-CD3.

Preferably, the antibody, or the antigen binding fragment thereof, for use according to the present invention is a bispecific antibody or a bispecific antigen binding fragment thereof.

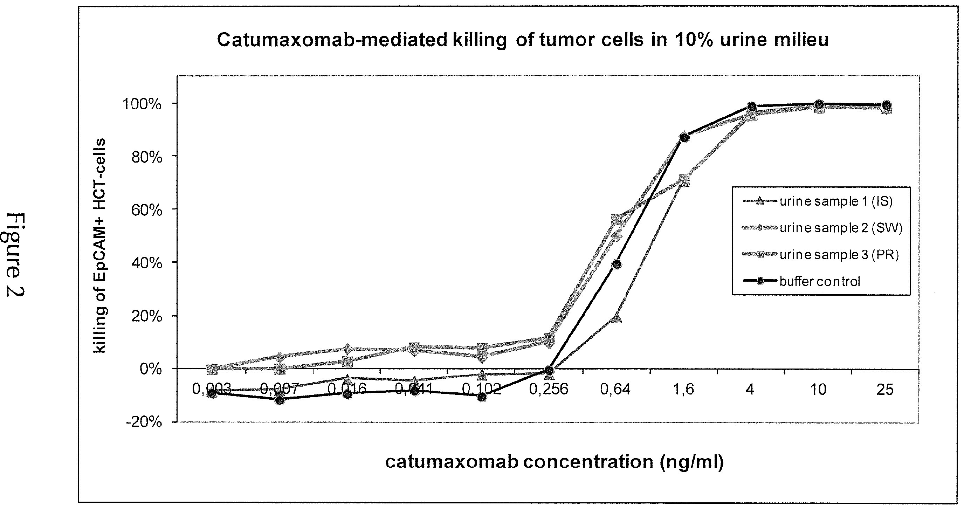

In the context of the present invention, bispecific antibodies (BiAbs) comprise (exactly) two specificities. They are the most preferred type of multispecific antibodies and antigen binding fragments thereof. A bispecific antibody in the context of the present invention may be of any bispecifc antibody format, e.g., as described in Spiess C., Zhai Q. and Carter P.J. (2015) Molecular Immunology 67: 95-106. For example, BiAbs may be whole antibodies, such as whole IgG-like molecules, or fragments thereof which are not whole antibodies but retain antibody properties. These may be small recombinant formats, e.g. as tandem single chain variable fragment molecules (taFvs), diabodies (Dbs), single chain diabodies (scDbs), bispecific T-cell engagers (BiTes) and various other derivatives of these (cf. e.g. Byrne H. et al. (2013) Trends Biotech, 31 (11): 621-632 with Figure 2 showing various bispecific antibody formats). Several BiAb formats can redirect effector cells against target cells that play key roles in disease processes. For example, several BiAb formats can retarget effector cells towards tumor cells and a variety of BiAb constructs were designed to retarget cells of the immune system, for example by binding to and triggering Fc receptors on the surface of effector cells or by binding to T cell receptor (TCR) complexes.

Preferably, the multispecific, in particular bispecific, antibody, or the antigen binding fragment thereof is at least bivalent, i.e. it has at least two paratopes. More preferably, the multispecific, in particular bispecific, antibody, or the antigen binding fragment thereof is bivalent, trivalent, tetravalent, or hexavalent. Even more preferably, the multispecific, in particular bispecific, antibody, or the antigen binding fragment thereof is bivalent or tetravalent. Most preferably, the antibody, or the antigen binding fragment thereof, for use according to the present invention is a bispecific, bivalent antibody, i.e. an antibody having two paratopes: one recognizing a T cell surface antigen and the other recognizing a cancer- and/or tumor-associated antigen.

It is also preferred that the antibody, or the antigen binding fragment thereof, for use according to the present invention is a bifunctional or trifunctional antibody or antigen binding fragment thereof, i.e. being capable of interacting with two or three, preferably different, binding sites simultaneously. More preferably, the antibody, or the antigen binding fragment thereof, for use according to the present invention is a trifunctional antibody or a trifunctional antigen binding fragment thereof, in particular a bispecific trifunctional antibody or a bispecific, trifunctional antigen binding fragment thereof.

In the context of the present invention, a "bifunctional" antibody or antigen binding fragment thereof refers to such a compound which typically also is bispecific. Normally such a compound has no further binding sites in addition to the two "specificities" (paratopes), even no unspecific binding sites. In contrast, "trifunctional" antibodies (trAb) are understood in the context of the present invention as a specific class of bispecific antibodies recruiting and activating T cells and, in particular, accessory immune cells, such as macrophages, dendritic cells, natural killer (NK) cells, and other Fc-receptor-expressing cells, simultaneously at the targeted cancer/tumor by, e.g. their Fc-receptor binding site. Thus, trifunctional bispecific antibodies have two antigen-binding sites (i.e. two paratopes). Typically, these two antigen-binding sites (paratopes) allow the antibodies to bind to cancer/tumor cells (cancer/tumor cell surface antigens) and to T cells (T cell surface antigens). Simultaneously, e.g. via their Fc moiety, in particular their Fcγ receptor binding site, positive accessory cells are recruited, for example monocytes/macrophages, natural killer cells, dendritic cells or other Fcγ receptor expressing cells. The simultaneous activation of these different classes of effector cells results in efficient killing of the tumor cells by various mechanisms such as phagocytosis and perforin-mediated cytotoxicity. Typically, the net effect of a trifunctional antibody is linking T cells and, in particular, Fcγ receptor positive accessory cells to tumor cells, leading to the destruction of the tumor cells.

Trifunctional antibodies evoke the removal of tumor cells in particular by means of (i) antibody-dependent cell-mediated cytotoxicity, (ii) T-cell mediated cell killing, and (iii) induction of anti-tumor immunity. In contrast, only the first mode of action is actually executed by conventional (monoclonal and monospecific) antibodies. Moreover, in contrast to conventional antibodies, bispecific, and in particular trifunctional, antibodies have a higher cytotoxic potential and they even bind to antigens, which are expressed relatively weakly. Thus, bispecific, and in particular trifunctional, antibodies are at an equivalent dose more potent (more than 1000-fold) in eliminating tumor cells compared to conventional antibodies.

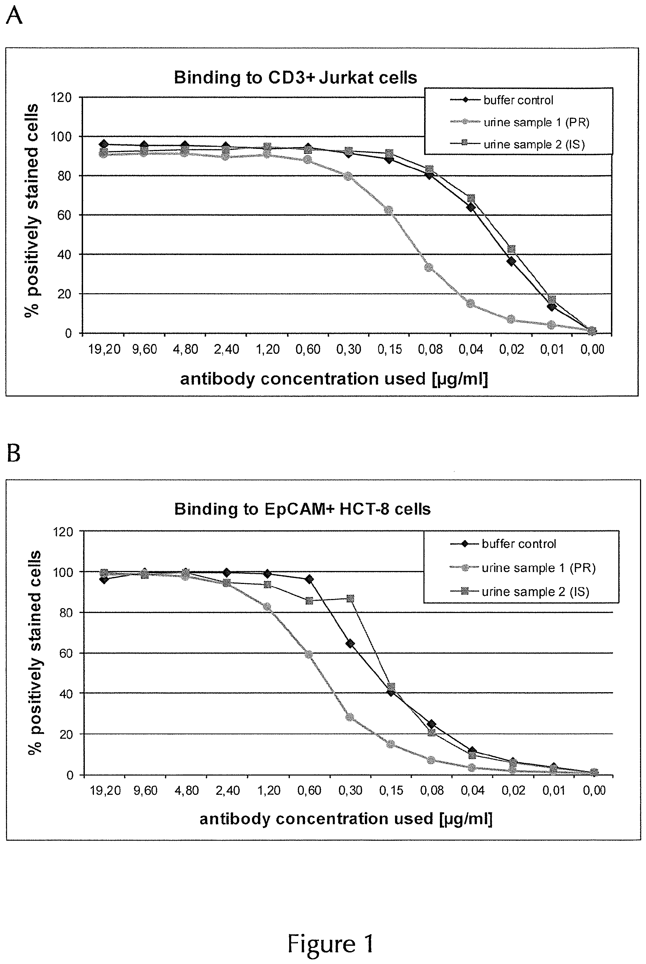

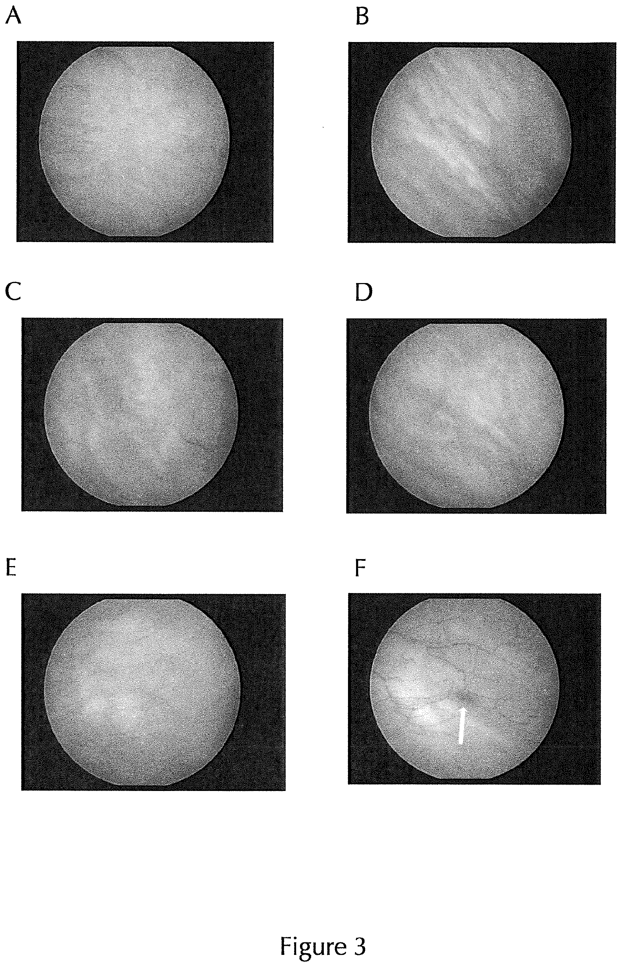

The antibody, or the antigen binding fragment thereof, for use according to the present invention may be of any antibody format. In particular, multispecific antibodies preferably encompass "whole" antibodies, such as whole IgG-or IgG-like molecules, while antigen binding fragments in the context of the present invention preferably refer to small recombinant formats, such as bispecific T-cell engagers (BiTes), tandem single chain variable fragment molecules (taFvs), diabodies (Dbs), single chain diabodies (scDbs) and various other derivatives of these (cf. bispecific antibody formats as described by Byrne H. et al. (2013) Trends Biotech, 31 (11): 621-632 with Figure 2 showing various bispecific antibody formats; Weidle U.H. et al. (2013) Cancer Genomics and Proteomics 10: 1-18, in particular Fig. 1 showing various bispecific antibody formats; and Chan, A.C. and Carter, P.J. (2010) Nat Rev Immu 10: 301-316 with Fig. 3 showing various bispecific antibody formats). Examples of bispecific antibody formats include, but are not limited to, quadroma, chemically coupled Fab (fragment antigen binding), and BiTE® (bispecific T cell engager). In one embodiment of the present invention the antibody used is preferably a BiTE® (bispecific T cell engager).

Thus, the antibody, or the antigen binding fragment thereof, for use according to the present invention may be selected from the group comprising Triomabs; hybrid hybridoma (quadroma); Multispecific anticalin platform (Pieris); Diabodies; Single chain diabodies; Tandem single chain Fv fragments; TandAbs, Trispecific Abs (Affimed) (105-110 kDa); Darts (dual affinity retargeting; Macrogenics); Bispecific Xmabs (Xencor); Bispecific T cell engagers (Bites; Amgen; 55kDa); Triplebodies; Tribody = Fab-scFv Fusion Protein (CreativeBiolabs) multifunctional recombinant antibody derivates (110 kDa); Duobody platform (Genmab); Dock and lock platform; Knob into hole (KIH) platform; Humanized bispecific IgG antibody (REGN1979) (Regeneron); Mab$^2$ bispecific antibodies (F-Star); DVD-Ig = dual variable domain immunoglobulin (Abbvie); kappa-lambda bodies; TBTI = tetravalent bispecific tandem Ig; and CrossMab.

The antibody, or the antigen binding fragment thereof, for use according to the present invention may be selected from bispecific IgG-like antibodies (BsIgG) comprising CrossMab; DAF (two-in-one); DAF (four-in-one); DutaMab; DT-IgG; Knobs-in-holes common LC; Knobs-in-holes assembly; Charge pair; Fab-arm exchange; SEEDbody; Triomab; LUZ-Y; Fcab; κλ-body; and Orthogonal Fab. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

Preferably, the antibody, or the antigen binding fragment thereof, for use according to the present invention may be selected from IgG-appended antibodies with an additional antigen-binding moiety comprising DVD-IgG; IgG(H)-scFv; scFv-(H)IgG; IgG(L)-scFv; scFV-(L)IgG; IgG(L,H)-Fv; IgG(H)-V; V(H)-IgG; IgG(L)-V; V(L)-IgG; KIH IgG-scFab; 2scFv-IgG; IgG-2scFv; scFv4-Ig; scFv4-Ig; Zybody; and DVI-IgG (four-in-one). These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

Preferably, the antibody, or the antigen binding fragment thereof, for use according to the present invention may be selected from bispecific antibody fragments comprising Nanobody; Nanobody-HAS; BiTE; Diabody; DART; TandAb; scDiabody; sc-Diabody-CH3; Diabody-CH3; Triple Body; Miniantibody; Minibody; TriBi minibody; scFv-CH3 KIH; Fab-scFv; scFv-CH-CL-scFv; F(ab')2; F(ab')2-scFv2; scFv-KIH; Fab-scFv-Fc; Tetravalent HCAb; scDiabody-Fc; Diabody-Fc; Tandem scFv-Fc; and Intrabody. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

Preferably, the antibody, or the antigen binding fragment thereof, for use according to the present invention does not comprise a binding site for an Fc receptor, in particular the antibody, or the antigen binding fragment thereof, does not comprise an Fc moiety such as an Fc region.

Preferably, the antibody, or the antigen binding fragment thereof, for use according to the present invention may be selected from bispecific fusion proteins comprising Dock and Lock; ImmTAC; HSAbody; scDiabody-HAS; and Tandem scFv-Toxin. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

In particular, the antibody, or the antigen binding fragment thereof, for use according to the present invention may be selected from bispecific antibody conjugates comprising IgG-IgG; Cov-X-Body; and scFv1-PEG-scFv2. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

It is also preferred, that the antibody, or the antigen binding fragment thereof, for use according to the present invention is selected from the group consisting of a bispecific T-cell engager (BiTE™) and a bispecific trifunctional antibody.

Preferably, the antibody, or the antigen binding fragment thereof, for use according to the present invention comprises a binding site for an Fc receptor. More preferably, the antibody, or the antigen binding fragment thereof, for use according to the present invention comprises an Fc moiety, in particular an Fc region.

As used herein, the term "Fc moiety" refers to a sequence derived from the portion of an immunoglobulin heavy chain beginning in the hinge region just upstream of the papain cleavage site and ending at the C-terminus of the immunoglobulin heavy chain. Preferably, the "Fc moiety" comprises a binding site for an Fc receptor. However, it is also preferred that an Fc moiety may mediate a functionality different from binding to an Fc receptor, for example binding to a protein of the complement system. Accordingly, an "Fc moiety" may be a complete Fc region or a part (e.g., a domain) thereof. Preferably, the "Fc moiety" mediates the full functionality of a complete Fc region, e.g. including Fc receptor binding and, optionally, binding to a protein from the complement system. Thus, the antibody as used according to the present invention preferably comprises a complete Fc region, whereby a complete Fc region comprises at least a hinge domain, a CH2 domain, and a CH3 domain. The Fc moiety may also comprise one or more amino acid insertions, deletions, or substitutions relative to a naturally-occurring Fc region. For example, at least one of a hinge domain, CH2 domain or CH3 domain (or portion thereof) may be deleted. For example, an Fc moiety may comprise or consist of: (i) hinge domain (or portion thereof) fused to a CH2 domain (or portion thereof), (ii) a hinge domain (or portion thereof) fused to a CH3 domain (or portion thereof), (iii) a CH2 domain (or portion thereof) fused to a CH3 domain (or portion thereof), (iv) a hinge domain (or portion thereof), (v) a CH2 domain (or portion thereof), or (vi) a CH3 domain or portion thereof.

As multispecific antibodies or antigen binding fragments thereof, trifunctional bispecific antibodies are especially preferred as they may be employed according to the present invention and exhibit three functions, in particular including their ability to bind (redirect) T-cells and to bind (target) tumor cells by the two specificities as described above, and - as a third function - trifunctional antibodies are in particular able to bind e.g. to monocytes, macrophages, dendritic cells, "natural killer" cells (NK cells) and/or activated neutrophils, in particular by their Fc receptor. Preferably, their Fc portion binds to Fc receptor-positive cells, which preferably at least express one type of an Fcγ receptor, preferably an Fcγ receptor type I, IIa and/or III. Nevertheless, bifunctional and bispecific antigen binding agents lacking said third function such as bispecific T-cell engager are also preferred due to their ability to redirect T cells, also in the bladder environment.

For example, the "third functionality" in the bispecific trifunctional antibody for use according to the present invention is a binding site for an Fc receptor and, thus, in particular the bispecific trifunctional antibody as used according to the present invention preferably comprises a binding site for an Fc receptor. More preferably the bispecific trifunctional antibody as used according to the present invention comprises an Fc moiety, such as an Fc region (fragment crystallizable region, also referred to as "Fc portion"). Such preferred trifunctional bispecific antibody for use according to the invention recruit, in particular by its binding site for an Fc receptor, immune cells expressing on its cell surface Fc receptors, in particular Fcγ receptor type I, IIa and/or III. Thus, the trifunctional bispecific antibodies in particular aggregate three distinct cell types, i.e. two types of immune cells (T cells and Fc receptor expressing immune cells) and the target cancer/tumor cells. Various modes of action of the immune system are thereby mobilized to attack the recruited target cancer/tumor cell.

The binding site for an Fc receptor, for example the Fc region, enables the (trifunctional) antibody to additionally recruit cells expressing an Fc receptor, such as Fcγ receptor positive accessory cells, for example macrophages, dendritic cells, natural killer (NK) cells, and other Fc-receptor-expressing cells. Since trifunctional antibodies are bispecific (or multispecific) antibodies, they are preferably able to recruit and activate (i) T cells and (ii) Fc-receptor expressing cells, such as accessory immune cells, for example monocytes/macrophages, natural killer cells, dendritic cells or other Fc receptor expressing cells, simultaneously at the (iii) targeted cancer/tumor cells. The simultaneous activation of these different classes of effector cells results in efficient killing of the tumor cells by various mechanisms such as, for example, phagocytosis and perforin-mediated cytotoxicity. Typically, the net effect of a preferred trifunctional antibody, which comprises an Fc receptor, is linking T cells and Fc receptor positive cells to target cells, e.g. tumor cells, leading to the destruction of the tumor cells. Trifunctional antibodies evoke the removal of tumor cells by means of (i) antibody-dependent cell-mediated cytotoxicity, (ii) T-cell mediated cell killing, and (iii) induction of anti-tumor immunity.

Preferably, the antibody, or the antigen binding fragment thereof, for use according to the present invention has an IgG-like format (based on IgG, also referred to as "IgG type"), whereby an antibody having an IgG-like format usually comprises two heavy chains and two light chains. In general, Immunoglobulin G (IgG) is known as a type of antibody. It is understood herein as a protein complex composed of four peptide chains-two identical heavy chains and two identical light chains arranged in a Y-shape typical of antibody monomers. Each IgG has typically two antigen binding sites, which may be different or identical. Representing about 75% of serum antibodies in humans, IgG is the most common type of antibody found in the circulation. Physiologically, IgG molecules are created and released by plasma B cells.

Examples of an antibody having an IgG-like format include a quadroma and various IgG-scFv formats (cf: Byrne H. et al. (2013) Trends Biotech, 31 (11): 621-632; Figure 2A-E), whereby a quadroma is preferred, which is preferably generated by fusion of two different hybridomas. Within the IgG class, antibodies may preferably be based on the IgG1, IgG2, IgG3 or IgG4 subclass, whereby an antibody based on IgG1 (also referred to as "IgG1 type") is preferred. The multispecific antibodies or antigen binding fragments, such as bispecific antibodies, for use according to the present invention may alternatively be based on any immunoglobulin class (e.g., IgA, IgG, IgM etc.) and subclass (e.g. IgA1, IgA2, IgG1, IgG2, IgG3, IgG4 etc.)

In general, the multispecific antibodies, such as bispecific antibodies, or the antigen binding fragments thereof, for use according to the present invention are produced by three main methods: (i) chemical conjugation, which involves chemical cross-linking; (ii) fusion of two different hybridoma cell lines; or (iii) genetic approaches involving recombinant DNA technology. The fusion of two different hybridomas produces a hybrid-hybridoma (or "quadroma") secreting a heterogeneous antibody population including bispecific molecules.

Alternative approaches included chemical conjugation of two different mAbs and/or smaller antibody fragments. Oxidative reassociation strategies to link two different antibodies or antibody fragments were found to be inefficient due to the presence of side reactions during reoxidation of the multiple native disulfide bonds. Current methods for chemical conjugation focus on the use of homo- or hetero-bifunctional crosslinking reagents. Recombinant DNA technology has yielded the greatest range of bsAbs, through artificial manipulation of genes and represents the most diverse approach for bsAb generation (45 formats in the past two decades; cf. Byrne H. et al. (2013) Trends Biotech, 31 (11): 621-632).

In particular by use of such recombinant DNA technology, also a variety of further multispecific antibodies have emerged recently. Multispecific antibodies, in particular with three or more paratopes, are in particular achieved by recombinant DNA techniques. In the context of the present invention, the antibody may in particular also have more than two specificities, and, thus, more than two paratopes, as at least two paratopes are required according to the present invention, for example one for the target cell and the other for a T cell. Accordingly, the antibody for use according to the invention may have further paratopes, in particular relating to further specificities, in addition to the two paratopes.

Preferably, the antibody, or the antigen binding fragment thereof, for use according to the invention is an IgG type antibody comprising a binding site for an Fc receptor, in particular an Fc region. More preferably, the antibody, or the antigen binding fragment thereof, for use according to the invention is a trifunctional bispecific antibody, which is a heterologous intact rat/mouse antibody comprising a binding site for an Fc receptor, in particular an Fc region. Thereby, an antibody with a subclass combination of mouse IgG2a and rat IgG2b is preferred. A heterologous intact rat/mouse antibody comprising a binding site for an Fc receptor, in particular an Fc region, with a heavy chain composed of murine IgG2a and rat IgG2b subclasses, each with their respective light chains, is particularly preferred.

In general, a trifunctional bispecific antibody for use according to the invention exhibits preferably one of the following isotype combinations in its Fc-region: rat-IgG2b/mouse-IgG2a, rat-IgG2b/mouse-IgG2b, rat-IgG2b/human-IgG1, or mouse-[VH-CH1,VL-CL]-human-IgG1/rat-[VH-CH1, VL-CL]-human-IgG1-[hinge]-human IgG3*-[CH2-CH3], wherein * = caucasian allotypes G3m(b+g) = no binding to protein A.

It is also preferred that the antibody, or the antigen binding fragment thereof, for use according to the invention comprises at least two different single-chain variable fragment (scFvs). A single-chain variable fragment (scFv) is herein understood as a fusion protein of the variable regions of the heavy (VH) and light chains (VL) of immunoglobulins, connected with a short linker peptide. Said peptide typically comprises at least 5, preferably at least 10, more preferred about 25 amino acids. The linker is usually rich in glycine for flexibility, as well as serine or threonine for solubility, and can either connect the N-terminus of the VH with the C-terminus of the VL, or vice versa. A scFv may retain the specificity of the original immunoglobulin, despite removal of the constant regions and the introduction of the linker. Typically, a scFv can be created directly from subcloned heavy and light chains derived from a hybridoma. ScFvs are generally used, e.g., in flow cytometry, immunohistochemistry, and as antigen-binding domains of artificial T cell receptors. In the context of the present invention, they are preferably used as antigen-binding domains of artificial T cell receptors.

Preferred bispecific IgG-like antibody formats comprise for example hybrid hybridoma (quadroma), knobs-into-holes with common light chain, various IgG-scFv formats, various scFv-IgG formats, two-in-one IgG, dual V domain IgG, IgG-V, and V-IgG, which are shown for example in Figure 3c of Chan, A.C. and Carter, P.J. (2010) Nat Rev Immu 10: 301-316 and described in said article. Further preferred bispecific IgG-like antibody formats include for example DAF, CrossMab, IgG-dsscFv, DVD, IgG-dsFV, IgG-scFab, scFab-dsscFv and Fv2-Fc, which are shown in Fig. 1A of Weidle U.H. et al. (2013) Cancer Genomics and Proteomics 10: 1 - 18 and described in said article. Further preferred bispecific IgG-like antibody formats include DAF (two-in-one); DAF (four-in-one); DutaMab; DT-IgG; Knobs-in-holes assembly; Charge pair; Fab-arm exchange; SEEDbody; Triomab; LUZ-Y; Fcab; κλ-body; Orthogonal Fab; DVD-IgG; IgG(H)-scFv; scFv-(H)IgG; IgG(L)-scFv; scFV-(L)IgG; IgG(L,H)-Fv; IgG(H)-V; V(H)-IgG; IgG(L)-V; V(L)-IgG; KIH IgG-scFab; 2scFv-IgG; IgG-2scFv; scFv4-Ig; scFv4-Ig; Zybody; and DVI-IgG (four-in-one) as shown and described for example in Spiess C., Zhai Q. and Carter P.J. (2015) Molecular Immunology 67: 95-106, in particular Fig. 1 and corresponding description, e.g. p. 95 - 101.

Preferably, the antibody, or the antigen binding fragment thereof, for use according to the invention is selected from the group consisting of Catumaxomab (anti-CD3 x anti-EpCAM), FBTA05/Lymphomun (anti-CD3 X anti-CD20), Ertumaxomab (anti-CD3 x anti-HER2/neu), Ektomun (anti-CD3 X anti-GD2), blinatumomab and solitomab, preferably the antibody is catumaxomab.

The most preferred example of trifunctional bispecific antibodies is catumaxomab (Removab®) (anti-EpCAM x anti-CD3). Further preferred examples of trifunctional bispecific antibodies include (i) FBTA05 (also called "Lymphomun"), a trifunctional anti-CD3 x anti-CD20 antibody, (ii) Ertumaxomab, a trifunctional anti-CD3 x anti-HER2 antibody, and (iii) TRBs02/TRBs07, two trifunctional antibodies specific for human melanoma (Ruf et al. (2004) Int J Cancer, 108: 725-732).

Alternatively, the antibody, or the antigen binding fragment thereof, for use according to the invention is selected from the group consisting of an anti-CD19 x anti-CD3 bispecific T-cell engager and an anti-EpCAM x anti-CD3 bispecific T-cell engager.

*Treatment protocols and dosage*

According to the differences in the modes of action, the established treatment protocols for multispecific, in particular bispecific, such as bispecific trifunctional, antibodies usually differ considerably from that for conventional (monoclonal and monospecific) antibodies. For example, catumaxomab was administered according to a regimen of four intraperitoneal infusions on days 0, 3, 7, and 10 at doses of 10, 20, 50, and 150 $\mu$g, respectively (cf. Heiss et al. (2010) Int J Cancer, 127: 2209-2221 and "Assessment Report for Removab", European Medicines Agency, Doc.Ref.: EMEA/CHMP/100434/2009). FBTA05/Lymphomun was administered as e.g. an i.v. infusion with escalating doses, starting with 10 $\mu$g on day 1, increasing, e.g. doubling, the doses on the following days, with final doses of up to 2000 $\mu$g (Buhmann et al. (2009) Bone Marrow Transplant, 43: 383-397: Table 2). Ertumaxomab was also administered as an i.v. infusion with escalating doses, starting with 10 $\mu$g on day 1, followed by 20, 50, 100, 150 or 200 $\mu$g, respectively, on each, day 7$\pm$1 and day 13$\pm$1 (Kiewe et al. (2006) Clin Cancer Res, 12: 3085-3091). Thus, for multispecific, in particular bispecific, such as bispecific trifunctional, antibodies in clinical trials, the treatment protocol typically followed an escalating dosing scheme. It is to be noted that the initial dose of multispecific, in particular bispecific, such as bispecific trifunctional, antibodies is a critical parameter, as those antibodies activate T cells, trigger the release of proinflammatory cytokines and up-regulate co-stimulatory factors, e.g. CD28.

Preferably, the antibody, or the antigen binding fragment thereof, for use according to the present invention is administered in one or more treatment cycles. In the context of the present invention, a treatment cycle is a course of one or more treatment(s) that may be repeated on a regular schedule with periods of rest in between. For example, the antibody, or the antigen binding fragment thereof, for use according to the present invention may be administered in one treatment cycle (e.g., one single dose or repeated doses) and, thereafter, it may be observed whether the cancer or tumor recurs. In particular when the cancer/tumor recurs, a further treatment cycle may be performed. However, a further treatment cycle may also be performed as a prophylactic measure. In particular, the interval between two treatments (e.g., two doses) within one treatment cycle does preferably not exceed one month (31 days), more preferably it does not exceed 3 weeks, whereas the interval between the end of one treatment cycle and the beginning of the next treatment cycle is preferably at least one month, preferably at least two months, more preferably at least 3 months even more preferably at least 4 months and most preferably at least 6 months.

Preferably, one treatment cycle comprises (i) one single dose or (ii) one initial dose and one or more subsequent doses. The treatment protocol according to the present invention is typically composed of a number of single administrations which form a treatment cycle. The patient may be subjected to one single or various treatment cycles. Each treatment cycle is typically composed of from 2 to 28, preferably from 2 to 20, more preferably from 3 to 10, and even more preferably from 5 to 8, e.g. 6 or 7, individual doses.

Preferably, one treatment cycle comprises one initial dose and one or more subsequent doses and (i) the one initial dose and the one or more subsequent doses are the same, or (ii) the one or more subsequent dose(s) is/are higher than the initial dose. In other words, it is preferred that within a treatment cycle (starting with an initial dose and ending with a final dose), the dose of each single administration (except the initial dose) is not lower than the previous dose administered, i.e. each subsequent dose is equal to or higher than the previous one. Such increased dosing, which is understood herein as escalating dosing, preferably also includes doses which are equal to the previous one. For example, only the initial dose may be lower and all subsequent doses may be the same (and higher than the initial dose) or the initial dose may be lower, the second dose may be higher than the initial dose, but lower than all subsequent doses, with all subsequent doses being preferably the same (and higher than the initial dose and the second dose). It is thus preferred that one or more of the subsequent doses are escalating doses, i.e. doses, which are higher than the previous dose. The final dose of a treatment cycle typically reflects the highest amount of antibody to be administered within one treatment cycle; i.e. the maximum dose. In particular, at the end of any treatment cycle one, two, three, four, five or more administrations reflecting the maximum dosing may be foreseen. In general, the guiding principle for dose escalation is to avoid exposing a patient to sub-therapeutic doses while preserving safety and maintaining rapid accrual.

Preferably, within one and the same treatment cycle no single dose is thus lower than the previous one. Whenever a subsequent dose is a second dose, the previous dose is the initial dose ("first dose" or "starting dose"). Where the subsequent dose is a third dose, the previous dose is the second dose. Thus, the term "previous dose" within the meaning of the present application refers to the dose immediately preceding the current dose to be administered to the patient, said patient being diagnosed with at least one type of cancer.

Typically, a single treatment cycle includes at least an initial (first) dose and a second dose. In a preferred embodiment a single treatment cycle may include an initial (first) dose, a second dose, a third dose, a fourth dose, a fifth dose and preferably, additionally a sixth dose. Within a single treatment cycle a subsequent dose may be preferably administered 1, 2, 3, 4, 5, 6, 7, 8, 9, 10, 11, 12, 13, 14, 15, 16, 17, 18, 19, 20 or 21 days after the previous dose, preferably the subsequent dose is administered 2 - 15 days after the previous dose, more preferably the subsequent dose is administered 5 - 10 days after the previous dose, even more preferably the subsequent dose is administered 6 - 8 days after the previous dose, and most preferably the subsequent dose is administered about 7 days after the previous dose. In other words, it is particularly preferred that the antibody, or the antigen binding fragment thereof, is administered weekly, preferably a single dose per week, for example via the intravesical route. The administration of subsequent doses of the said antibody or antigen binding fragment thereof was shown to reduce the (exceeding) release of proinflammatory cytokines. Intermittent rising levels of proinflammatory cytokines are typically reduced below detection level over the course of the treatment. Simultaneously, it promotes cytotoxic immune responses against neoplasms in the urinary tract.

Preferably, the antibody, or the antigen binding fragment thereof, for use according to the present invention is administered at a single dose in a range of 0.1 to 5000 $\mu$g, preferably 1 to 1000 $\mu$g or 5 to 500 $\mu$g, more preferably 10 to 300 $\mu$g, most preferably 20 to 100 $\mu$g. In other words, each dose of the antibody, or the antigen binding fragment thereof, for use according to the present invention is in a range of 0.1 to 5000 $\mu$g, preferably 1 to 1000 $\mu$g or 5 to 500 $\mu$g, more preferably 10 to 300 $\mu$g, most preferably 20 to 100 $\mu$g. In the context of the present invention, a "single dose" (or "each dose") is an individual dose, which is administered to one patient at one administration time.

The total dose (as the sum of each single dose within a treatment cycle) of the antibody, or the antigen binding fragment thereof, for use according to the present invention per treatment cycle is preferably from 100 $\mu$g to 1500 $\mu$g, more preferably from 200 $\mu$g to 1000 $\mu$g, and even more preferably from 300 $\mu$g to 800 $\mu$g.

Preferably, the initial dose of the antibody, or the antigen binding fragment thereof, is in a range of 0.5 to 500 $\mu$g, preferably 1 to 200 $\mu$g, more preferably 5 to 100 $\mu$g, most preferably 10 to 70 $\mu$g. The initial dose is the first and preferably the lowest dose of one treatment cycle.

It is also preferred that a single dose of the multispecific antibody or antigen binding fragment thereof to be administered is in the range 0.5 to 1000 $\mu$g, more preferably from 1 to 500 $\mu$g, even more preferably from 5 to 200 $\mu$g, and most preferably from 10 to 150 $\mu$g.

Preferably, the first subsequent dose exceeds the amount administered as initial dose, preferably by a factor of 1.1 to 10.0, more preferably by a factor of 1.2 to 5.0 and even more preferably by a factor of 1.5 to 3.0, and, optionally, the second subsequent dose and each following subsequent dose exceeds the amount administered as initial dose by a factor of 1.1 to 10.0, preferably by a factor of 1.5 to 5.0.

Therein, the third, fourth and fifth subsequent dose may be the same as the second subsequent dose. Preferably, the second subsequent dose is higher than the first subsequent dose. Optionally, a third subsequent and each following dose may be a repeated dose of the first or second subsequent dose, i.e. the dose may stay constant. Alternatively, the third and each further subsequent dose is higher than the second subsequent dose within the same treatment cycle.

Correspondingly, the multispecific antibody, or the antigen binding fragment thereof, as described herein is also used for the preparation of a pharmaceutical composition for the treatment of an individual diagnosed with cancer, wherein said pharmaceutical composition is preferably administered intravesically and wherein preferably said pharmaceutical composition is administered by such a volume that it corresponds to an initial dose of the multispecific antibody or antigen binding fragment thereof (dissolved in solution) from 10 $\mu$g to 200 $\mu$g, preferably from 15 $\mu$g to 100 $\mu$g, e.g. 20 $\mu$g or 50 $\mu$g and/or a maximum dose of 50 to 200 $\mu$g, e.g. 100 $\mu$g.

The maximum dose (within a treatment cycle) is preferably selected from a range of 25 $\mu$g to 1000 $\mu$g, preferably from 50 $\mu$g to 500 $\mu$g, more preferably 75 $\mu$g - 150 $\mu$g, e.g. 100 $\mu$g.

Preferably, within one treatment cycle each subsequent dose is administered 1 to 31 days after the previous dose, preferably 1 to 21 days after the previous dose, more preferably 5 to 10 days after the previous dose and even preferably 7 days after the previous dose. That dose is preferably the "subsequent dose". Each single dose is preferably administered intravesically, e.g. every 7 days (weekly). Especially for elderly patients, which show the highest incidence and prevalence of neoplastic diseases of the urinary bladder, administrations of low frequency such as weekly administrations or less are preferred.

The antibody, or the antigen binding fragment thereof, as described herein is typically for use in a treatment of a neoplasm of the urinary tract, wherein said treatment preferably encompasses one single or repeated treatment cycles.

Each treatment cycle may be repeated once, twice, three times or more often. Typically, a treatment cycle of e.g. 6 to 10 doses to be administered every 1 to 30 days, e.g. every 1 to 10 days, preferably every 7th day, will last for a period of between 30 to 60 days. Several treatment cycles are typically interrupted by treatment-free periods of between 1 and 12 months, preferably at least 2 months, more preferably at least 3 months, even more preferably at least 4 months and most preferably at least 6 months. Thereafter, the treatment may be repeated by another treatment cycle which may be essentially identical to the previous treatment cycle (identical dosing and administration protocol). The dosing of the second, third and any further treatment cycle may alternatively also be modified as compared to the previous treatment cycle (depending on the effects observed in the course of the previous cycle). Typically, the dosing of each single administration of the second and third treatment cycle is either the same or slightly (e.g. by up to factor 3.0) increased over the initial dosing. At the end of the treatment protocol, e.g. by the fourth, fifth or sixth treatment cycle of the dosing of each single administration may be lowered again, e.g. to reflect the dosing of or even less than the first cycle.

In a preferred embodiment, a (single) dose of the antibody, or the antigen binding fragment thereof, is administered once a week, e.g. for 2, 3, 4, 5, 6, 7, 8, 9, 10, 11, 12, 13 or 14 weeks. Alternatively, a (single) dose of the antibody, or the antigen binding fragment thereof, is, e.g., administered at least twice per week, e.g. daily. Daily administration is preferably for 2, 3, 4, 5, 6, 7, 8, 9, 10, 11, 12, 13, 14, 15, 16, 17, 18, 19, 20, 21, 22, 23, 24, 25, 26, 27, 28, 29 or 30 consecutive days. To reduce impact on the patient's quality of life and to augment compliance, administration preferably is less frequent than daily, e.g. only every second, third, fourth, fifth or sixth day. Most preferably, the administration frequency is at most weekly, more preferably only every second or third week. In certain embodiments of the present invention, the administration may be performed only one a month. However, in the context of the present invention, once-a-week administrations are preferred as such a time interval balances well compliance on the one hand and contiguous medical surveillance by the practitioner on the other hand.

*Combination therapy*

The antibody, or the antigen binding fragment thereof, for use according to the present invention may be administered as stand-alone therapy or in combination with one or more other components (also referred to herein as "combination agents").

Preferably, the antibody, or the antigen binding fragment thereof, for use according to the present invention is administered as stand-alone therapy, for example after surgical removal of the tumor. Thereby, the term "stand-alone therapy" means in particular that the antibody, or the antigen binding fragment thereof, for use according to the present invention is the only agent, which is administered to treat the neoplasm of the urinary tract as described herein. Preferably, no other agents or drugs are required (in combination with the antibody, or the antigen binding fragment thereof, for use according to the present invention) to treat the neoplasm of the urinary tract as described herein.

The antibody, or the antigen binding fragment thereof, for use according to the present invention is particularly preferred as stand-alone therapy in all cancer stages. The antibody, or the antigen binding fragment thereof, for use according to the present invention is particularly preferred as stand-alone therapy in early cancer stages, in particular at stage 0a or at stage 0is of bladder cancer.

For example, the antibody, or the antigen binding fragment thereof, for use according to the present invention may not be administered in combination with activated peripheral blood lymphocytes (PBLs). Furthermore, the antibody, or the antigen binding fragment thereof, for use according to the present invention may, for example, not be administered in combination with IL-2.

It is also preferred that the antibody, or the antigen binding fragment thereof, as described herein is administered in combination, in particular in combination with an anti-cancer drug or in combination with autologous immune effector cells. Such a combination therapy may be used in all cancer stages. However, a combination therapy as described herein is particularly preferred in later cancer stages, in particular at stage I or at stage II of bladder cancer.

The term "combination therapy" or "administered in combination", as used herein, refers in particular to an administration ensuring that the components (drugs) of the combination therapy are - at least at a certain time point - simultaneously exerting their effects in the (human) body. In other words, if the administration of a first component precedes the administration of a second component for such a long time that the first component is no longer available and/or effective in the (human) body when the second component is administered (e.g., due to degradation processes), this is typically not considered as "combination therapy" or as "administered in combination" in the sense of the present invention. The skilled person typically takes the half-time of the components into account when envisaging a combination therapy.

Preferably, the antibody, or the antigen binding fragment thereof, as described herein is administered in combination with adoptive cell transfer. In general, the cells to be transferred may have originated from the patient him- or herself and then been altered before being transferred back, or, they may have come from another individual. The cells are most commonly derived from the immune system, with the goal of transferring improved immune functionality and characteristics along with the cells back to the patient. Transferring autologous cells, or cells from the patient, minimizes graft-versus-host disease (GVHD). Accordingly, it is preferred that the antibody, or the antigen binding fragment thereof, as described herein is administered in combination with autologous immune effector cells, preferably PBMCs (peripheral blood mononuclear cells). In particular if the antibody, or the antigen binding fragment thereof, as described herein is administered locally, such as intravesically, it is preferably administered together with autologous immune effector cells, preferably PBMCs (peripheral blood mononuclear cells). Thereby, the locally available immune effector cell population is increased. Autologous PBMCs can be obtained from the peripheral blood of the patient according to standard methods like ficoll density centrifugation. PBMCs can be applied in the range of $10^4$-$10^8$ cells.

It is also preferred that the antibody, or the antigen binding fragment thereof, as described herein is administered in combination with an anti-cancer drug. An anti-cancer drug may be any small or large molecule or compound, which is therapeutically active in the treatment of cancer, in particular of cancer of the urinary tract. Such anti-cancer drugs, in particular anti-cancer drugs for treatment of cancers of the urinary tract, are well known to the skilled person. Moreover, also combinations of different anti-cancer drugs may be administered in combination with the antibody, or the antigen binding fragment thereof, as described herein.

Preferably, the anti-cancer drug to be administered in combination with the antibody, or the antigen binding fragment thereof, as described herein is a cytotoxic agent, an anti-cancer antibody or BCG (Bacillus Calmette-Guerin). More preferably, the anti-cancer drug to be administered in combination with the antibody, or the antigen binding fragment thereof, as described herein is a cytotoxic agent or an anti-cancer antibody. Even more preferably, the anti-cancer drug to be administered in combination with the antibody, or the antigen binding fragment thereof, as described herein is a cytotoxic agent.

Cytotoxic agents prevent or inhibit cell function, thereby preventing rapid growth and division of cancer cells and other cells in the body. Preferred cytotoxic agents include alkylating agents, antimetabolites, anti-microtubule agents, topoisomerase inhibitors and cytotoxic antibiotics. Preferably, the cytotoxic agent is selected from the group consisting of cisplatin, mitomycin (in particular mitomycin C), valrubicin, docetaxel, thiotepa, and gemcitabine.

The anti-cancer antibody is preferably a monoclonal, monospecific anti-cancer antibody, which is preferably selected from the group consisting of trastuzumab, alemtuzumab, atezolizumab, avelumab, bevacizumab, brentuximab vedotin, cetuximab, gemtuzumab ozogamicin, ibritumomab, tiuxetan, ipilimumab, nimotuzumab, ofatumumab, panitumumab, pembrolizumab, rituximab and tositumomab. It is also preferred that the anti-cancer antibody is a monoclonal, monospecific anti-cancer antibody, which is selected from the group consisting of trastuzumab, alemtuzumab, atezolizumab, avelumab, bevacizumab, brentuximab vedotin, cetuximab, gemtuzumab ozogamicin, ibritumomab, tiuxetan, ipilimumab, nimotuzumab, nivolumab, ofatumumab, panitumumab, pembrolizumab, rituximab and tositumomab.

Preferably, the antibody, or the antigen-binding fragment thereof, for use according to the present invention is administered in combination with an anticancer drug, in particular with an anticancer antibody, as described herein - but not in combination with (autologous) immune effector cells, such as, for example (activated) peripheral blood lymphocytes. This means that preferably in a combination therapy as described herein the antibody, or the antigen-binding fragment thereof, for use according to the present invention and the anticancer drug are the only agents, which are administered to treat the neoplasm of the urinary tract as described herein.

Alternatively, it is also preferred that the antibody, or the antigen-binding fragment thereof, for use according to the present invention is administered in combination with adoptive cell transfer as described herein - but not in combination with an anticancer drug as described herein. This means that preferably in a combination therapy as described herein the antibody, or the antigen-binding fragment thereof, for use according to the present invention and the (autologous) immune effector cells are the only agents, which are administered to treat the neoplasm of the urinary tract as described herein.

Moreover, it is also preferred that the antibody, or the antigen-binding fragment thereof, for use according to the present invention is administered in combination with (i) an anticancer drug as described herein, in particular with an anticancer antibody as described herein, and (ii) with adoptive cell transfer as described herein, in particular with (autologous) immune effector cells as described herein.

Preferably, the antibody, or the antigen binding fragment thereof, as described herein and the combination agent, such as an anti-cancer drug or (autologous) immune effector cells, are administered consecutively. For example, the antibody, or the antigen binding fragment thereof, as described herein is preferably administered before the combination agent, such as the anti-cancer drug or (autologous) immune effector cells. It is also preferred that the antibody, or the antigen binding fragment thereof, as described herein is administered after the combination agent, such as the anti-cancer drug or (autologous) immune effector cells.

In consecutive administration, the time between administration of the first component and administration of the second component is preferably no more than one week, more preferably no more than 3 days, even more preferably no more than 2 days and most preferably no more than 24 h are in between administration of the first component and administration of the second component. It is particularly preferred that both are administered at the same day with the time between administration of the first component (and administration of the second component being preferably no more than 6 hours, more preferably no more than 3 hours, even more preferably no more than 2 hours and most preferably no more than 1 h.

Alternatively, the antibody, or the antigen binding fragment thereof, as described herein may also be administered concurrently with the combination agent, such as an anticancer drug or (autologous) immune effector cells, i.e. at about the same time. "At about the same time", as used herein, means in particular simultaneous administration or that directly after administration of the combination agent, such as an anticancer drug or (autologous) immune effector cells, the antibody, or the antigen binding fragment thereof, as described herein is administered or directly after administration of the antibody, or the antigen binding fragment thereof, as described herein the combination agent, such as an anticancer drug or (autologous) immune effector cells, is administered. The skilled person understands that "directly after" includes the time necessary to prepare the second administration - in particular the time necessary for exposing and disinfecting the location for the second administration as well as appropriate preparation of the "administration device" (e.g., syringe, pump, etc.). Simultaneous administration also includes if the periods of administration of the antibody, or the antigen binding fragment thereof, as described herein and of the combination agent, such as an anti-cancer drug or (autologous) immune effector cells, overlap or if, for example, one component is administered over a longer period of time, such as 30 min, 1 h, 2 h or even more, e.g. by infusion, and the other component is administered at some time during such a long period. Administration of the antibody, or the antigen binding fragment thereof, as described herein and of the combination agent, such as an anti-cancer drug or (autologous) immune effector cells, at about the same time is in particular preferred if different routes of administration and/or different administration sites are used.

Preferably, the antibody, or the antigen binding fragment thereof, as described herein and the combination agent, such as an anticancer drug or (autologous) immune effector cells, are (i) comprised by the same composition or (ii) are administered separately.

If they are comprised by the same composition, a pharmaceutical composition as described in the following may be used.

Separate administration is in particular preferred, if different routes of administration are envisaged. For example, the combination agent, such as an anticancer drug or (autologous) immune effector cells, may be administered systemically, such as i.v. or p.o., and the antibody, or the antigen binding fragment thereof, as described herein may be administered intravesically.

*Pharmaceutical composition for use in the treatment of a neoplasm of the urinary tract*

In a further aspect, the present invention provides a pharmaceutical composition comprising the antibody, or the antigen binding fragment thereof, as described herein for use in the treatment of a neoplasm of the urinary tract as described herein.

More particularly, the present invention relates to a pharmaceutical composition for the treatment of a neoplasm of the urinary tract as described above, e.g. by intravesical administration. The pharmaceutical composition comprises the multispecific antibody, or the antigen binding fragment thereof, as described above. Preferably, an appropriate dosing (dosing regimen) is applied for administering the antibody, or the antigen binding fragment thereof, as described herein, such as the doses regimen, treatment schedule and route of administration as described above for the antibody, or the antigen binding fragment thereof.

Thus, the present invention provides in a further aspect a pharmaceutical composition for use in the treatment of a neoplasm of the urinary tract, wherein said pharmaceutical composition comprises the antibody, or the antigen binding fragment thereof, as described herein, in particular a bispecific, e.g. a bispecific trifunctional, antibody. Preferred embodiments of the pharmaceutical composition refer to preferred embodiments of the antibody for use according to the present invention as described herein. Preferred uses of the pharmaceutical composition refer to preferred uses of the antibody, or the antigen binding fragment thereof, as described herein.

Preferably, the pharmaceutical composition comprises the antibody, or the antigen binding fragment thereof, as described herein in a "therapeutically effective amount", this being sufficient to show benefit to the individual. The actual amount administered, and rate and time-course of administration, will depend on the nature and severity of the neoplasm of the urinary tract.

The pharmaceutical composition may also comprise a combination of at least two different antibodies, or antigen binding fragments thereof, as described herein. Thereby, different targets which are associated with the same neoplasm can be addressed which preferably increases efficacy. Thus, the dose of the single drug may advantageously be reduced.

Preferably, the pharmaceutical composition further comprises a pharmaceutically acceptable carrier and/or vehicle, or any excipient, buffer, stabilizer or other materials well known to those skilled in the art.

As a further ingredient, the pharmaceutical composition may in particular comprise a (compatible) pharmaceutically acceptable carrier and/or vehicle. In the context of the present invention, a pharmaceutically acceptable carrier typically includes the liquid or non-liquid basis of the pharmaceutical composition. The term "compatible" as used herein means that these constituents of the pharmaceutical composition are capable of being mixed with the antibody, or the antigen-binding fragment thereof, as defined above in such a manner that no interaction occurs which would substantially reduce the pharmaceutical effectiveness of the pharmaceutical composition under typical use conditions. Pharmaceutically acceptable carriers and vehicles must, of course, have sufficiently high purity and sufficiently low toxicity to make them suitable for administration to a subject to be treated.

Preferably, the pharmaceutical composition is in the form of a lyophilized powder or in the form of a liquid composition, preferably an aqueous solution. Hence, the pharmaceutical composition of the present invention may be provided as a dried, lyophilized powder or, more preferably in solution (dissolved in a vehicle). If provided as lyophilized powder by the manufacturer, it is usually dissolved in an appropriate solution (aqueous solution; such as water for injection or saline, optionally buffered such as PBS) shortly prior to administration. Vials of liquid medication can be single use or multi-use.

In another preferred embodiment, the antibody and/or the pharmaceutical composition for use according to the present invention is not lyophilized. Thus, it is preferred that the antibody, or the antigen binding fragment thereof, for use according to the present invention is not lyophilized, but provided in a solution, preferably in an aqueous solution, more preferably in an aqueous buffered solution.

It is thus particularly preferred that the pharmaceutical composition is provided in liquid form. Thus, the pharmaceutically acceptable carrier will typically comprise one or more (compatible) pharmaceutically acceptable liquid carriers. Examples of (compatible) pharmaceutically acceptable liquid carriers include pyrogen-free water,isotonic saline or buffered (aqueous) solutions, e.g. citrate buffered solutions; polyols, such as, for example, polypropylene glycol, glycerol, sorbitol, mannitol and polyethylene glycol; alginic acid, further inorganic or organic polymers such as PLGA, preferably to provide a sustained release effect to the present active agent. Preferably, in a liquid pharmaceutical composition the carrier may be pyrogen-free water; isotonic saline or buffered (aqueous) solutions, e.g. phosphate, citrate etc. buffered solutions. Particularly for injection or instillation of the pharmaceutical composition, water or preferably a buffer, more preferably an aqueous buffer, such as citrate buffer, may be used.

Accordingly, it is preferred that the pharmaceutical composition comprises a buffer, preferably an organic acid buffer (i.e. a buffer based on an organic acid), such as citrate buffer, succinate buffer and tartrate buffer, more preferably the pharmaceutical composition comprises a citrate buffer. The organic acid buffer is thus preferably selected from the group consisting of citrate buffer, succinate buffer, tartrate buffer, and phosphate-citrate buffer, more preferably selected from the group consisting of citrate buffer, succinate buffer and tartrate buffer. It is particularly preferred that the buffer is a citrate buffer. In general, a buffer may (also) contain a sodium salt, preferably at least 30 mM of a sodium salt, a calcium salt, preferably at least 0.05 mM of a calcium salt, and/or optionally a potassium salt, preferably at least 1 mM of a potassium salt. The sodium, calcium and/or potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, NaI, NaBr, $Na_2CO_3$, $NaHCO_3$, $Na_2SO_4$, examples of the optional potassium salts include e.g. KCl, KI, KBr, $K_2CO_3$, $KHCO_3$, $K_2SO_4$, and examples of calcium salts include e.g. $CaCl_2$, $CaI_2$, $CaBr_2$, $CaCO_3$, $CaSO_4$, $Ca(OH)_2$. Furthermore, organic anions of the aforementioned cations may be contained in the buffer.

The pharmaceutical composition may also comprise saline (0.9% NaCl), Ringer-Lactate solution or PBS (phosphate buffered saline). For example, the pharmaceutical composition may be provided as stock solution of the antibody, or the antigen binding fragment thereof, in an appropriate buffer, such as an organic acid buffer as described above, preferably citrate buffer, and only just before administration that stock solution may be diluted by saline (0.9% NaCl), Ringer-Lactate solution or PBS to achieve the antibody concentration to be administered.

Furthermore, one or more compatible solid or liquid fillers or diluents or encapsulating compounds may be used as well for the pharmaceutical composition, which are suitable for administration to a subject to be treated. Further examples of compounds which may be comprised by the pharmaceutical composition include sugars, such as, for example, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid. In addition, preservatives, stabilizers, antioxidants and/or other additives may be included, as required. The pharmaceutical composition may, thus, also comprise stabilizing agents such as Tween® 80 or Tween® 20. Optionally, excipients conferring sustained release properties to the antibody, or the antigen binding fragment thereof, as described herein may also be comprised by the pharmaceutical composition.

The pharmaceutical composition may further comprises an anticancer drug as described above.

Preferably, the pH value of the pharmaceutical composition is from 6.5 to 8.2, preferably from 7.0 to 7.8, more preferably from 7.2 to 7.6, most preferably from 7.4. Such a pH is adapted to both, the urinary tract environment and the stability needs of the present active agent. Accordingly, a pharmaceutical composition having such a pH is suitable for e.g. local administration in the urinary tract without causing irritation and without impairing the stability of the antibody, or the antigen binding fragment as described herein.

In a preferred embodiment, the pharmaceutical composition comprises no further components in addition to (i) the antibody, or the antigen binding fragment thereof, as described herein; (ii) a buffer as described herein; and, optionally, (iii) water for injection, saline and/or PBS.

*Subjects to be treated*

The subject to be treated is preferably a human or non-human animal, in particular a mammal or a human. Amongst humans, the inventive treatment protocol for administering the antibody, or the antigen binding fragment thereof, as described above may be particularly useful. Preferably, subjects are patients having a neoplasm of the urinary bladder. For example, young (less than 15 years old) or elderly (more than 60 years old) patients may be treated according to the present invention. Especially preferred are older patients, being the population group with the highest incidence and prevalence of a neoplasm of the urinary tract. For elderly patients, it is of particular advantage to administer the drug by a route which requires a physician, as thereby compliance is ensured. At the same time, the administration should be preferably pain-free.

In general, patients having a neoplastic disease of the urinary tract, irrespective of their age, who are preferably not under immunosuppressive treatment may particularly benefit from the use of the multispecific antibody or antigen binding fragment thereof according to the invention.

*Kit comprising the multispecific antibody*

A kit comprising the antibody or the antigen binding fragment thereof, as described herein or the pharmaceutical composition described herein and a package insert or label with directions to treat a neoplasm of the urinary tract, preferably by local administration in the urinary tract, is provided.

The kit may be comprised in one or more containers, preferably in one container.

Such a kit is preferably used in prevention and/or treatment of a neoplasm of the urinary tract as described herein.

Additionally, the kit may also comprise a (i) urinary catheter, (ii) a urinary catheter syringe, (iii) a further anti-cancer drug as described above and/or (vi) a solution suitable for reconstituting the lyophilized powder of the pharmaceutical composition or a solution suitable for diluting the stock solution of the pharmaceutical composition.

*Method for treating a subject suffering from a neoplasm of the urinary tract*

A method for treating a subject suffering from a neoplasm of the urinary tract by administering a multispecific antibody, or an antigen binding fragment thereof, the multispecific antibody comprising

(i) a specificity against a T cell surface antigen, and
(ii) a specificity against a tumor-associated cell surface antigen,

is provided.

Preferably, the antibody, or the antigen binding fragment thereof, is as described above. More preferably, the pharmaceutical composition as described above is administered. Thus, for said method of treatment, the multispecific antibody or antigen binding fragment thereof is typically provided as a pharmaceutical composition. The pharmaceutical composition or the antibody is preferably administered locally in the urinary tract, e.g. intravesically.

Further, the present method of treatment preferably also refers to a combination therapy allowing the administration of the antibody, or the antigen binding fragment thereof, as described herein to be combined with another anti-cancer drug as described herein. The anti-cancer drug may be administered by any suitable route, e.g. systemically or intravesically, preferably by an administration being separate from the administration of the antibody as described herein.

Preferably, the antibody, or the antigen binding fragment thereof, as described herein is preferably administered in a dosage regimen, in a treatment protocol and via a route of administration as described above, e.g. by local administration in the urinary tract, e.g. by intravesical administration. Accordingly, in the method of treatment as described herein the antibody, or the antigen binding fragment thereof, as described herein is preferably administered as described above.

BRIEF DESCRIPTION OF THE FIGURES

In the following a brief description of the appended figures will be given. The figures are intended to illustrate the present invention in more detail. However, they are not intended to limit the subject matter of the invention in any way.

Figure 1 shows for Example 1 (A) the binding of catumaxomab to CD3-expressingJurkat cells in 100% urine. $5 \times 10^5$

target cells were incubated at 2-8°C for 60 min at the indicated catumaxomab concentrations and (B) the binding of catumaxomab to EpCAM positive HCT-8 cells in 100% urine. 5 x $10^5$ target cells were incubated at 2-8°C for 60 min at the indicated catumaxomab concentrations.

Figure 2    shows for Example 2 the biological activity of catumaxomab in 10% urine milieu by applying an allogeneic cytotoxicity assay. 1x$10^5$ Peripheral blood mononuclear cells (PBMC) were mixed with 1x$10^4$ EpCAM+ HCT-8 tumor cells in the presence of the indicated amounts of antibodies. Tumor cell killing was calculated and plotted in [%].

Figure 3    shows for Example 3 the endoscopic imaging of the bladder of patient 1 about two weeks before the intravesical application of catumaxomab. The arrow in panel F indicates a growing papillary structure typical for superficial bladder cancer.

Figure 4    shows for Example 3 the endoscopic imaging of the bladder of patient 1 two weeks after the last application of catumaxomab. The mucosa was completely normal again in the area analyzed before treatment.

EXAMPLES

In the following, particular examples illustrating various embodiments and aspects of the invention are presented.

Example 1: Binding activity of catumaxomab in an urine milieu

The goal of this study was to evaluate the binding activity of catumaxomab in an urine milieu. To evaluate the binding activity of catumaxomab, cell lines expressing target antigens corresponding to catumaxomab's specificities are used. Since the bispecific binding activity of catumaxomab is characterized by binding to CD3+ T-cells and to EpCAM+ tumor cells (Chelius et al. Structural and functional characterization of the trifunctional antibody catumaxomab. mAbs 2(3):1-12 (2010)), binding activity of catumaxomab was evaluated by FACS-analysis (flow cytometry) using cell lines expressing the relevant target antigens, namely CD3+ Jurkat cells and EpCAM+ HCT-8 cells. To evaluate the binding activity of catumaxomab in an urine milieu, binding of catumaxomab in neutral phosphate-buffered saline PBS (buffer control) was compared to the binding in two human urine samples (100% urine).

5 x $10^5$ target cells (CD3+ Jurkat cells or EpCAM+ HCT-8 cells) were incubated at 2-8°C for 60 min at catumaxomab concentrations of 0.0 μg/ml, 0.01 μg/ml, 0.02 μg/ml, 0.04 μg/ml, 0.08 μg/ml, 0.15 μg/ml, 0.30 μg/ml, 0.60 μg/ml, 1.20 μg/ml, 2.40 μg/ml, 4.80 μg/ml, 9.60 μg/ml and 19.20 μg/ml. Target cells were resuspended either in PBS buffer or in urine samples (100% urine). Then, cells were washed two times and cell bound catumaxomab was detected with Fluorescence labeled (FITC) rat-anti-mouse IgG (Jurkat) or mouse-anti-rat IgG (HCT-8) secondary detection antibodies (Dianova). Positively stained cells were evaluated using a FACS-Calibur cytometer (Becton Dickinson).

Results are shown in Figure 1. Figure 1 shows the successful binding of catumaxomab to CD3 expressing Jurkat cells (Fig. 1A) and EpCAM positive HCT-8 cells (Fig. 1B) in buffer control and in 100% urine. In particular, there was no difference in binding in urine sample 2 compared to buffer control. Binding of catumaxomab in urine sample 1 showed also no difference to buffer control at the higher antibody concentrations. A slight impairment of binding in urine sample 1 compared to buffer control was observed at lower antibody concentrations but not at higher ones. Thus, results indicate that catumaxomab is able to bind to its target antigens EpCAM and CD3 without impairment even in 100 % urine milieu.

Example 2: Biological activity of catumaxomab in an urine milieu

Next, the biological activity of catumaxomab in an urine milieu was investigated. The bispecific antibody catumaxomab induces the efficient destruction of EpCAM+ tumor cells by redirection and activation of T-cells and FcγR+ immune cells (Lindhofer H, Hess J, Ruf P: Trifunctional Triomab® antibodies for cancer therapy. In: Bispecific Antibodies. Kontermann RE (ed.). Springer Heidelberg Dordrecht London New York, 289 (2011)). Thus, the biological activity of catumaxomab is ideally evaluated *in vitro* by using allogeneic cytotoxicity assays wherein the killing of targeted tumor cells is analyzed (Chelius et al. Structural and functional characterization of the trifunctional antibody catumaxomab. mAbs 2(3):1-12 (2010)).

Accordingly, the catumaxomab-mediated killing of EpCAM+ HCT-8 tumor cells was evaluated in an allogeneic cytotoxicity assay. Thereby, the "killing activity" of catumaxomab in PBS buffer control was compared to its activity in samples containing 10 % urine. Three different urine samples were tested. Peripheral blood mononuclear cells (PBMC) (1x$10^5$ cells) from a healthy donor were isolated by Ficoll density centrifugation and subsequently mixed with 1x$10^4$ EpCAM+ HCT-8 tumor cells in the presence of the indicated amounts of antibodies in 96-well flat bottomed plates. 10 Vol% of urine were added in "urine sample 1", "urine sample 2" and "urine sample 3". After 4 days of co-cultivation at 37°C and 5% $CO_2$, soluble PBMC were washed twice with PBS without $Ca^{2+}$ and $Mg^{2+}$. Adherent tumor cells were then stained with tetrazolium hydroxide (XTT, cell proliferation kit II, Roche) and proliferation was measured until the $OD_{650\ nm-490\ nm}$ of the tumor cell control samples reached 2.5 - 3.0. Tumor cell killing (%) was calculated according to the formula:

$$(OD_{tumor\ cells\ +\ PBMC} - OD_{tumor\ cells\ +\ PBMC\ +\ antibody}) / (OD_{tumor\ cells\ +\ PBMC} - OD_{medium}) \times 100\%.$$

Each sample was measured in duplicates and mean values were calculated.

Results are shown in Figure 2. Figure 2 shows that the biological activity of catumaxomab in urine samples was not impaired in comparison to the buffer control. In all cases 100% killing of tumor cells was observed down to catumaxomab concentrations as low as 4 ng/ml. It is noted that the concentrations was set to 10% for technical analytical reasons only: Higher concentrations of urine disturb the analysis in that assay because of the influence of the urine itself on tumor growth and tumor cell killing. However, it is not expected that higher urine concentrations would have provided different results with regard to the cytotoxic properties of catumaxomab, which is further supported by the positive *in vivo* results shown in the following examples.

Example 3: Compassionate use treatment of a 70 years old female bladder cancer patient

A 70 years old female patient ("patient 1") with urothelial cell carcinoma first diagnosed in 2004 and confirmed EpCAM-positive tumor cells by urine cytology was treated with 6 doses of catumaxomab antibody administered intravesically according to the treatment schedule summarized in Table 1 below. Each antibody dose was administered to the empty bladder by a catheter in 40 ml PBS solution (pH 7,4) and held for at least two hours before voiding to allow the binding of the antibody.

Table 1: Treatment schedule of patient 1:

| Day | 0 | 7 | 13 | 21 | 28 | 35 |
|---|---|---|---|---|---|---|
| Catumaxomab ($\mu$g) | 20 | 50 | 100 | 100 | 100 | 100 |

Accordingly, patient 1 received 6 weekly instillations with increasing catumaxomab doses of up to 100 $\mu$g. The total amount of antibody applied was 470 $\mu$g.

Cytokine levels in plasma samples were analyzed by using the Luminex system 200 (Luminex, TX, USA) together with the premixed 8-plex fluorokine X-Map kit (R&D Systems, MN, USA) comprising the cytokines IL-2, IL-4, IL-6, IL-8, IL-10, IL-17, IFN-$\gamma$ and TNF-$\alpha$. Samples were collected at the times points as indicated in Tables 2A and 2B, stored at -20°C and measured in batch. Samples collected on treatment days were taken before antibody instillation. The detection limit of cytokines is 3.2 pg/ml. Systemic catumaxomab concentrations were measured by ELISA as described (Ruf et al. Pharmacokinetics, immunogenicity and bioactivity of the therapeutic antibody catumaxomab intraperitoneally administered to cancer patients. Br J Clin Pharmacol. 69(6): 617-625 (2010)). The quantification limit of the ELISA method is 125 pg/ml. HAMA (human anti-mouse antibodies) were quantified by using the medac ELISA kit (medac, Hamburg, Germany) following the instructions of the manufacturer. Negative samples (neg.) have values below 40 ng/ml.

Results are shown in Tables 2A and 2B below.

Table 2A: Systemic concentrations of cytokines IFN-$\gamma$, IL-10, IL-17, IL-2 and IL-4 in patient 1

| Day | Antibody ($\mu$g) | IFN-$\gamma$ (pg/ml) | IL-10 (pg/ml) | IL-17 (pg/ml) | IL-2 (pg/ml) | IL-4 (pg/ml) |
|---|---|---|---|---|---|---|
| 0 | 20 | < 3.2 | < 3.2 | < 3.2 | < 3.2 | < 3.2 |
| 1 | | < 3.2 | < 3.2 | < 3.2 | < 3.2 | < 3.2 |
| 7 | 50 | | | | | |
| 8 | | < 3.2 | < 3.2 | < 3.2 | < 3.2 | < 3.2 |
| 13 | 100 | < 3.2 | < 3.2 | < 3.2 | < 3.2 | < 3.2 |
| 21 | 100 | | | | | |
| 22 | | < 3.2 | < 3.2 | < 3.2 | < 3.2 | < 3.2 |

(continued)

| Day | Antibody ($\mu$g) | IFN-$\gamma$ (pg/ml) | IL-10 (pg/ml) | IL-17 (pg/ml) | IL-2 (pg/ml) | IL-4 (pg/ml) |
|---|---|---|---|---|---|---|
| 28 | 100 | < 3.2 | < 3.2 | < 3.2 | < 3.2 | < 3.2 |
| 29 | | < 3.2 | < 3.2 | < 3.2 | < 3.2 | < 3.2 |
| 35 | 100 | < 3.2 | < 3.2 | < 3.2 | < 3.2 | < 3.2 |
| 36 | | < 3.2 | < 3.2 | < 3.2 | < 3.2 | < 3.2 |
| 49 | | < 3.2 | < 3.2 | < 3.2 | < 3.2 | < 3.2 |

Table 2B: Systemic concentrations of cytokines IL-6, IL-8 and TNF-$\alpha$ and systemic catumaxomab antibody and HAMA in patient 1

| Day | Antibody ($\mu$g) | IL-6 (pg/ml) | IL-8 (pg/ml) | TNF-$\alpha$ (pg/ml) | Systemic Catumaxomab (pg/ml) | HAMA |
|---|---|---|---|---|---|---|
| 0 | 20 | < 3.2 | 8 | 7 | <125 | neg. |
| 1 | | < 3.2 | 9 | 6 | <125 | |
| 7 | 50 | | | | | |
| 8 | | 13 | < 3.2 | < 3.2 | <125 | |
| 13 | 100 | 10 | < 3.2 | < 3.2 | <125 | neg. |
| 21 | 100 | | | | | |
| 22 | | < 3.2 | < 3.2 | < 3.2 | <125 | |
| 28 | 100 | < 3.2 | < 3.2 | < 3.2 | <125 | neg. |
| 29 | | < 3.2 | < 3.2 | < 3.2 | <125 | |
| 35 | 100 | < 3.2 | < 3.2 | < 3.2 | <125 | neg. |
| 36 | | < 3.2 | < 3.2 | < 3.2 | <125 | |
| 49 | | < 3.2 | < 3.2 | < 3.2 | <125 | neg. |

According to the patient's own statements and the physical examinations of the attending physician the antibody treatment was very well tolerated without any obvious signs of negative side effects. Especially there were no reports of fever or flu-like symptoms. Accordingly, no significant systemic cytokine release was observed (Tables 2A and 2B). Only after the second instillation of 50 $\mu$g catumaxomab weak intermediate plasma levels of IL-6 of 13 and 10 pg/ml were measured. In the course of further treatment, IL-6 values returned below the detection limit (3.2 pg/ml). Low amounts of IL-8 and TNF-$\alpha$ were already detectable before treatment start - but no treatment-related induction of these cytokines was observed. On the contrary, the cytokine values of IL-8 and TNF-$\alpha$ decreased below the detection limit in the course of the treatment. This result suggests that chronic inflammation, which is often observed in the environment of tumors, could be reduced during the course of therapy.

As shown in Table 2B, concentrations of catumaxomab were below the quantification limit of 125 pg/ml and, thus, no systemic catumaxomab antibody was detectable in the blood of the patient. This indicates that the local antibody treatment (i.e. intravesical administration) of the bladder did not result in a systemic release of the drug. In accordance with this finding no induction of human anti-mouse antibodies (HAMA) occurred. 14 days after the end of the antibody therapy, which comprised six weekly drug applications, the patient was still negative for HAMA (Table 2B).

Urine samples of the patient were analyzed for EpCAM+ tumor cells before and after the treatment. EpCAM+ tumor cells were detected by an established and described immunocytochemistry protocol (Jäger et al. Immunomonitoring Results of a Phase II/III Study of Malignant Ascites Patients Treated with the Trifunctional Antibody Catumaxomab (Anti-EpCAM x anti-CD3). Cancer Res. 72(1):24-32 (2012)) that was modified for analysis of urine samples. Briefly, cells in 30 ml urine were centrifuged on cytospins. Slides were double stained for EpCAM and cytokeratin. EpCAM staining was performed with the EpCAM-specific antibody HO-3 (Ruf et al. Characterisation of the new EpCAM-specific antibody HO-3: implications for trifunctional antibody immunotherapy of cancer. Br J Cancer 97 (3):315-321 (2007)) directly labeled with Alexa Fluor 594 Texas Red. For cytokeratin staining the anti-cytokeratin 8, 18, 19 antibodies A45B-B3 (Micromet) together with the corresponding Alexa Fluor 488-labeled secondary anti-mouse IgG1 detection antibody (Molecular Probes) were used. All cytospins were analyzed by a computerized image analysis system (MDS, Applied Imaging) counting double stained cells. For the quantification of CD45+ leucocytes additional cytospins were stained with anti-CD45 antibody (Caltag Laboratories, Hamburg, Germany) and its corresponding secondary anti-mouse IgG1 antibody Alexa Fluor 488 (Molecular Probes).

Results are shown in Table 3 below.

Table 3: Detection of EpCAM+ tumor cells in urine

| Day | 0 (before treatment start) | 36 | 39 | 148 | 211 | 339 | 701 |
|---|---|---|---|---|---|---|---|
| Number of tumor cells detected in 30 ml urinary sample | 23 | 0 | 0 | 0 | 0 | 0 | 0 |
| Number of leucocytes detected in 30 ml urinary sample | 496 | 6 | n.d. | n.d. | n.d. | n.d. | n.d. |

Immediately before treatment start (day 0) 23 tumor cells could be detected in 30 ml urine, whereas one day after the completion of the treatment (day 36) no tumor cells were detected anymore (Table 3). Most importantly, follow up samples on days 148, 211, 339 and 701 were still negative for tumor cells. For confirmation, a cytological analysis was performed by a further laboratory from an urine sample taken on day 703 (February 17, 2017), which also showed no evidence for tumor cells.

This result shows the efficacy of the treatment. Tumor cells are frequently found in the urine, especially of advanced bladder cancer patients, and urine cytology is a recommended method in the follow-up care of non-muscle-invasive urothelial carcinomas of the bladder (Babjuk et al. EAU guidelines on non-muscle-invasive urothelial carcinoma of the bladder, the 2011 Update. Eur Urol 59:997-1008 (2011)). In addition to EpCAM+ tumor cells, leucocytes were also analyzed before and after treatment at the days indicated in Table 3. Before treatment start (day 0), 496 CD45+ leucocytes could be detected, whereas one day after the completion of the treatment (day 36) only 6 leucocytes were detected. This result suggests that (i) immune effector cells were present intravesically at treatment start, which could be redirected against the tumor site by the trifunctional antibody, and (ii) the reduction of leucocytes one day after the completion of the treatment may indicate that leucocytes are still immobilized at the tumor site.

About two weeks before the first intravesical application of catumaxomab, the situation in the bladder was determined by endoscopic imaging of the tumor lesions. Results are shown in Figure 3. The arrow in Figure 3F indicates a growing papillary structure typical for superficial bladder cancer.

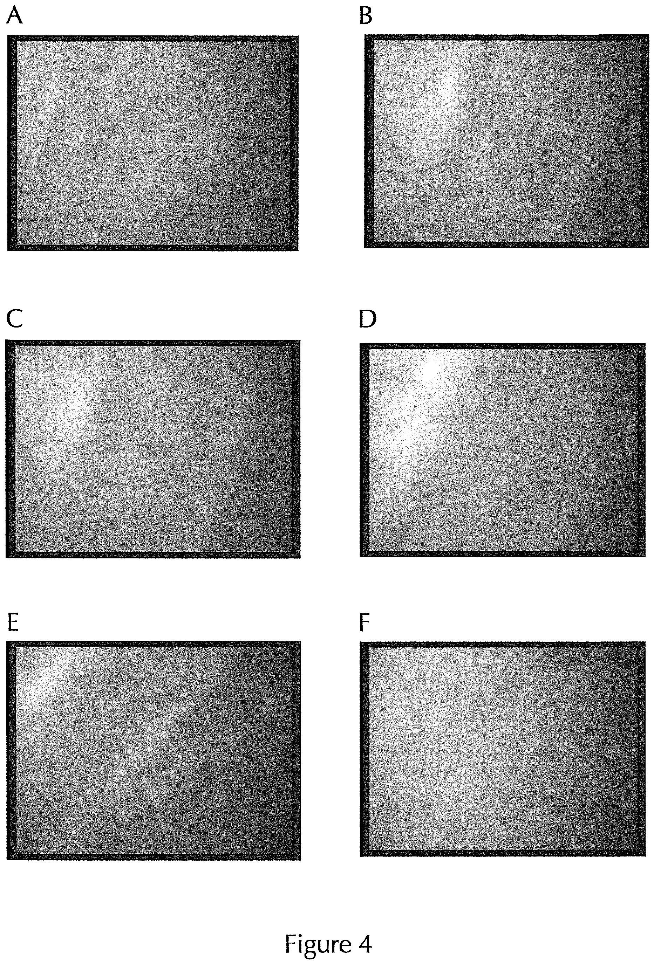

Two weeks after the last intravesical catumaxomab application the situation in the bladder of patient 1 was re-evaluated by endoscopic imaging. Results are shown in Figure 4. It follows from Figure 4's endoscopic imaging of the bladder of patient 1 two weeks after the last application of catumaxomab, that the mucosa was completely normal again in the same area as analyzed before (cf. Figure 3). This shows that the neoplastic conditions were cured by means of the multispecific antibody.

Example 4: Compassionate use treatment of a 72 years old male patient with bladder cancer

A 72 years old male patient ("patient 2") with bladder cancer after resection was treated with 6 doses of catumaxomab antibody administered intravesically according to the treatment schedule summarized in Table 4 below. Each antibody dose was administered to the empty bladder by a catheter in 40 ml PBS solution (pH 7.4) and held for at least two hours before voiding to allow the binding of the antibody.

Table 4: Treatment schedule of patient 2 - first treatment cycle

| Day | 0 | 7 | 14 | 21 | 28 | 35 |
|---|---|---|---|---|---|---|
| Catumaxomab ($\mu$g) | 20 | 50 | 100 | 100 | 100 | 100 |

After approximately 6 months the patient recidivated (detection of atypic cells in an urine sample with the Urovysion test, Abbott) and therefore received a second treatment cycle comprising 7 weekly doses of catumaxomab as indicated in Table 5. Each antibody dose was administered to the empty bladder by a catheter in 40 ml PBS solution (pH 7.4) and held for at least two hours before voiding to allow the binding of the antibody.

Table 5: Treatment schedule of patient 2 - second treatment cycle

| Day | 225 | 232 | 239 | 246 | 253 | 260 | 267 |
|---|---|---|---|---|---|---|---|

(continued)

| Catumaxomab (μg) | 50 | 100 | 100 | 100 | 100 | 100 | 100 |
|---|---|---|---|---|---|---|---|

Cytokine levels in plasma samples were analyzed by using the Luminex system 200 (Luminex, TX, USA) together with the premixed 8-plex fluorokine X-Map kit (R&D Systems, MN, USA) comprising the cytokines IL-2, IL-4, IL-6, IL-8, IL-10, IL-17, IFN-γ and TNF-α. Samples were collected at the indicated times points, stored at -20°C and measured in batch. Samples collected on treatment days were taken before antibody instillation. The detection limit of cytokines is 3,2 pg/ml. catu = catumaxomab. Systemic catumaxomab concentrations were measured by ELISA as described previously (Ruf et al. Pharmacokinetics, immunogenicity and bioactivity of the therapeutic antibody catumaxomab intraperitoneally administered to cancer patients. Br J Clin Pharmacol. 69(6): 617-625 (2010)). The quantification limit of the ELISA method is 125 pg/ml. HAMA (human anti-mouse antibodies) were quantified by using the medac ELISA kit (medac, Hamburg, Germany) following the instructions of the manufacturer. Negative samples have values below 40 ng/ml.

Results are shown in Tables 6A and 6B (first treatment cycle) and in Tables 7A and 7B (second treatment cycle).

Table 6A: Systemic concentrations of cytokines IFN-γ, IL-10, IL-17, IL-2 and IL-4 in patient 2 - first treatment cycle.

| Day | Antibody (μg) | IFN-γ (pg/ml) | IL-10 (pg/ml) | IL-17 (pg/ml) | IL-2 (pg/ml) | IL-4 (pg/ml) |
|---|---|---|---|---|---|---|
| 0 | 20 | < 3.2 | < 3.2 | < 3.2 | < 3.2 | < 3.2 |
| 7 | 50 | < 3.2 | < 3.2 | < 3.2 | < 3.2 | < 3.2 |
| 8 | | < 3.2 | < 3.2 | < 3.2 | < 3.2 | < 3.2 |
| 14 | 100 | | | | | |
| 15 | | < 3.2 | < 3.2 | < 3.2 | < 3.2 | < 3.2 |
| 21 | 100 | < 3.2 | < 3.2 | < 3.2 | < 3.2 | < 3.2 |
| 22 | | < 3.2 | < 3.2 | < 3.2 | < 3.2 | < 3.2 |
| 28 | 100 | < 3.2 | < 3.2 | < 3.2 | < 3.2 | < 3.2 |
| 29 | | < 3.2 | < 3.2 | < 3.2 | < 3.2 | < 3.2 |
| 35 | 100 | < 3.2 | < 3.2 | < 3.2 | < 3.2 | < 3.2 |
| 36 | | < 3.2 | < 3.2 | < 3.2 | < 3.2 | < 3.2 |

Table 6B: Systemic concentrations of cytokines IL-6, IL-8 and TNF-α and systemic catumaxomab antibody and HAMA in patient 2 - first treatment cycle.

| Day | Antibody (μg) | IL-6 (pg/ml) | IL-8 (pg/ml) | TNF-α (pg/ml) | Systemic Catumaxomab (pg/ml) | HAMA |
|---|---|---|---|---|---|---|
| 0 | 20 | < 3.2 | 9 | 9 | <125 | 40 |
| 7 | 50 | < 3.2 | 8 | 7 | <125 | 67 |
| 8 | | < 3.2 | 10 | 7 | 214 | |
| 14 | 100 | | | | 162 | |
| 15 | | 16 | 9 | 3 | | |
| 21 | 100 | 13 | 9 | 3 | <125 | 198 |
| 22 | | 13 | 10 | 3 | <125 | |
| 28 | 100 | < 3.2 | < 3.2 | < 3.2 | <125 | 163 |
| 29 | | < 3.2 | < 3.2 | < 3.2 | 170 | |
| 35 | 100 | < 3.2 | < 3.2 | < 3.2 | <125 | 60 |
| 36 | | < 3.2 | < 3.2 | < 3.2 | <125 | |

Table 7A: Systemic concentrations of cytokines IFN-γ, IL-10, IL-17, IL-2 and IL-4 in patient 2 - second treatment cycle.

| Day | Antibody (μg) | IFN-γ (pg/ml) | IL-10 (pg/ml) | IL-17 (pg/ml) | IL-2 (pg/ml) | IL-4 (pg/ml) |
|---|---|---|---|---|---|---|
| 225 | 50 | < 3.2 | < 3.2 | < 3.2 | < 3.2 | < 3.2 |
| 226 | | < 3.2 | < 3.2 | < 3.2 | < 3.2 | < 3.2 |
| 232 | 100 | < 3.2 | < 3.2 | < 3.2 | < 3.2 | < 3.2 |
| 233 | | < 3.2 | < 3.2 | < 3.2 | < 3.2 | < 3.2 |
| 239 | 100 | < 3.2 | < 3.2 | < 3.2 | < 3.2 | < 3.2 |
| 240 | | < 3.2 | < 3.2 | < 3.2 | < 3.2 | < 3.2 |
| 246 | 100 | < 3.2 | < 3.2 | < 3.2 | < 3.2 | < 3.2 |
| 247 | | < 3.2 | < 3.2 | < 3.2 | < 3.2 | < 3.2 |
| 253 | 100 | < 3.2 | < 3.2 | < 3.2 | < 3.2 | < 3.2 |
| 254 | | < 3.2 | < 3.2 | < 3.2 | < 3.2 | < 3.2 |
| 260 | 100 | < 3.2 | < 3.2 | < 3.2 | < 3.2 | < 3.2 |
| 261 | | < 3.2 | < 3.2 | < 3.2 | < 3.2 | < 3.2 |
| 267 | 100 | < 3.2 | < 3.2 | < 3.2 | < 3.2 | < 3.2 |
| 268 | | < 3.2 | < 3.2 | < 3.2 | < 3.2 | < 3.2 |

Table 7B: Systemic concentrations of cytokines IL-6, IL-8 and TNF-α and systemic catumaxomab antibody and HAMA in patient 2 - second treatment cycle.

| Day | Antibody (μg) | IL-6 (pg/ml) | IL-8 (pg/ml) | TNF-α (pg/ml) | Systemic Catumaxomab (pg/ml) | HAMA |
|---|---|---|---|---|---|---|
| 225 | 50 | < 3.2 | < 3.2 | < 3.2 | <125 | 159 |
| 226 | | < 3.2 | < 3.2 | < 3.2 | <125 | |
| 232 | 100 | < 3.2 | < 3.2 | < 3.2 | <125 | 63 |
| 233 | | < 3.2 | < 3.2 | < 3.2 | <125 | |
| 239 | 100 | < 3.2 | < 3.2 | < 3.2 | <125 | 63 |
| 240 | | < 3.2 | < 3.2 | < 3.2 | <125 | |
| 246 | 100 | < 3.2 | < 3.2 | < 3.2 | <125 | 64 |
| 247 | | < 3.2 | < 3.2 | < 3.2 | <125 | |
| 253 | 100 | < 3.2 | < 3.2 | < 3.2 | <125 | 154 |
| 254 | | < 3.2 | < 3.2 | < 3.2 | <125 | |
| 260 | 100 | < 3.2 | < 3.2 | < 3.2 | <125 | 281 |
| 261 | | < 3.2 | < 3.2 | < 3.2 | <125 | |
| 267 | 100 | < 3.2 | < 3.2 | < 3.2 | <125 | |
| 268 | | < 3.2 | < 3.2 | < 3.2 | <125 | |

According to the patient's own statements and the physical examinations of the attending physician the antibody treatment was very well tolerated without any obvious signs of negative side effects. Especially there were no reports of fever or flu-like symptoms. Accordingly, no significant systemic cytokine release was observed for both treatment cycles (Tables 6A/6B and 7A/7B). Only after the third instillation of 100 μg catumaxomab in the first treatment cycle, weak intermediate plasma levels of IL-6 of 16-13 pg/ml were measured (Table 6B). In the course of further treatment

IL-6 values returned below the detection limit of 3.2 pg/ml. Low amounts of IL-8 and TNF-α were already detectable before treatment start, but no treatment-related induction of these cytokines was observed. On the contrary, the cytokine values decreased below the detection limit in the course of the treatment. This result suggests that chronic inflammation, which is often observed in the environment of tumors, could be reduced during the course of therapy. Interestingly, IL-8 and TNF-α values and all other measured cytokines remained below the detection limit for the complete second treatment cycle (Tables 7A/7B).

Moreover, very low systemic catumaxomab antibody in the pg/ml range was detectable in the blood of the patient only after the second and after the fifth application of the first treatment cycle. All other concentrations measured were below the quantification limit of 125 pg/ml (Tables 6A/6B and 7A/7B). This indicates that the local antibody treatment of the bladder did not result in a significant systemic release of the drug. In accordance with this finding no distinct induction of human anti-mouse antibodies (HAMA) occurred. The patient had a borderline HAMA value of 40 ng/ml (values below 40 ng/ml are considered negative) already before treatment start. During the treatment, HAMA values first slightly increased but then decreased again almost back to the initial value (first treatment cycle, Table 6B). Importantly, even during the second treatment cycle HAMA values increased only slightly up to 281 ng/ml. Such a course does not represent a typical catumaxomab-induced HAMA reaction. In previous studies with intraperitoneally administered catumaxomab HAMA concentrations of up to $10^4$-$10^5$ ng/ml were reached (Ruf et al. Pharmacokinetics, immunogenicity and bioactivity of the therapeutic antibody catumaxomab intraperitoneally administered to cancer patients. Br J Clin Pharmacol. 69(6): 617-625 (2010)). Thus, catumaxomab, in particular administered intravesically, was obviously only weakly immunogenic in the described example.

Urine samples of the patient were analyzed for EpCAM+ tumor cells before and after the treatment. EpCAM+ tumor cells were detected by an established and described immunocytochemistry protocol as described in Jäger et al. Immunomonitoring Results of a Phase II/III Study of Malignant Ascites Patients Treated with the Trifunctional Antibody Catumaxomab (Anti-EpCAM x anti-CD3). Cancer Res. 72(1):24-32 (2012), which was modified for the analysis of urine samples. Briefly, cells in 30 ml urine were centrifuged on cytospins. Slides were double stained for EpCAM and cytokeratin. EpCAM staining was performed with the EpCAM-specific antibody HO-3 (Ruf et al. Characterisation of the new EpCAM-specific antibody HO-3: implications for trifunctional antibody immunotherapy of cancer. Br J Cancer 97 (3):315-321 (2007)) directly labeled with Alexa Fluor 594 Texas Red. For cytokeratin staining the anti-cytokeratin 8, 18, 19 antibodies A45B-B3 (Micromet) together with the corresponding Alexa Fluor 488-labeled secondary anti-mouse IgG1 detection antibody (Molecular Probes) were used. All cytospins were analyzed by a computerized image analysis system (MDS, Applied Imaging) counting double stained cells.

Results obtained during the second treatment cycle are shown in Table 8 below. Results obtained on follow-up days 319 (January 21, 2016) and 463 (June 15, 2016) are shown below in Table 9.

Table 8: Reduction of EpCAM+ tumor cells in urine during the second treatment cycle

| Day | Catumaxomab [μg] | EpCAM+ tumor cells |
|-----|------------------|--------------------|
| 225 | 50 | |
| 226 | | 111 |
| 232 | 100 | |
| 233 | | 83 |
| 239 | 100 | |
| 240 | | 26 |
| 246 | 100 | |
| 247 | | 27 |
| 253 | 100 | |
| 254 | | 8 |
| 260 | 100 | |
| 261 | | 6 |
| 267 | 100 | |
| 268 | | 4 |

Table 9: Reduction of EpCAM+ tumor cells in follow-up urine samples after the second treatment cycle

| Day | EpCAM+ tumor cells |
| --- | --- |
| 319 | 0 |
| 463 | 0 |

These results show the clinical efficacy of catumaxomab. Urine cytology of the patient was positive before treatment. Various urothelial cells showed enlarged, hyperchromatic cell nuclei and, in favor of the cell nuclei, a changed nucleus- plasma ratio. In the background, single lymphocytes and neutrophile granulocytes were observed.

Ten days after treatment with catumaxomab, various cells were observed in urine cytology without aberrant phenotype containing few lymphocytes and very few neutrophile granulocytes. No atypic cells were observed.

However, about six months later the patient recidivated with positive UroVysion FISH-test. Therefore, the patient received a second catumaxomab treatment cycle. Numbers of EpCAM+ tumor cells continuously decreased during the second treatment cycle from 111 down to 4 EpCAM positive tumor cells, as shown in Table 8. These results indicate a catumaxomab-mediated anti-tumor response *in vivo*. Noteworthy, the samples from day 261 and 268 presented only morphologically disordered cells and cell debris.

Most importantly, in follow-up urine samples obtained on days 319 and 463 no EpCAM-positive tumor cells were detected.

**Claims**

1. An isolated multispecific antibody, or an antigen binding fragment thereof, comprising

   (i) a specificity against CD3, and
   (ii) a specificity against a cancer- and/or tumor-associated antigen,

   for use in the treatment of a neoplasm of the urinary bladder,
   wherein the antibody, or the antigen binding fragment thereof, is administered intravesically.

2. The antibody, or the antigen binding fragment thereof, for use according to claim 1, wherein the antibody, or the antigen binding fragment thereof, is administered via instillation.

3. The antibody, or the antigen binding fragment thereof, for use according to claim 1 or 2, wherein the neoplasm of the urinary tract is a neoplasm of the urothelium, preferably urothelial cell carcinoma.

4. The antibody, or the antigen binding fragment thereof, for use according to any one of claims 1 - 3, wherein the neoplasm of the urinary bladder is selected from the group consisting of (i) carcinoma in situ of the urinary bladder; (ii) non-muscular invasive urothelial cancer localized at the trigone of bladder, at the dome of bladder, at the lateral wall of bladder, at the anterior wall of bladder, at the posterior wall of bladder, at the bladder neck, at the ureteric orifice and/or at the urachus; and (iii) muscular invasive urothelial cancer localized at the trigone of bladder, at the dome of bladder, at the lateral wall of bladder, at the anterior wall of bladder, at the posterior wall of bladder, at the bladder neck, at the ureteric orifice and/or at the urachus.

5. The antibody, or the antigen binding fragment thereof, for use according to any one of claims 1 - 4, wherein the neoplasm of the urinary bladder is selected from transitional cell carcinoma, squamous cell carcinoma, adenocarcinoma, sarcoma, small cell carcinoma, and a secondary deposit from a cancer elsewhere in the body, preferably the neoplasm of the urinary bladder is a transitional cell carcinoma.

6. The antibody, or the antigen binding fragment thereof, for use according to any of claims 1 to 5, wherein the cancer- and/or tumor-associated antigen is selected from the group consisting of EpCAM, HER2/neu, CEA, MAGE, proteoglycan, VEGF, EGFR, mTOR, PIK3CA, RAS, alpha(v)beta(3)-integrin, HLA, HLA-DR, ASC, carbonic anhydrase, CD1, CD2, CD4, CD6, CD7, CD8, CD11, CD13, CD14, CD19, CD20, CD21, CD22, CD23, CD24, CD30 CD33, CD37, CD38, CD40, CD41, CD47, CD52, CD133, c-erb-2, CALLA, MHCII, CD44v3, CD44v6, p97, GM1, GM2, GM3, GD1a, GD1b, GD2, GD3, GT1b, GT3, GQ1, NY-ESO-1, NFX2, SSX2, SSX4, Trp2, gp100, tyrosinase, MUC-1, telomerase, survivin, p53, PD-L1, CA125, Wue antigen, Lewis Y antigen, HSP-27, HSP-70, HSP-72, HSP-90, Pgp, MCSP, EphA2 and cell surface targets GC182, GT468 or GT512; preferably the cancer- and/or tumor-associated antigen is selected from the group consisting of EpCAM, HER2/neu, CEA, MAGE, VEGF, EGFR, mTOR, PD-L1, PIK3CA, RAS, GD2, CD19, CD20 and CD33, more preferably the cancer- and/or tumor-associated antigen is selected from the group consisting of EpCAM, HER2/neu, CEA, GD2, CD19, CD20 and CD33, even more preferably the cancer- and/or tumor-associated antigen is selected from the group consisting of EpCAM, HER2/neu, GD2 and CD20 and most preferably the cancer- and/or tumor-associated antigen is EpCAM.

7. The antibody, or the antigen binding fragment thereof, for use according to any of claims 1 to 6, wherein the antibody, or the antigen binding fragment thereof, comprises a binding site for an Fc receptor, preferably the antibody, or the antigen binding fragment thereof, comprises an Fc moiety.

8. The antibody, or the antigen binding fragment thereof, for use according to any of claims 1 to 7, wherein the antibody, or the antigen binding fragment thereof, is selected from the group consisting of catumaxomab, lymphomun, ertumaxomab, ektomab, blinatumomab and solitomab, preferably the antibody is catumaxomab.

9. The antibody, or the antigen binding fragment thereof, for use according to any of claims 1 to 8, wherein the antibody, or the antigen binding fragment thereof, is administered in one or more treatment cycles; preferably wherein one treatment cycle comprises (i) one single dose or (ii) one initial dose and one or more subsequent doses; more preferably wherein one treatment cycle comprises one initial dose and one or more subsequent doses and wherein the one initial dose and the one or more subsequent doses are the same, or wherein the one or more subsequent dose(s) is/are higher than the initial dose; in particular wherein the first subsequent dose exceeds the amount administered as initial dose, preferably by a factor of 1.1 to 10.0, more preferably by a factor of 1.2 to 5.0, and, optionally, the second and each subsequent dose exceeds the amount administered as initial dose by a factor of 1.1 to 10.0, preferably 1.5 to 5.0.

10. The antibody, or the antigen binding fragment thereof, for use according to any of claims 1 to 9, wherein the antibody, or the antigen binding fragment thereof, is administered as stand-alone therapy.

11. The antibody, or the antigen binding fragment thereof, for use according to any of claims 1 to 9, wherein the antibody, or the antigen binding fragment thereof, is administered in combination with autologous immune effector cells, preferably in combination with PBMCs.

12. The antibody, or the antigen binding fragment thereof, for use according to any of claims 1 to 9 and 11, wherein the antibody, or the antigen binding fragment thereof, is administered in combination with an anti-cancer drug; preferably wherein said anti-cancer drug is a cytotoxic agent or an anti-cancer antibody; more preferably wherein the cytotoxic agent is selected from the group consisting of mitomycin, valrubicin, docetaxel, thiotepa, cisplatin and gemcitabine; and/or the anti-cancer antibody is preferably a monoclonal, monospecific anti-cancer antibody, more preferably selected from the group consisting of trastuzumab, alemtuzumab, atezolizumab, avelumab, bevacizumab, brentuximab vedotin, cetuximab, gemtuzumab ozogamicin, ibritumomab, tiuxetan, ipilimumab, nimotuzumab, nivolumab, ofatumumab, panitumumab, pembrolizumab, rituximab and tositumomab.

13. A pharmaceutical composition comprising the antibody, or the antigen binding fragment thereof, as defined in any one of claims 1 and 6 to 8, for use in the treatment of a neoplasm of the urinary bladder according to any one of claims 1 to 5 and 9 to 12, wherein the pharmaceutical composition is administered intravesically and wherein the pharmaceutical composition preferably comprises a pharmaceutically acceptable carrier or vehicle; more preferably the pharmaceutical composition comprises a buffer, preferably an organic acid buffer, such as citrate buffer, succinate buffer and tartrate buffer.

14. A kit comprising (i) the antibody or the antigen binding fragment thereof, as defined in any of claims 1 and 6 to 8 or the pharmaceutical composition as defined in claim 13 and (ii) a package insert or label with directions to treat a neoplasm of the urinary bladder, preferably by local administration to the urinary bladder; for use in prevention and/or treatment of a neoplasm of the urinary bladder, wherein the antibody or the antigen binding fragment thereof is administered intravesically.

**Patentansprüche**

1. Ein isolierter multispezifischer Antikörper, oder ein antigenbindendes Fragment davon, umfassend

(i) eine Spezifität gegen CD3, und

(ii) eine Spezifität gegen ein Krebs- und/oder Tumor-assoziiertes Antigen,

zur Verwendung bei der Behandlung eines Neoplasmas der Harnblase,

wobei der Antikörper, oder das antigenbindende Fragment davon, intravesikal verabreicht wird.

2. Der Antikörper, oder das antigenbindende Fragment davon, zur Verwendung gemäß Anspruch 1, wobei der Antikörper oder das antigenbindende Fragment davon durch Instillation verabreicht wird.

3. Der Antikörper, oder das antigenbindende Fragment davon, zur Verwendung nach Anspruch 1 oder 2, wobei das Neoplasma der Harnwege ein Neoplasma des Urothels, vorzugsweise ein Urothelzellkarzinom, ist.

4. Der Antikörper, oder das antigenbindende Fragment davon, zur Verwendung nach einem der Ansprüche 1 bis 3, wobei das Neoplasma der Harnblase ausgewählt ist aus der Gruppe bestehend aus (i) Carcinoma in situ der Harnblase; (ii) nichtmuskulärem invasivem Urothelkarzinom, das am Blasendreieck, am Blasendach, an der Seitenwand der Blase, an der vorderen Blasenwand, an der hinteren Blasenwand, am Blasenhals, an der Harnleiteröffnung und/oder am Urachus lokalisiert ist und (iii) muskuläres invasives Urothelkarzinom, das am Blasendreieck, am Blasendach, an der seitlichen Blasenwand, an der vorderen Blasenwand, an der hinteren Blasenwand, am Blasenhals, an der Harnleiteröffnung und/oder am Urachus lokalisiert ist.

5. Der Antikörper, oder das antigenbindende Fragment davon, zur Verwendung nach einem der Ansprüche 1 bis 4, wobei das Neoplasma der Harnblase ausgewählt ist aus Transitionalzellkarzinom, Plattenepithelkarzinom, Adenokarzinom, Sarkom, kleinzelligem Karzinom und einer sekundären Ablagerung von einem Krebs an anderer Stelle im Körper, wobei das Neoplasma der Harnblase vorzugsweise ein Transitionalzellkarzinom ist.

6. Der Antikörper, oder das antigenbindende Fragment davon, zur Verwendung nach einem der Ansprüche 1 bis 5, wobei das krebs- und/oder tumorassoziierte Antigen ausgewählt ist aus der Gruppe bestehend aus EpCAM, HER2/neu, CEA, MAGE, Proteoglycan, VEGF, EGFR, mTOR, PIK3CA, RAS, alpha(v)beta(3)-Integrin, HLA, HLA-DR, ASC, Carboanhydrase, CD1, CD2, CD4, CD6, CD7, CD8, CD11, CD13, CD14, CD19, CD20, CD21, CD22, CD23, CD24, CD30 CD33, CD37, CD38, CD40, CD41, CD47, CD52, CD133, c-erb-2, CALLA, MHCII, CD44v3, CD44v6, p97, GM1, GM2, GM3, GD1a, GD1b, GD2, GD3, GT1b, GT3, GQ1, NY-ESO-1, NFX2, SSX2, SSX4, Trp2, gp100, Tyrosinase, MUC-1, Telomerase, Survivin, p53, PD-L1, CA125, Wue-Antigen, Lewis-Y-Antigen, HSP-27, HSP-70, HSP-72, HSP-90, Pgp, MCSP, EphA2 und Zelloberflächenziele GC182, GT468 oder GT512; vorzugsweise ist das krebs- und/oder tumorassoziierte Antigen ausgewählt aus der Gruppe bestehend aus EpCAM, HER2/neu, CEA, MAGE, VEGF, EGFR, mTOR, PD-L1, PIK3CA, RAS, GD2, CD19, CD20 und CD33, noch bevorzugter ist das krebs- und/oder tumorassoziierte Antigen ausgewählt aus der Gruppe bestehend aus EpCAM, HER2/neu, CEA, GD2, CD19, CD20 und CD33, noch mehr bevorzugt ist das krebs- und/oder tumorassoziierte Antigen ausgewählt aus der Gruppe bestehend aus EpCAM, HER2/neu, GD2 und CD20, und am meisten bevorzugt ist das krebs- und/oder tumorassoziierte Antigen EpCAM.

7. Der Antikörper, oder das antigenbindende Fragment davon, zur Verwendung nach einem der Ansprüche 1 bis 6, wobei der Antikörper, oder das antigenbindende Fragment davon, eine Bindungsstelle für einen Fc-Rezeptor umfasst, vorzugsweise umfasst der Antikörper, oder das antigenbindende Fragment davon, eine Fc-Einheit.

8. Der Antikörper, oder das antigenbindende Fragment davon, zur Verwendung nach einem der Ansprüche 1 bis 7, wobei der Antikörper, oder das antigenbindende Fragment davon, ausgewählt ist aus der Gruppe bestehend aus Catumaxomab, Lymphomun, Ertumaxomab, Ektomab, Blinatumomab und Solitomab, vorzugsweise ist der Antikörper Catumaxomab.

9. Der Antikörper, oder das antigenbindende Fragment davon, zur Verwendung nach einem der Ansprüche 1 bis 8, wobei der Antikörper, oder das antigenbindende Fragment davon, in einem oder mehreren Behandlungszyklen verabreicht wird, wobei ein Behandlungszyklus vorzugsweise (i) eine Einzeldosis oder (ii) eine Anfangsdosis und eine oder mehrere nachfolgende Dosen umfasst; wobei ein Behandlungszyklus mehr bevorzugt eine Anfangsdosis und eine oder mehrere nachfolgende Dosen umfasst und wobei die eine Anfangsdosis und die eine oder mehrere nachfolgende Dosen gleich sind oder wobei die eine oder mehrere nachfolgende(n) Dosis(en) höher ist/sind als die Anfangsdosis; insbesondere wobei die erste nachfolgende Dosis die als Anfangsdosis verabreichte Menge übersteigt, vorzugsweise um einen Faktor von 1,1 bis 10,0, mehr bevorzugt um einen Faktor von 1,2 bis 5,0, und gegebenenfalls die zweite und jede weitere Dosis die als Anfangsdosis verabreichte Menge um einen Faktor von 1,1 bis 10,0, mehr bevorzugt 1,5 bis 5,0, übersteigt.

10. Der Antikörper, oder das antigenbindende Fragment davon, zur Verwendung nach einem der Ansprüche 1 bis 9, wobei der Antikörper, oder das antigenbindende Fragment davon, als alleinige Therapie verabreicht wird.

11. Der Antikörper, oder das antigenbindende Fragment davon, zur Verwendung nach einem der Ansprüche 1 bis 9, wobei der Antikörper, oder das antigenbindende Fragment davon, in Kombination mit autologen Immuneffektorzellen, vorzugsweise in Kombination mit PBMCs, verabreicht wird.

12. Der Antikörper, oder das antigenbindende Fragment davon, zur Verwendung nach einem der Ansprüche 1 bis 9 und 11, wobei der Antikörper, oder das antigenbindende Fragment davon, in Kombination mit einem Anti-Krebs-Medikament verabreicht wird; vorzugsweise wobei das Anti-Krebs-Medikament ein zytotoxisches Mittel oder ein Anti-Krebs-Antikörper ist; noch bevorzugter wobei das zytotoxische Mittel ausgewählt ist aus der Gruppe bestehend aus Mitomycin, Valrubicin, Docetaxel, Thiotepa, Cisplatin und Gemcitabin; und/oder der Antikrebs-Antikörper vorzugsweise ein monoklonaler, monospezifischer Antikrebs-Antikörper ist, mehr bevorzugt ausgewählt aus der Gruppe bestehend aus Trastuzumab, Alemtuzumab, Atezolizumab, Avelumab, Bevacizumab, Brentuximab Vedotin, Cetuximab, Gemtuzumab Ozogamicin, Ibritumomab, Tiuxetan, Ipilimumab, Nimotuzumab, Nivolumab, Ofatumumab, Panitumumab, Pembrolizumab, Rituximab und Tositumomab.

13. Pharmazeutische Zusammensetzung, umfassend den Antikörper, oder das antigenbindende Fragment davon, wie in einem der Ansprüche 1 und 6 bis 8 definiert, zur Verwendung bei der Behandlung eines Neoplasmas der Harnblase gemäß einem der Ansprüche 1 bis 5 und 9 bis 12, wobei die pharmazeutische Zusammensetzung intravesikal verabreicht wird und wobei die pharmazeutische Zusammensetzung vorzugsweise einen pharmazeutisch annehmbaren Träger oder ein pharmazeutisch annehmbares Vehikel umfasst; noch bevorzugter umfasst die pharmazeutische Zusammensetzung einen Puffer, vorzugsweise einen organischen Säurepuffer, wie Citratpuffer, Succinatpuffer und Tartratpuffer.

14. Kit, umfassend (i) den Antikörper oder das antigenbindende Fragment davon, wie in einem der Ansprüche 1 und 6 bis 8 definiert, oder die pharmazeutische Zusammensetzung, wie in Anspruch 13 definiert, und (ii) eine Packungsbeilage oder ein Etikett mit Anweisungen zur Behandlung eines Neoplasmas der Harnblase, vorzugsweise durch lokale Verabreichung an die Harnblase; zur Verwendung bei der Prävention und/oder Behandlung eines Neoplasmas der Harnblase, wobei der Antikörper, oder das antigenbindende Fragment davon, intravesikal verabreicht wird.

## Revendications

1. Anticorps multispécifique isolé, ou fragment de liaison à l'antigène de celui-ci, comprenant

   (i) une spécificité contre CD3, et
   (ii) une spécificité contre un antigène associé à un cancer et/ou à une tumeur,

   pour une utilisation dans le traitement d'un néoplasme de la vessie,
   dans lequel l'anticorps, ou le fragment de liaison à l'antigène de celui-ci, est administré par voie intravésicale.

2. Anticorps, ou fragment de liaison à l'antigène de celui-ci, pour une utilisation selon la revendication 1, dans lequel l'anticorps, ou le fragment de liaison à l'antigène de celui-ci, est administré par instillation.

3. Anticorps, ou fragment de liaison à l'antigène de celui-ci, pour une utilisation selon la revendication 1 ou 2, dans lequel le néoplasme du tractus urinaire est un néoplasme de l'urothélium, de préférence un carcinome à cellules urothéliales.

4. Anticorps, ou fragment de liaison à l'antigène de celui-ci, pour une utilisation selon l'une quelconque des revendications 1 à 3, dans lequel le néoplasme de la vessie est choisi dans le groupe constitué par (i) un carcinome in situ, de la vessie ; (ii) un cancer urothélial invasif non musculaire, localisé au niveau du trigone de la vessie, au niveau du dôme de la vessie, au niveau de la paroi latérale de la vessie, au niveau de la paroi antérieure de la vessie, au niveau de la paroi postérieure de la vessie, au niveau du col de la vessie, au niveau de l'orifice urétéral et/ou au niveau de l'ouraque; et (iii) un cancer urothélial invasif musculaire, localisé au niveau du trigone de la vessie, au niveau du dôme de la vessie, au niveau de la paroi latérale de la vessie, au niveau de la paroi antérieure de la vessie, au niveau de la paroi postérieure de la vessie, au niveau du col de la vessie, au niveau de l'orifice urétéral et/ou au niveau de l'ouraque.

5. Anticorps, ou fragment de liaison à l'antigène de celui-ci, pour une utilisation selon l'une quelconque des revendications 1 à 4, dans lequel le néoplasme de la vessie est choisi parmi un carcinome à cellules de transition, un carcinome à cellules squameuses, un adénocarcinome, un sarcome, un carcinome à petites cellules et un dépôt secondaire à partir d'un cancer ailleurs dans le corps, de préférence le néoplasme de la vessie est un carcinome à cellules de transition.

6. Anticorps, ou fragment de liaison à l'antigène de celui-ci, pour une utilisation selon l'une quelconque des revendications 1 à 5, dans lequel l'antigène associé à un cancer et/ou à une tumeur est choisi dans le groupe constitué par EpCAM, HER2/neu, CEA, MAGE, un protéoglycane, VEGF, EGFR, mTOR, PIK3CA, RAS, l'alpha(v)bêta(3)-intégrine, HLA, HLA-DR, ASC, l'anhydrase carbonique, CD1, CD2, CD4, CD6, CD7, CD8, CD11, CD13, CD14, CD19, CD20, CD21, CD22, CD23, CD24, CD30, CD33, CD37, CD38, CD40, CD41, CD47, CD52, CD133, c-erb-2, CALLA, MHCII, CD44v3, CD44v6, p97, GM1, GM2, GM3, GD1a, GD1b, GD2, GD3, GT1b, GT3, GQ1, NY-ESO-1, NFX2, SSX2, SSX4, Trp2, gp100, la tyrosinase, MUC-1, la télomérase, la survivine, p53, PD-L1, CA125, l'antigène Wue, l'antigène Lewis Y, HSP-27, HSP-70, HSP-72, HSP-90, Pgp, MCSP, EphA2 et des cibles de surface cellulaire GC182, GT468 ou GT512 : de préférence l'antigène associé à un cancer et/ou à une tumeur est choisi dans le groupe constitué par EpCAM, HER2/neu, CEA, MAGE, VEGF, EGFR, mTOR, PD-L1, PIK3CA, RAS, GD2, CD19, CD20 et CD33, plus préférablement l'antigène associé à un cancer et/ou à une tumeur est choisi dans le groupe constitué par EpCAM, HER2/neu, CEA, GD2, CD19, CD20 et CD33, encore plus préférablement l'antigène associé à un cancer et/ou à une tumeur est choisi dans le groupe constitué par EpCAM, HER2/neu, GD2 et CD20 et idéalement l'antigène associé à un cancer et/ou à une tumeur est EpCAM.

7. Anticorps, ou fragment de liaison à l'antigène de celui-ci, pour une utilisation selon l'une quelconque des revendications 1 à 6, dans lequel l'anticorps, ou le fragment de liaison à l'antigène de celui-ci, comprend un site de liaison pour un récepteur Fc, de préférence l'anticorps, ou le fragment de liaison à l'antigène de celui-ci, comprend une fraction Fc.

8. Anticorps, ou fragment de liaison à l'antigène de celui-ci, pour une utilisation selon l'une quelconque des revendications 1 à 7, dans lequel l'anticorps ou le fragment de liaison à l'antigène de celui-ci, est choisi dans le groupe constitué par le catumaxomab, le lymphomun, l'ertumaxomab, l'ektomab, le blinatumomab et le solitomab, de préférence l'anticorps est le catumaxomab.

9. Anticorps, ou fragment de liaison à l'antigène de celui-ci, pour une utilisation selon l'une quelconque des revendications 1 à 8, dans lequel l'anticorps, ou le fragment de liaison à l'antigène de celui-ci, est administré en un ou plusieurs cycles de traitement ; de préférence dans lequel un cycle de traitement comprend (i) une dose unique ou (ii) une dose initiale et une ou plusieurs doses ultérieures ; plus préférablement dans lequel un cycle de traitement comprend une dose initiale et une ou plusieurs doses ultérieures et dans lequel la dose initiale et la ou les doses ultérieures sont identiques, ou dans lequel la ou les dose(s) ultérieure(s) est/sont supérieure(s) à la dose initiale ; en particulier dans lequel la première dose ultérieure dépasse la quantité administrée en tant que dose initiale, de préférence d'un facteur de 1,1 à 10,0, plus préférablement d'un facteur de 1,2 à 5,0, et, éventuellement, la deuxième dose et chaque dose ultérieure dépasse la quantité administrée en tant que une dose initiale d'un facteur de 1,1 à 10,0, de préférence de 1,5 à 5,0.

10. Anticorps, ou fragment de liaison à l'antigène de celui-ci, pour une utilisation selon l'une quelconque des revendications 1 à 9, dans lequel l'anticorps, ou le fragment de liaison à l'antigène de celui-ci, est administré en tant que thérapie autonome.

11. Anticorps, ou fragment de liaison à l'antigène de celui-ci, pour une utilisation selon l'une quelconque des revendications 1 à 9, dans lequel l'anticorps, ou le fragment de liaison à l'antigène de celui-ci, est administré en combinaison avec des cellules effectrices immunitaires autologues, de préférence en combinaison avec des PBMCs.

12. Anticorps, ou fragment de liaison à l'antigène de celui-ci, pour une utilisation selon l'une quelconque des revendications 1 à 9 et 11, dans lequel l'anticorps, ou le fragment de liaison à l'antigène de celui-ci, est administré en combinaison avec un médicament anticancéreux ; dans lequel de préférence ledit médicament anticancéreux est un agent cytotoxique ou un anticorps anticancéreux ; plus préférablement dans lequel agent cytotoxique est choisi dans le groupe constitué par la mitomycine, la valrubicine, le docétaxel, le thiotépa, le cisplatine et la gemcitabine ;

et/ou l'anticorps anticancéreux est de préférence un anticorps anticancéreux monoclonal monospécifique, plus préférablement choisi dans le groupe constitué par le trastuzumab, l'alemtuzumab, l'atézolizumab, l'avélumab, le bévacizumab, le brentuximab védotine, le cétuximab, le gemtuzumab ozogamicine, l'ibritumomab, le tiuxétan, l'ipilimumab, le nimotuzumab, le nivolumab, l'ofatumumab, le panitumumab, le pembrolizumab, le rituximab et le tositumomab.

13. Composition pharmaceutique comprenant l'anticorps, ou le fragment de liaison à l'antigène de celui-ci, tel que défini selon l'une quelconque des revendications 1 et 6 à 8, pour une utilisation dans le traitement d'un néoplasme de la vessie selon l'une quelconque des revendications 1 à 5 et 9 à12, dans laquelle la composition pharmaceutique est administrée par voie intravésicale et dans laquelle la composition pharmaceutique comprend de préférence un support ou un véhicule pharmaceutiquement acceptable ; plus préférablement la composition pharmaceutique comprend un tampon, de préférence un tampon acide organique, tel qu'un tampon citrate, un tampon succinate et un tampon tartrate.

14. Kit comprenant (i) l'anticorps ou le fragment de liaison à l'antigène de celui-ci, tel que défini dans l'une quelconque des revendications 1 et 6 à 8 ou la composition pharmaceutique telle que définie dans la revendication 13 et (ii) un insert ou une étiquette d'emballage avec des instructions pour traiter un néoplasme de la vessie, de préférence par administration locale à la vessie ; pour une utilisation dans la prévention et/ou le traitement d'un néoplasme de la vessie, dans lequel l'anticorps ou le fragment de liaison à l'antigène de celui-ci est administré par voie intravésicale.

A

B

Figure 1

Figure 2

A

B

C

D

E

F

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- World Cancer Report 2014. World Health Organization, 2014
- **FAIR WR ; FUKS ZY ; SCHER HL.** Cancer: Principles & Practice of Oncology. J.B. Lippincott Co, 1993, 1052-1072
- Urothelial tumors of the bladder. **MESSING EM et al.** Campbell-Walsh Urology. Saunders Elsevier, 2007, 1445
- **BÖHLE.** Recent knowledge on BCG's mechanism of action in the treatment of superficial bladder cancer. *Braz J Urol,* 2000, vol. 26, 488
- **BURGER et al.** The application of adjuvant autologous intravesical macrophage cell therapy vs. BCG in non-muscle invasive bladder cancer: a multicenter randomized trial. *J Transl Med,* 2010, vol. 8, 54
- **BABJUK M.** EAU Guidelines on Non-Muscle-Invasive Urothelial Carcinoma of the Bladder. *Eur Urol,* 2008, vol. 54 (2), 303
- **DENZINGER S et al.** Versus deferred cystectomy for initial high-risk pT1G3 urothelial carcinoma of the bladder: do risk factors define feasibility of bladder-sparing approach?. *Eur Urol,* 2008, vol. 53 (1), 146
- **WITJES.** Management of BCG failures in superficial bladder cancer: a review. *Eur Urol,* 2006, vol. 49 (5), 790
- **HERR.** Is maintenance Bacillus Calmette-Guérin really necessary?. *Eur Urol,* 2008, vol. 54 (5), 971-3
- **ZLOTTA AR et al.** What are the immunologically active components of bacille Calmette-Guérin in therapy of superficial bladder cancer?. *Int J Cancer,* 2000, vol. 87 (6), 844
- **LUO Y et al.** Role of Th1-stimulating cytokines in bacillus Calmette-Guérin (BCG)-induced macrophage cytotoxicity against mouse bladder cancer MBT-2 cells. *Clin Exp Immunol,* 2006, vol. 146 (1), 181
- **AYARI C et al.** Bladder tumour infiltrating mature dendritic cells and macrophages as predictors of response to bacillus Calmette-Guerin immunotherapy. *Eur Urol,* 2009, vol. 55 (6), 1386
- **DE REIJKE.** Editorial comment on: Bladder tumour infiltrating mature dendritic cells and macrophages as predictors of response to bacillus Calmette-Guérin immunotherapy. *Eur Urol,* 2009, vol. 55 (6), 1395

- **TAKAYAMA H et al.** Increased infiltration of tumor associated macrophages is associated with poor prognosis of bladder carcinoma in situ after intravesical bacillus Calmette-Guerin instillation. *J Urol,* 2009, vol. 181 (4), 1894
- **BRANDAU.** Tumour-associated macrophages: predicting bacillus Calmette-Guerin immunotherapy outcomes. *J Urol,* 2009, vol. 181 (4), 1532
- **SIRACUSANO S et al.** The role of granulocytes following intravesical BCG prophylaxis. *Eur Urol,* 2007, vol. 51 (6), 1589
- **BRANDAU S et al.** The role of granulocytes following intravesical BCG prophylaxis. *Eur Urol,* 2007, vol. 51, 1589-99
- **CARTHON BC ; WOLCHOK JD ; YUAN J ; KAMAT A ; NG TANG DS ; SUN J et al.** Preoperative CTLA-4 blockade: tolerability and immune monitoring in the setting of a presurgical clinical trial. *Clin Cancer Res,* 2010, vol. 16 (10), 2861-71
- **NAIDOO J ; PAGE DB ; LI BT ; CONNELL LC ; SCHINDLER K ; LACOUTURE ME ; POSTOW MA ; WOLCHOK JD.** Toxicities of the anti-PD-1 and anti-PD-L1 immune checkpoint antibodies. *Ann Oncol.,* 2015, vol. 26 (12), 2375
- **CHAMPIAT S ; LAMBOTTE O ; BARREAU E ; BELKHIR R ; BERDELOU A ; CARBONNEL F ; CAUQUIL C ; CHANSON P ; COLLINS M ; DURRBACH A.** Management of immune checkpoint blockade dysimmune toxicities: a collaborative position paper. *Ann Oncol.,* 28 December 2015, mdv623
- **KOWALSKI et al.** A phase II study of oportuzumab monatox: an immunotoxin therapy for patients with noninvasive urothelial carcinoma in situ previously treated with bacillus Calmette-Guérin. *J Urol,* 2012, vol. 188, 1712
- **VAN DIJK, M. A. ; VAN DE WINKEL, J. G.** *Curr. Opin. Chem. Biol.,* 2001, vol. 5, 368-374
- **JAKOBOVITS, A. et al.** *Proc. Natl. Acad. Sci. USA,* 1993, vol. 90, 2551-2555
- **JAKOBOVITS, A. et al.** *Nature,* 1993, vol. 362, 255-258
- **BRUGGEMANN, M. et al.** *Year Immunol.,* 1993, vol. 7, 3340
- **HOOGENBOOM, H. R. ; WINTER, G.** *J. Mol. Biol.,* 1992, vol. 227, 381-388
- **MARKS, J. D. et al.** *J. Mol. Biol.,* 1991, vol. 222, 581-597
- **COLE et al.** Monoclonal Antibodies and Cancer Therapy. Alan R. Liss, 1985, 77

- **BOERNER, P. et al.** *J. Immunol.,* 1991, vol. 147, 86-95
- **HOLLIGER ; HUDSON.** *Nature Biotechnology,* 2005, vol. 9, 1126-1136
- **VAN DER BRUGGEN P ; STROOBANT V ; VIGNERON N ; VAN DEN EYNDE B.** Peptide database: T cell-defined tumor antigens. *Cancer Immun,* 2013, http://www.cancerimmunity.org/peptide
- **KIEVIT et al.** *Int. J. Cancer,* 1997, vol. 71, 237-245
- **LOZZA et al.** *Anticancer Res.,* 1997, vol. 17, 525-529
- **MOTA et al.** *Am. J Pathol.,* 1997, vol. 150, 1223-1229
- **MACS et al.** *J. Cancer Res. Clin. Oncol.,* 1996, vol. 122, 296-300
- **TOLLIVER ; O'BRIEN.** *South Med. J.,* 1997, vol. 90, 89-90
- **TSURUTA.** *Urol. Int.,* 1997, vol. 58, 20-24
- **HUANG et al.** *Exper Rev. Vaccines,* 2002, vol. 1, 49-63
- **ZANTEK et al.** *Cell Growth Differ.,* 1999, vol. 10, 629-38
- **CARLES-KINCH et al.** *Cancer Res.,* 2002, vol. 62, 2840-7
- **CHENG.** *Cytokine Growth Factor Rev.,* 2002, vol. 13, 75-85
- **DAHLENBORG et al.** *Int. J Cancer,* 1997, vol. 70, 63-71
- **ZAJAC et al.** *Int. J Cancer,* 1997, vol. 71, 491-496
- **DESHPANDE ; DANISHEFSKY.** *Nature,* 1997, vol. 387, 164-166
- **KAWAKAMI ; ROSENBERG.** *Int. Rev. Immunol.,* 1997, vol. 14, 173-192
- **MOLLDREM et al.** *Blood,* 1996, vol. 88, 2450-7
- **MOLLDREM et al.** *Blood,* 1997, vol. 90, 2529-34
- **JONES et al.** *Anticancer Res.,* 1997, vol. 17, 685-687
- Human and Mouse CD Marker Handbook. BD Bioscience
- **HEISS et al.** The trifunctional antibody catumaxomab for the treatment of malignant ascites due to epithelial cancer: results of a prospective randomized phase II/III trial. *Int. J Cancer,* 2010, vol. 127, 2209-2221
- **OTT et al.** Humoral response to catumaxomab correlates with clinical outcome: results of the pivotal phase II/III study in patients with malignant ascites. *Int. J. Cancer,* 2012, vol. 130, 2195-2203
- **LONGE, JACQUELINE L.** Gale Encyclopedia Of Cancer: A Guide To Cancer And Its Treatments. Thomson Gale, 2005, 137
- **BRUNNER et al.** EpCAM is predominantly expressed in high grade and advanced stage urothelial carcinoma of the bladder. *J Clin Pathol,* 2008, vol. 61 (3), 307
- **SPIESS C. ; ZHAI Q. ; CARTER P.J.** *Molecular Immunology,* 2015, vol. 67, 95-106
- **BYRNE H. et al.** *Trends Biotech,* 2013, vol. 31 (11), 621-632
- **WEIDLE U.H. et al.** *Cancer Genomics and Proteomics,* 2013, vol. 10, 1-18
- **CHAN, A.C. ; CARTER, P.J.** *Nat Rev Immu,* 2010, vol. 10, 301-316
- **WEIDLE U.H. et al.** *Cancer Genomics and Proteomics,* 2013, vol. 10, 1-18
- **RUF et al.** *Int J Cancer,* 2004, vol. 108, 725-732
- **HEISS et al.** *Int J Cancer,* 2010, vol. 127, 2209-2221
- Assessment Report for Removab. European Medicines Agency
- **BUHMANN et al.** *Bone Marrow Transplant,* 2009, vol. 43, 383-397
- **KIEWE et al.** *Clin Cancer Res,* 2006, vol. 12, 3085-3091
- **CHELIUS et al.** Structural and functional characterization of the trifunctional antibody catumaxomab. *mAbs,* 2010, vol. 2 (3), 1-12
- Trifunctional Triomab® antibodies for cancer therapy. **LINDHOFER H ; HESS J ; RUF P.** Bispecific Antibodies. Springer Heidelberg Dordrecht, 2011, 289
- **RUF et al.** Pharmacokinetics, immunogenicity and bioactivity of the therapeutic antibody catumaxomab intraperitoneally administered to cancer patients. *Br J Clin Pharmacol.,* 2010, vol. 69 (6), 617-625
- **JÄGER et al.** Immunomonitoring Results of a Phase II/III Study of Malignant Ascites Patients Treated with the Trifunctional Antibody Catumaxomab (Anti-EpCAM x anti-CD3). *Cancer Res.,* 2012, vol. 72 (1), 24-32
- **RUF et al.** Characterisation of the new EpCAM-specific antibody HO-3: implications for trifunctional antibody immunotherapy of cancer. *Br J Cancer,* 2007, vol. 97 (3), 315-321
- **BABJUK et al.** EAU guidelines on non-muscle-invasive urothelial carcinoma of the bladder, the 2011 Update. *Eur Urol,* 2011, vol. 59, 997-1008